# EUROPEAN PATENT APPLICATION

(11) **EP 4 759 817 A1**
(43) Date of publication of application: **17.06.2026**
(21) Application number: 24851113.1
(22) Date of filing: 09.08.2024
(51) Int. Cl.: C07D 498/04, C07D 493/00, C07D 495/04, C07D 405/14, A61K 31/5386, A61K 31/498, A61P 35/00

(54) **HETEROARYL COMPOUND, PREPARATION METHOD THEREFOR, AND USE THEREOF IN MEDICINE**

(30) Priority: 09.08.2023 CN 202310996501; 07.09.2023 CN 202311151377; 27.10.2023 CN 202311408086; 11.01.2024 CN 202410042848
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: LI, Xin, Shanghai 200245 (CN); SHEN, Feng, Shanghai 200245 (CN); GUI, Bin, Shanghai 200245 (CN); WANG, Wei, Shanghai 200245 (CN); KONG, Luyao, Shanghai 200245 (CN); HE, Feng, Shanghai 200245 (CN)
(74) Representative: Dragotti & Associati S.P.A.
(86) International application number: PCT/CN2024/110882
(87) International publication number: WO 2025/031468

(57) **Abstract**

A heteroaryl compound, a preparation method therefor, and a use thereof in medicine. Specifically, provided are a heteroaryl compound represented by general formula (I), a preparation method therefor, a pharmaceutical composition containing the compound, and a use thereof as a therapeutic agent, especially a use thereof as a PRMT5 inhibitor and a use thereof in treating and/or preventing cancer.

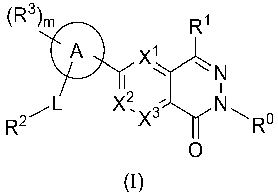

## Description

### Technical Field

The present disclosure belongs to the pharmaceutical field and relates to a heteroaryl compound, and a preparation method therefor and the use thereof in medicine. Specifically, the present disclosure relates to a heteroaryl compound represented by general formula (I), and a preparation method therefor, a pharmaceutical composition containing the compound, the use thereof as a PRMT5 inhibitor and the use thereof in treating and/or preventing cancer.

### Background Art

Arginine methylation, a prevalent post-translational modification form in mammalian cells, is primarily controlled by protein arginine methyltransferases (PRMTs). The PRMT family consists of nine members, which can be classified into three types based on their catalytic activity and product types: 1) Type I, including PRMT1, PRMT2, PRMT3, PRMT4 and PRMT8; 2) Type II, including PRMT5 and PRMT7; 3) Type III, represented solely by PRMT7.

As an important type II arginine methyltransferase, protein methylarginine transferase 5 (PRMT5) can induce the symmetric dimethylation of arginine residues on histone and non-histone proteins, thereby regulating a variety of physiological functions in mammalian cells, including genomic epigenetic modification, regulation of RNA splicing, DNA repair, regulation of Treg cell gene expression, antigen presentation methylation, etc. Studies have shown that PRMT5 overexpression plays an important role in proliferative diseases, metabolic diseases, haematological diseases, and various cancers.

Homozygous deletion of the chromosome 9p21 (chr9p21) locus occurs in approximately 15% of human cancers, with a frequency of 50% or more in glioblastoma multiforme. The chr9p21 locus contains the CDKN2A gene, which is an important tumour suppressor gene that encodes the key tumour suppressors p19-ARF and p16-INK4a. Deletion of the chr9p21 gene often involves co-deletion of genes adjacent to CDKN2A. Therefore, the gene encoding methylthioadenosine phosphorylase (MTAP), located within 100 kb of CDKN2A, is homozygous deleted in up to 80%-90% of CDKN2A-deficient tumours.

MTAP is a key enzyme in the methionine salvage pathway of the "methionine cycle" and metabolises 5-methylthioadenosine (MTA), a byproduct of polyamine synthesis, thereby regenerating methionine and adenine. Therefore, tumour cells with MTAP gene deletion will cause a large accumulation of intracellular/extracellular MTA, which acts as a specific competitive inhibitor of PRMT5 by specifically binding to the PRMT5-SAM binding pocket, thereby inhibiting PRMT5 activity. Studies have shown that PRMT5 is a synthetic lethal gene in MTAP-deficient cells, making PRMT5 inhibitors promising next-generation targeted drugs for the treatment of MTAP-deleted tumours.

Several PRMT5 inhibitors with different mechanisms are currently under clinical development. Among them, first-generation PRMT5 inhibitors, which are mostly substrate-competitive or SAM-competitive inhibitors, not only kill MTAP-deficient tumour cells but also suppress the proliferation of normal cells. Haematological toxicity is the main dose-limiting adverse reaction of current first-generation PRMT5 inhibitors.

Therefore, there is an urgent medical need to develop selective PRMT5-MTA complex inhibitors that specifically inhibit the proliferation of MTAP-deficient tumour cells while effectively avoiding dose-limiting toxicities mediated by wild-type PRMT5.

The published patent applications for PRMT5-MTA inhibitor compounds include WO 2021050915 A1, WO 2022192745 A1, WO 2023202626 A1, WO 2023174250 A1, WO 2024027703 A1, WO 2024114635 A1, WO 2024008176 A1, WO 2023098439 A1, WO 2023125540 A1, CN 116178347 A, WO 2021163344 A1, WO 2022115377 A1, WO 2022132914 A1, WO 2022169948 A1 and WO 2022026892 A1.

### Summary of the Invention

The object of the present disclosure is to provide a compound represented by general formula (I) or a pharmaceutically acceptable salt thereof: 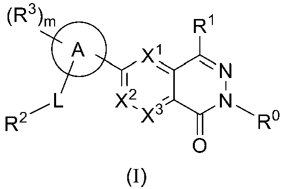 wherein:
ring A is aryl or heteroaryl;
R⁰ is selected from a hydrogen atom, alkyl, haloalkyl, deuterioalkyl, hydroxyalkyl, alkoxy, haloalkoxy, deuterioalkoxy, alkoxyalkyl, aminoalkyl, cycloalkyl, cycloalkylalkyl, heterocyclyl and heterocyclylalkyl;
R¹ is selected from -(CR^{1a}R^{1b})r-NH₂, 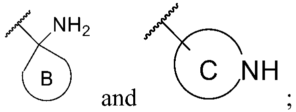
R^{1a} and R^{1b} are the same or different, and are each independently selected from a hydrogen atom, a deuterium atom, halogen, hydroxyl, cyano, amino, alkyl, haloalkyl, deuterioalkyl, hydroxyalkyl, alkoxy, haloalkoxy, deuterioalkoxy and cycloalkyl;
or R^{1a} and R^{1b} on the same carbon atom together form =O;
or R^{1a} and R^{1b} together with the carbon atom to which they are attached form cycloalkyl or heterocyclyl, wherein the cycloalkyl or heterocyclyl is optionally substituted with one or more R;
ring B is cycloalkyl or heterocyclyl, wherein the cycloalkyl or heterocyclyl is optionally substituted with one or more R;
ring C is nitrogen-containing heterocyclyl, wherein the nitrogen-containing heterocyclyl is optionally substituted with one or more R;
L is selected from a bond, alkylene, O, S(O)ᵥ, NR^{a}, C(O), NR^{a}C(O) and C(O)NR^{a};
R^{a} is selected from a hydrogen atom, alkyl, haloalkyl, hydroxyalkyl, cycloalkyl and cycloalkylalkyl;
R² is selected from tricyclic cycloalkyl, tricyclic heterocyclyl, tricyclic aryl and tricyclic heteroaryl, wherein the tricyclic cycloalkyl, tricyclic heterocyclyl, tricyclic aryl and tricyclic heteroaryl are each independently and optionally substituted with one or more R⁴;
each of R³ and R⁴ is the same or different, and is independently selected from a deuterium atom, halogen, hydroxyl, cyano, nitro, amino, alkenyl, alkynyl, alkyl, haloalkyl, deuterioalkyl, hydroxyalkyl, alkoxy, haloalkoxy, deuterioalkoxy, alkoxyalkyl, aminoalkyl, cycloalkyl, cycloalkylalkyl, cycloalkyloxy, heterocyclyl, heterocyclylalkyl, heterocyclyloxy, aryl, arylalkyl, heteroaryl, heteroarylalkyl, - (CH₂)ₜNR¹⁴R¹⁵, -OR¹⁶, -S(O)ᵥR¹⁶, -C(O)NR¹⁴R¹⁵, -C(O)R¹⁶, -C(O)OR¹⁶, - NR¹⁷C(O)R¹⁶, -S(O)ᵥNR¹⁴R¹⁵ and -NR¹⁷S(O)ᵥR¹⁶, wherein the alkyl, alkenyl, alkynyl, alkoxy, cycloalkyl, cycloalkylalkyl, cycloalkyloxy, heterocyclyl, heterocyclylalkyl, heterocyclyloxy, aryl, arylalkyl, heteroaryl and heteroarylalkyl are each independently and optionally substituted with one or more R;
or two R⁴ on the same atom together form =O;
or two R⁴ together with the ring atom on R² to which they are attached form cycloalkyl, heterocyclyl, aryl or heteroaryl, wherein the cycloalkyl, heterocyclyl, aryl or heteroaryl is optionally substituted with one or more R;
X¹ is CR^{5a} or N;
X² is CR^{5b} or N;
X³ is CR⁵ or N;
R^{5a}, R^{5b} and R⁵ are the same or different, and are each independently selected from a hydrogen atom, a deuterium atom, halogen, hydroxyl, cyano, amino, alkenyl, alkynyl, alkyl, haloalkyl, deuterioalkyl, hydroxyalkyl, alkoxy, haloalkoxy, deuterioalkoxy, alkoxyalkyl, aminoalkyl, cycloalkyl, cycloalkylalkyl, heterocyclyl, heterocyclylalkyl, aryl, arylalkyl, heteroaryl, heteroarylalkyl, -(CH₂)ₜNR¹⁴R¹⁵, - OR¹⁶, -C(O)NR¹⁴R¹⁵, -C(O)R¹⁶, -C(O)OR¹⁶ and -NR¹⁷C(O)R¹⁶, wherein the alkyl, alkenyl, alkynyl, alkoxy, cycloalkyl, cycloalkylalkyl, heterocyclyl, heterocyclylalkyl, aryl, arylalkyl, heteroaryl and heteroarylalkyl are each independently and optionally substituted with one or more R;
R¹⁴, R¹⁵ and R¹⁶ at each occurrence are the same or different, and are each independently selected from a hydrogen atom, alkyl, alkenyl, alkynyl, cycloalkyl, cycloalkylalkyl, heterocyclyl, heterocyclylalkyl, aryl and heteroaryl, wherein the alkyl, alkenyl, alkynyl, cycloalkyl, cycloalkylalkyl, heterocyclyl, heterocyclylalkyl, aryl and heteroaryl are each independently and optionally substituted with one or more R;
or R¹⁴ and R¹⁵ together with the nitrogen atom to which they are attached form heterocyclyl, wherein the heterocyclyl is optionally substituted with one or more R;
each R¹⁷ at each occurrence is the same or different, and is independently selected from a hydrogen atom, alkyl, cycloalkyl and cycloalkylalkyl;
each R at each occurrence is the same or different, and is independently selected from oxo, a deuterium atom, halogen, hydroxyl, alkenyl, alkynyl, cyano, nitro, amino, -NH alkyl, -N(alkyl)₂, alkyl, haloalkyl, deuterioalkyl, hydroxyalkyl, alkoxy, haloalkoxy, deuterioalkoxy, alkoxyalkyl, aminoalkyl, cycloalkyl, cycloalkylalkyl, cycloalkyloxy, heterocyclyl, heterocyclylalkyl, heterocyclyloxy, aryl, arylalkyl, aryloxy, heteroaryl, heteroarylalkyl and heteroaryloxy;
m is 0, 1, 2, 3 or 4;
r is 0, 1, 2 or 3;
v is 0, 1 or 2; and
t is 0, 1, 2 or 3.

In some embodiments of the present disclosure, provided are the compounds represented by general formula (I), general formula (II), general formula (III), general formula (III-1), general formula (III-2) or general formula (IV), or the pharmaceutically acceptable salts thereof, which are not any of the compounds disclosed in WO 2024027703 A1 and WO 2023202626 A1.

In some embodiments of the present disclosure, provided are the compounds represented by general formula (I), general formula (II), general formula (III), general formula (III-1), general formula (III-2) or general formula (IV) or the pharmaceutically acceptable salts thereof, which do not have the following structures:

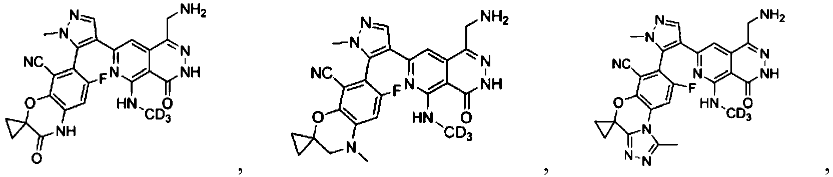

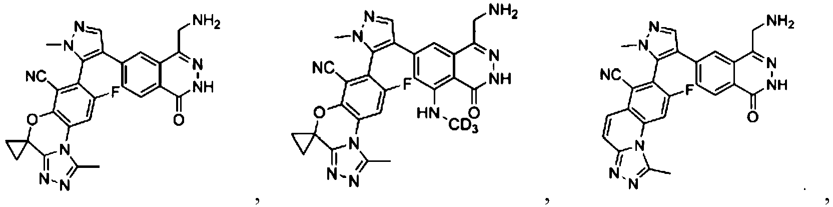

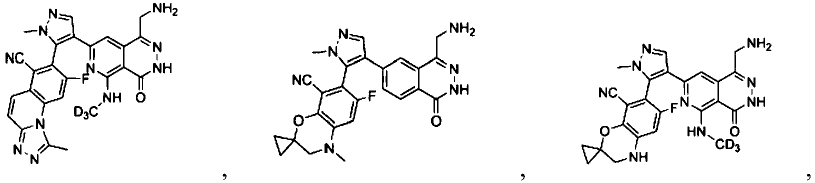

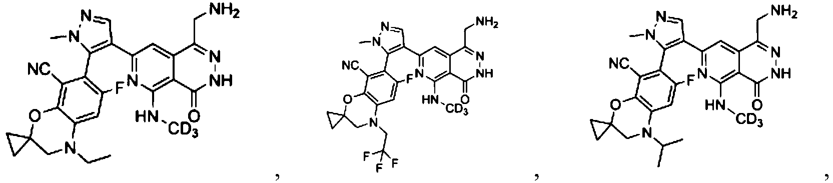

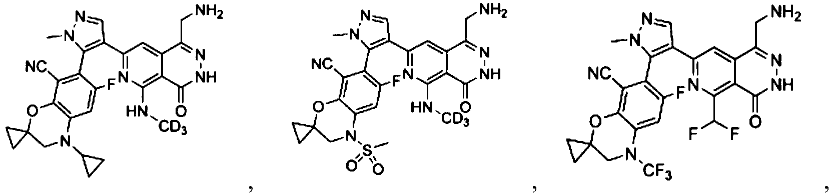

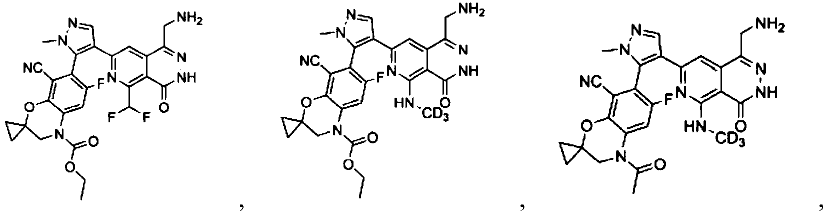

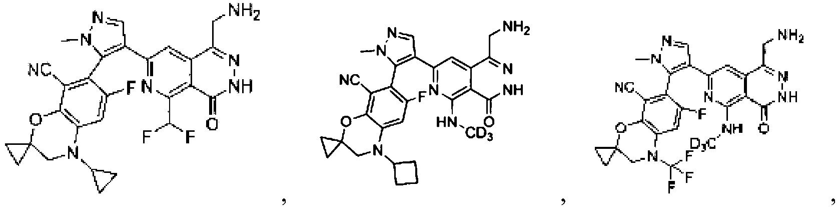

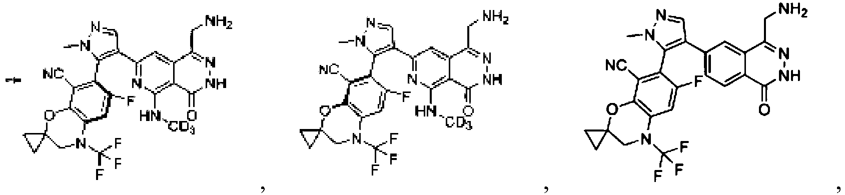

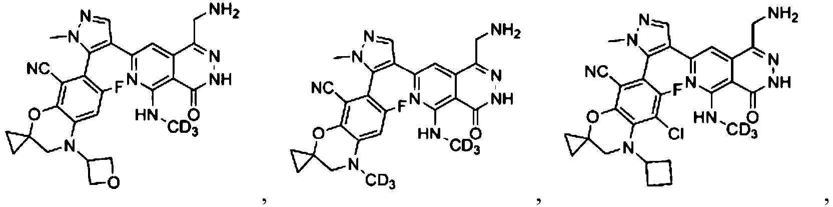

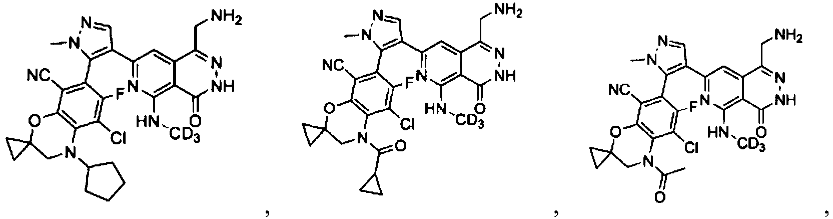

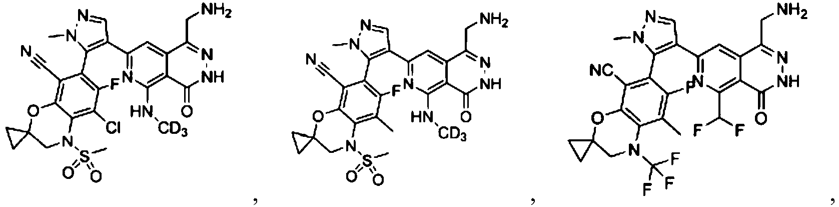

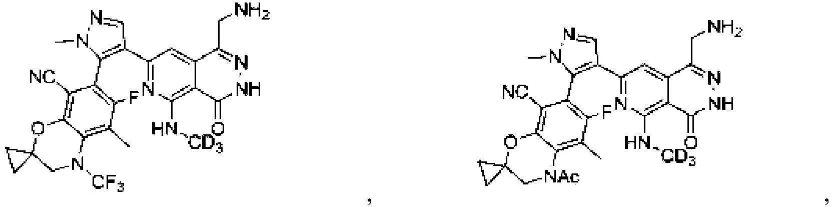

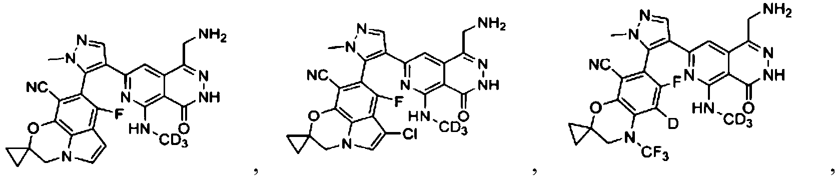

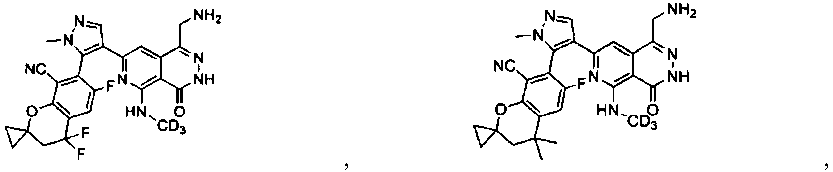

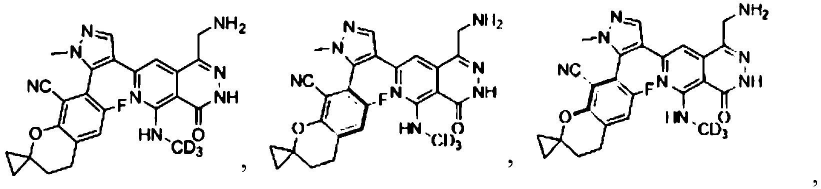

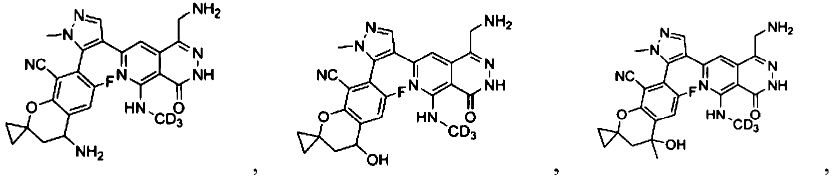

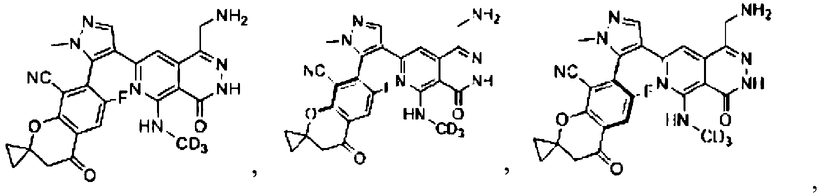

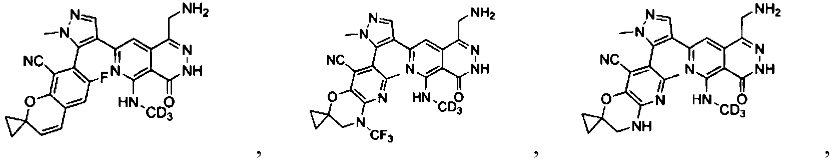

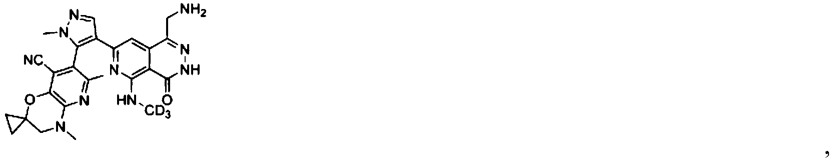

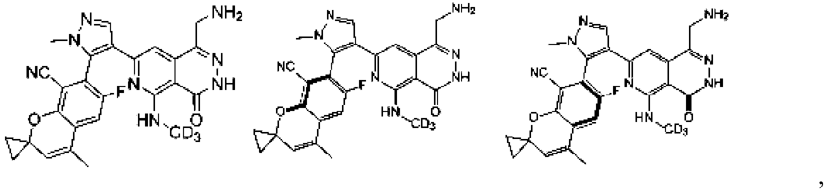

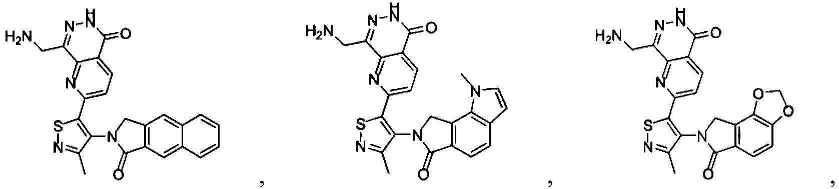

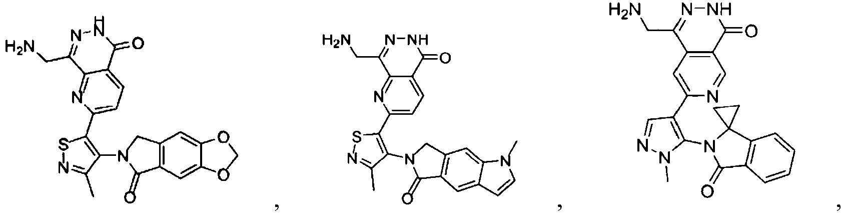

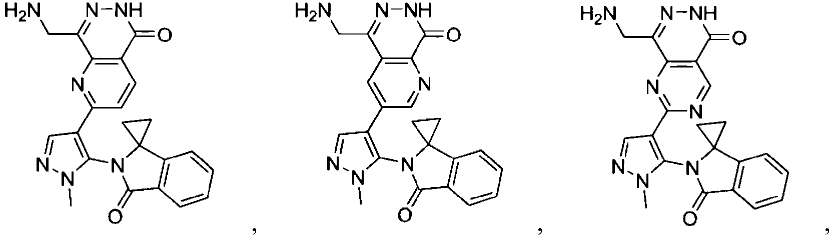

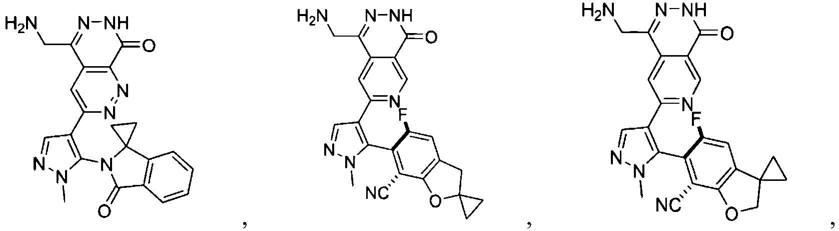

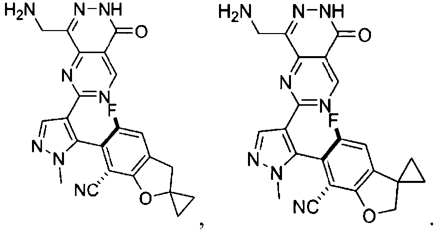

In some embodiments of the present disclosure, provided is the compound represented by general formula (I) or the pharmaceutically acceptable salt thereof, wherein ring A is 6- to 10-membered aryl or 5- to 10-membered heteroaryl; in some embodiments, ring A is 5- to 10-membered heteroaryl; in some embodiments, ring A is 5- or 6-membered heteroaryl; in some embodiments, ring A is 5-membered heteroaryl; in some embodiments, ring A is triazolyl or pyrazolyl; in some embodiments, ring A is pyrazolyl.

In some embodiments of the present disclosure, provided is the compound represented by general formula (I) or the pharmaceutically acceptable salt thereof, wherein 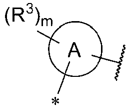 is 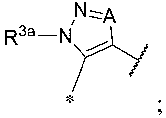 R^{3a} is selected from a hydrogen atom, alkenyl, alkynyl, alkyl, haloalkyl, deuterioalkyl, hydroxyalkyl, alkoxyalkyl, aminoalkyl, cycloalkyl, cycloalkylalkyl, heterocyclyl, heterocyclylalkyl, aryl, arylalkyl, heteroaryl and heteroarylalkyl, wherein the alkyl, alkenyl, alkynyl, cycloalkyl, cycloalkylalkyl, heterocyclyl, heterocyclylalkyl, aryl, arylalkyl, heteroaryl and heteroarylalkyl are each independently and optionally substituted with one or more R; A is N or CR^{3b}; R^{3b} is a hydrogen atom or R³; R and R³ are as defined in general formula (I); in some embodiments, 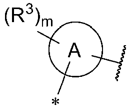 is 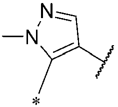 or 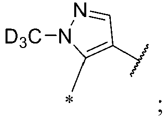 in some embodiments, 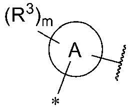 is 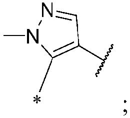 in some embodiments, 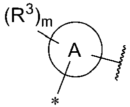 is 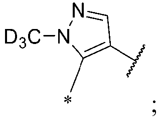 the * end is attached to L.

In some embodiments of the present disclosure, provided is the compound represented by general formula (I) or the pharmaceutically acceptable salt thereof, wherein each R³ is the same or different, and is independently selected from a deuterium atom, halogen, hydroxyl, cyano, C₁₋₆ alkyl, C₁₋₆ haloalkyl, C₁₋₆ deuterioalkyl and 3- to 8-membered cycloalkyl; in some embodiments, each R³ is the same or different, and is independently C₁₋₆ alkyl or C₁₋₆ deuterioalkyl; in some embodiments, each R³ is the same or different, and is independently selected from C₁₋₆ alkyl, C₁₋₆ haloalkyl and C₁₋₆ deuterioalkyl; in some embodiments, each R³ is the same or different, and is independently selected from halogen, C₁₋₆ alkyl and C₁₋₆ haloalkyl; in some embodiments, each R³ is the same or different, and is independently halogen or C₁₋₆ alkyl; in some embodiments, R³ is C₁₋₆ alkyl; in some embodiments, R³ is C₁₋₆ deuterioalkyl; in some embodiments, R³ is methyl or trideuteriomethyl; in some embodiments, R³ is methyl; in some embodiments, R³ is trideuteriomethyl.

In some embodiments of the present disclosure, provided is the compound represented by general formula (I) or the pharmaceutically acceptable salt thereof, wherein m is 1.

In some embodiments of the present disclosure, provided is the compound represented by general formula (I) or the pharmaceutically acceptable salt thereof, wherein R³ is C₁₋₆ alkyl or C₁₋₆ deuterioalkyl; and m is 1; in some embodiments, R³ is C₁₋₆ deuterioalkyl; and m is 1.

In some embodiments of the present disclosure, provided is the compound represented by general formula (I) or the pharmaceutically acceptable salt thereof, wherein L is a bond.

In some embodiments of the present disclosure, provided is the compound represented by general formula (I) or the pharmaceutically acceptable salt thereof, wherein R¹ is-(CR^{1a}R )r-NH₂; R^{1a}, R^{1b} and r are as defined in general formula (I); in some embodiments, R¹ is -CH₂-NH₂.

In some embodiments of the present disclosure, provided is the compound represented by general formula (I) or the pharmaceutically acceptable salt thereof, wherein r is 1 or 2; in some embodiments, r is 1.

In some embodiments of the present disclosure, provided is the compound represented by general formula (I) or the pharmaceutically acceptable salt thereof, wherein ring B is 3- to 6-membered cycloalkyl or 3- to 6-membered heterocyclyl; in some embodiments, ring B is selected from cyclopropyl, cyclobutyl and oxetanyl.

In some embodiments of the present disclosure, provided is the compound represented by general formula (I) or the pharmaceutically acceptable salt thereof, wherein R¹ is 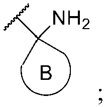 ring B is as defined in general formula (I); in some embodiments, R¹ is selected from

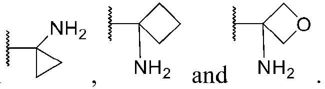

In some embodiments of the present disclosure, provided is the compound represented by general formula (I) or the pharmaceutically acceptable salt thereof, wherein ring C is 3- to 6-membered nitrogen-containing heterocyclyl; in some embodiments, ring C is azetidinyl; in some embodiments, ring C is

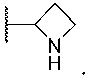

In some embodiments of the present disclosure, provided is the compound represented by general formula (I) or the pharmaceutically acceptable salt thereof, wherein R¹ is 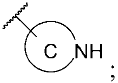 ring C is as defined in general formula (I); in some embodiments, R¹ is

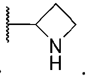

In some embodiments of the present disclosure, provided is the compound represented by general formula (I) or the pharmaceutically acceptable salt thereof, wherein R¹ is selected from -CH₂-NH₂,

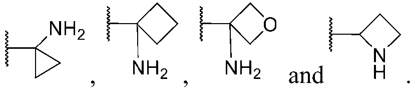

In some embodiments of the present disclosure, provided is the compound represented by general formula (I) or the pharmaceutically acceptable salt thereof, wherein X² is CR^{5b}; R^{5b} is as defined in general formula (I); in some embodiments, X² is CH.

In some embodiments of the present disclosure, provided is the compound represented by general formula (I) or the pharmaceutically acceptable salt thereof, wherein R^{5b} is selected from a hydrogen atom, a deuterium atom, halogen, cyano, C₁₋₆ alkyl, C₁₋₆ haloalkyl, C₁₋₆ deuterioalkyl, C₁₋₆ alkoxy, C₁₋₆ haloalkoxy, C₁₋₆ deuterioalkoxy and 3- to 6-membered cycloalkyl; in some embodiments, R^{5b} is selected from a hydrogen atom, halogen, cyano, C₁₋₆ alkyl, C₁₋₆ haloalkyl, C₁₋₆ alkoxy and 3- to 6-membered cycloalkyl; in some embodiments, R^{5b} is selected from a hydrogen atom, halogen and C₁₋₆ alkyl; in some embodiments, R^{5b} is a hydrogen atom.

In some embodiments of the present disclosure, provided is the compound represented by general formula (I) or the pharmaceutically acceptable salt thereof, wherein X³ is CR⁵; R⁵ is as defined in general formula (I); in some embodiments, X³ is CH.

In some embodiments of the present disclosure, provided is the compound represented by general formula (I) or the pharmaceutically acceptable salt thereof, wherein X¹ is CR^{5a} or N; X² is CR^{5b}; X³ is CR⁵; R^{5a}, R^{5b} and R⁵ are as defined in general formula (I); in some embodiments, X¹ is CR^{5a}; X² is CR^{5b}; X³ is CR⁵; R^{5a}, R^{5b} and R⁵ are as defined in general formula (I); in some embodiments, X¹ is CH; X² is CH; X³ is CR⁵; R⁵ is as defined in general formula (I); in some embodiments, X¹ is CH; X² is CH; X³ is CH; in some embodiments, X¹ is CH; X² is CH; X³ is CH or C-CN; in some embodiments, X¹ is CH; X² is CH; X³ is C-CN.

In some embodiments of the present disclosure, provided is the compound represented by general formula (I) or the pharmaceutically acceptable salt thereof, wherein R⁰ is a hydrogen atom or C₁₋₆ alkyl; in some embodiments, R⁰ is a hydrogen atom.

In some embodiments of the present disclosure, provided is the compound represented by general formula (I) or the pharmaceutically acceptable salt thereof, which is a compound represented by general formula (II) or a pharmaceutically acceptable salt thereof: 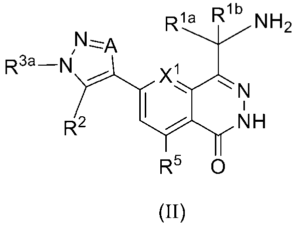 wherein:
R^{3a} is selected from a hydrogen atom, alkenyl, alkynyl, alkyl, haloalkyl, deuterioalkyl, hydroxyalkyl, alkoxyalkyl, aminoalkyl, cycloalkyl, cycloalkylalkyl, heterocyclyl, heterocyclylalkyl, aryl, arylalkyl, heteroaryl and heteroarylalkyl, wherein the alkyl, alkenyl, alkynyl, cycloalkyl, cycloalkylalkyl, heterocyclyl, heterocyclylalkyl, aryl, arylalkyl, heteroaryl and heteroarylalkyl are each independently and optionally substituted with one or more R;
A is N or CR^{3b};
R^{3b} is a hydrogen atom or R³;
X¹, R, R^{1a}, R^{1b}, R², R³ and R⁵ are as defined in general formula (I).

In some embodiments of the present disclosure, provided are the compounds represented by general formula (I) or general formula (II) or the pharmaceutically acceptable salts thereof, which are compounds represented by general formula (III) or pharmaceutically acceptable salts thereof: 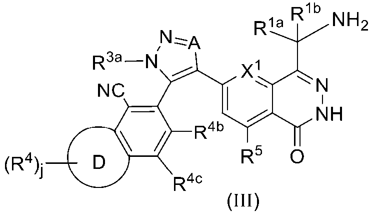 wherein:
ring D is selected from bicyclic cycloalkyl, bicyclic heterocyclyl, bicyclic aryl and bicyclic heteroaryl;
R^{4b} and R^{4c} are the same or different, and are each independently a hydrogen atom or R⁴;
j is 0, 1, 2 or 3;
A, X¹, R^{1a}, R^{1b}, R^{3a}, R⁴ and R⁵ are as defined in general formula (II).

In some embodiments of the present disclosure, provided are the compounds represented by general formula (I), general formula (II) or general formula (III) or the pharmaceutically acceptable salts thereof, which are compounds represented by general formula (III-1) or general formula (III-2) or pharmaceutically acceptable salts thereof: 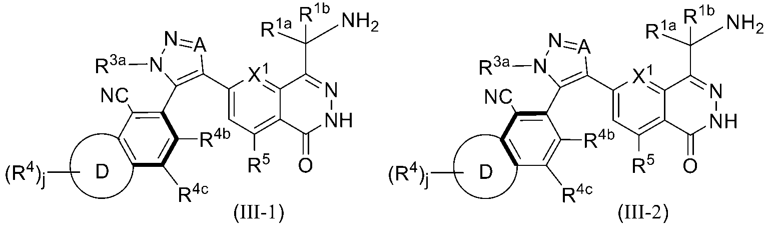 wherein:
ring D, A, X¹, R^{1a}, R^{1b}, R^{3a}, R⁴, R^{4b}, R^{4c}, R⁵ and j are as defined in general formula (III).

In some embodiments of the present disclosure, provided are the compounds represented by general formula (III), general formula (III-1) or general formula (III-2) or the pharmaceutically acceptable salts thereof, wherein ring D is 7- to 9-membered bicyclic heterocyclyl; in some embodiments, ring D is 8- or 9-membered bicyclic heterocyclyl; in some embodiments, ring D is 8-membered bicyclic heterocyclyl;
in some embodiments, ring D is selected from 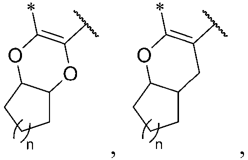 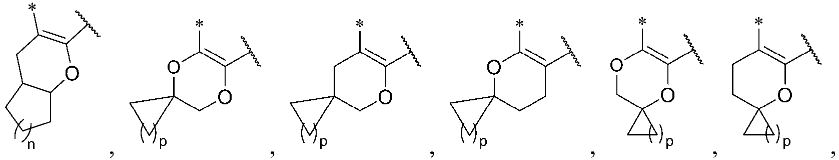 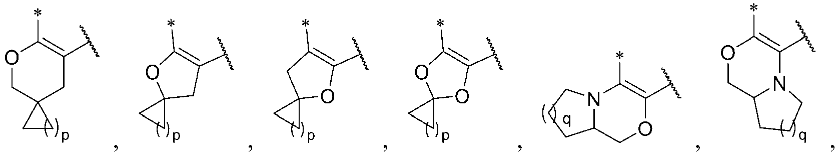 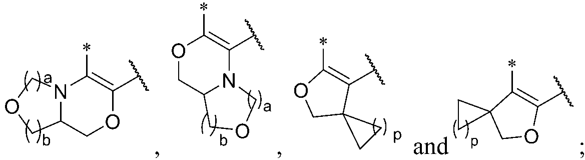 n is 0, 1 or 2 (in some embodiments, 0 or 1); p is 1, 2 or 3 (in some embodiments, 1); q is 1 or 2 (in some embodiments, 1); a is 1 or 2 (in some embodiments, 2); b is 1 or 2 (in some embodiments, 1);
in some embodiments, ring D is selected from 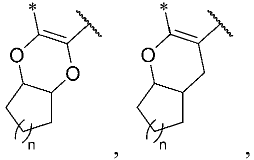 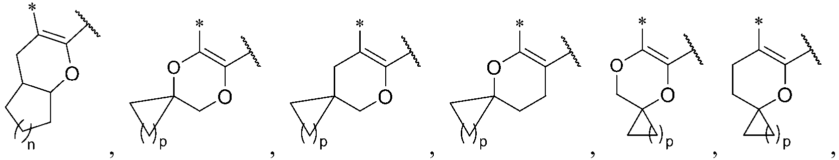 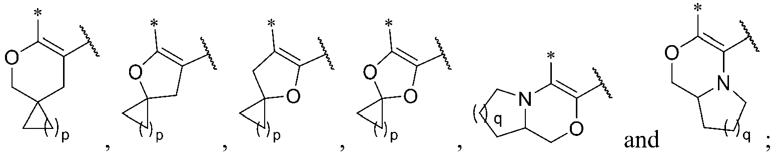 n is 0, 1 or 2 (in some embodiments, 0 or 1); p is 1, 2 or 3 (in some embodiments, 1); q is 1 or 2 (in some embodiments, 1);
in some embodiments, ring D is selected from 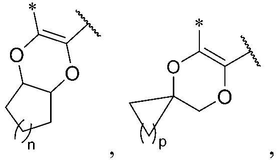 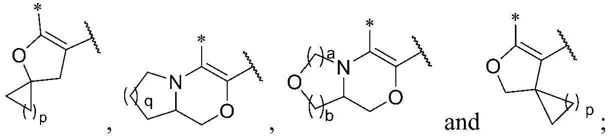 a is 1 or 2 (in some embodiments, 2); b is 1 or 2 (in some embodiments, 1); n is 0, 1 or 2 (in some embodiments, 0 or 1); p is 1, 2 or 3 (in some embodiments, 1); q is 1 or 2 (in some embodiments, 1); in some embodiments, ring D is selected from 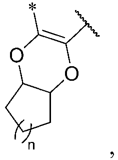 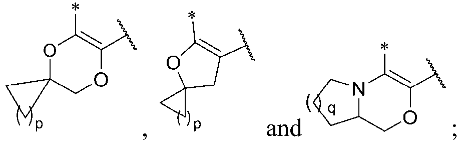 n is 0, 1 or 2 (in some embodiments, 0 or 1); p is 1, 2 or 3 (in some embodiments, 1); q is 1 or 2 (in some embodiments, 1); in some embodiments, ring D is 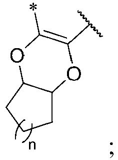 n is 0, 1 or 2 (in some embodiments, 0 or 1; in some embodiments, 1); in some embodiments, ring D is selected from 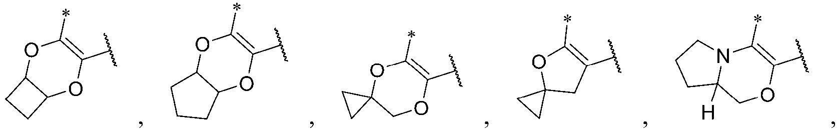 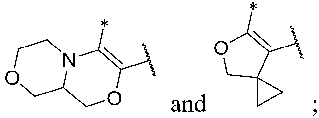 in some embodiments, ring D is selected from 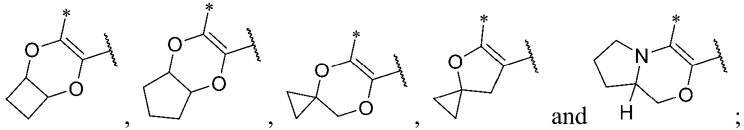 in some embodiments, ring D is selected from 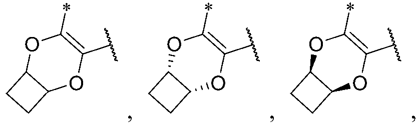 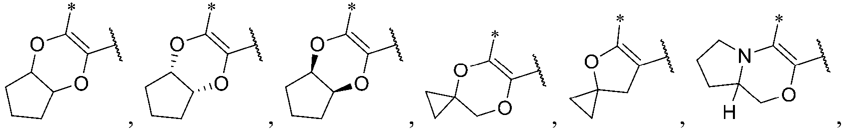 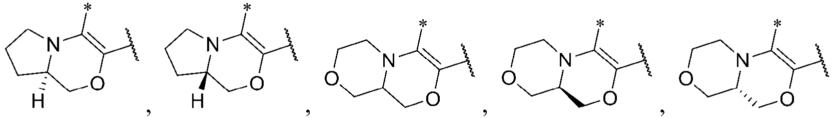 and 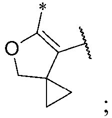 in some embodiments, ring D is selected from 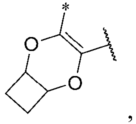 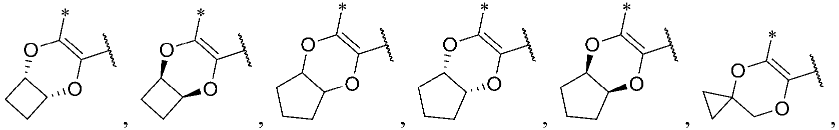 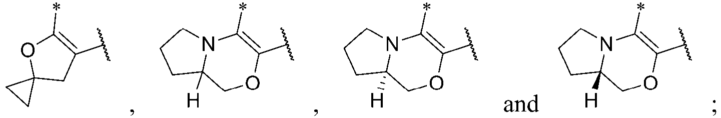 in some embodiments, ring D is selected from 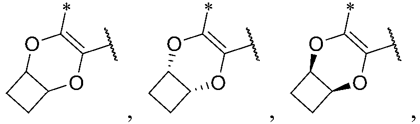 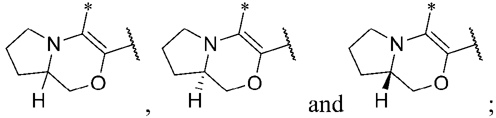 in some embodiments, ring D is selected from 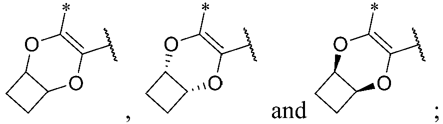 in some embodiments, ring D is 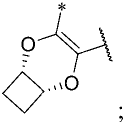 in some embodiments, ring D is 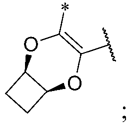 the * end is attached to the side proximal to the cyano, and ring D can be substituted with R⁴ at any substitutable position.

In some embodiments of the present disclosure, provided are the compounds represented by general formula (I), general formula (II), general formula (III), general formula (III-1) or general formula (III-2) or the pharmaceutically acceptable salts thereof, wherein R^{1a} and R^{1b} are the same or different, and are each independently selected from a hydrogen atom, a deuterium atom, halogen, C₁₋₆ alkyl and C₁₋₆ haloalkyl; or R^{1a} and R^{1b} on the same carbon atom together form =O; in some embodiments, R^{1a} and R^{1b} are the same or different, and are each independently a hydrogen atom or a deuterium atom; or R^{1a} and R^{1b} on the same carbon atom together form =O; in some embodiments, R^{1a} and R^{1b} are the same or different, and are each independently a hydrogen atom or a deuterium atom; in some embodiments, R^{1a} and R^{1b} are hydrogen atoms; in some embodiments, R^{1a} and R^{1b} are deuterium atoms; in some embodiments, R^{1a} and R^{1b} on the same carbon atom together form =O.

In some embodiments of the present disclosure, provided are the compounds represented by general formula (I), general formula (II), general formula (III), general formula (III-1) or general formula (III-2) or the pharmaceutically acceptable salts thereof, wherein X¹ is CR^{5a}; R^{5a} is as defined in general formula (I); in some embodiments, X¹ is CR^{5a}; R^{5a} is selected from a hydrogen atom, halogen and C₁₋₆ alkyl; in some embodiments, X¹ is CH.

In some embodiments of the present disclosure, provided are the compounds represented by general formula (I), general formula (II), general formula (III), general formula (III-1) or general formula (III-2) or the pharmaceutically acceptable salts thereof, wherein R^{5a} is selected from a hydrogen atom, a deuterium atom, halogen, cyano, C₁₋₆ alkyl, C₁₋₆ haloalkyl, C₁₋₆ deuterioalkyl, C₁₋₆ alkoxy, C₁₋₆ haloalkoxy, C₁₋₆ deuterioalkoxy and 3- to 6-membered cycloalkyl; in some embodiments, R^{5a} is selected from a hydrogen atom, halogen, cyano, C₁₋₆ alkyl, C₁₋₆ haloalkyl, C₁₋₆ alkoxy and 3- to 6-membered cycloalkyl; in some embodiments, R^{5a} is selected from a hydrogen atom, halogen and C₁₋₆ alkyl; in some embodiments, R^{5a} is a hydrogen atom.

In some embodiments of the present disclosure, provided are the compounds represented by general formula (I), general formula (II), general formula (III), general formula (III-1) or general formula (III-2) or the pharmaceutically acceptable salts thereof, wherein A is CR^{3b}; R^{3b} is as defined in general formula (II); in some embodiments, A is CR^{3b}; R^{3b} is selected from a hydrogen atom, halogen and C₁₋₆ alkyl; in some embodiments, A is CH.

In some embodiments of the present disclosure, provided are the compounds represented by general formula (I), general formula (II), general formula (III), general formula (III-1) or general formula (III-2) or the pharmaceutically acceptable salts thereof, wherein R³ is selected from a deuterium atom, halogen, hydroxyl, cyano, C₁₋₆ alkyl, C₁₋₆ haloalkyl, C₁₋₆ deuterioalkyl and 3- to 8-membered cycloalkyl; in some embodiments, R³ is halogen or C₁₋₆ alkyl; in some embodiments, R³ is C₁₋₆ alkyl.

In some embodiments of the present disclosure, provided are the compounds represented by general formula (I), general formula (II), general formula (III), general formula (III-1) or general formula (III-2) or the pharmaceutically acceptable salts thereof, wherein R^{3b} is selected from a hydrogen atom, a deuterium atom, halogen, hydroxyl, cyano, C₁₋₆ alkyl, C₁₋₆ haloalkyl, C₁₋₆ deuterioalkyl and 3- to 8-membered cycloalkyl; in some embodiments, R^{3b} is selected from a hydrogen atom, halogen and C₁₋₆ alkyl; in some embodiments, R^{3b} is a hydrogen atom or C₁₋₆ alkyl; in some embodiments, R^{3b} is a hydrogen atom.

In some embodiments of the present disclosure, provided are the compounds represented by general formula (II), general formula (III), general formula (III-1) or general formula (III-2) or the pharmaceutically acceptable salts thereof, wherein X¹ is CH; and/or A is CH.

In some embodiments of the present disclosure, provided are the compounds represented by general formula (I) or general formula (II) or the pharmaceutically acceptable salts thereof, which are compounds represented by general formula (IV) or pharmaceutically acceptable salts thereof: 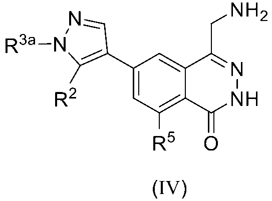 wherein:
R², R^{3a} and R⁵ are as defined in general formula (II).

In some embodiments of the present disclosure, provided are the compounds represented by general formula (I), general formula (II) or general formula (IV) or the pharmaceutically acceptable salts thereof, wherein R² is 10- to 14-membered tricyclic aryl or 9- to 14-membered tricyclic heteroaryl, wherein the 10- to 14-membered tricyclic aryl and 9- to 14-membered tricyclic heteroaryl are each independently and optionally substituted with one or more R⁴; R⁴ is as defined in general formula (I); in some embodiments, R² is 10- to 14-membered tricyclic aryl, wherein the 10- to 14-membered tricyclic aryl is optionally substituted with one or more R⁴; R⁴ is as defined in general formula (I); in some embodiments, R² is 9- to 14-membered tricyclic heteroaryl; wherein the 9- to 14-membered tricyclic heteroaryl is optionally substituted with one or more R⁴; R⁴ is as defined in general formula (I); in some embodiments, R² is 11- to 13-membered tricyclic aryl, wherein the 11- to 13-membered tricyclic aryl is optionally substituted with one or more R⁴; R⁴ is as defined in general formula (I).

In some embodiments of the present disclosure, provided are the compounds represented by general formula (I), general formula (II) or general formula (IV) or the pharmaceutically acceptable salts thereof, wherein R² is selected from 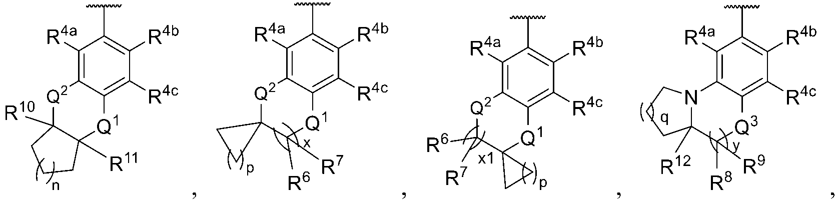 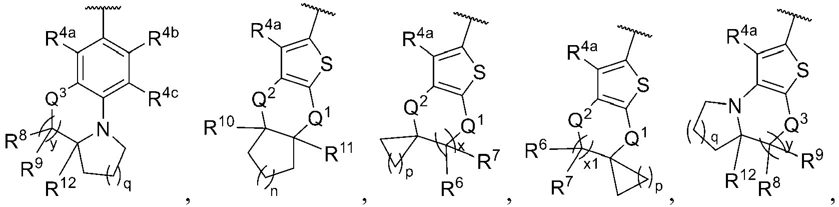 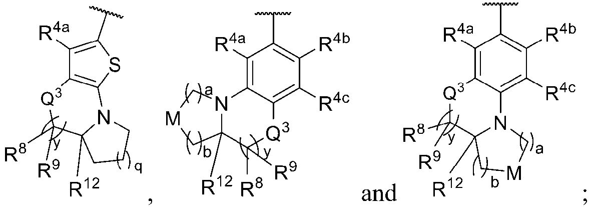 in some embodiments, R² is selected from 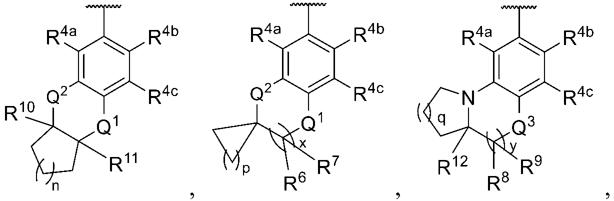 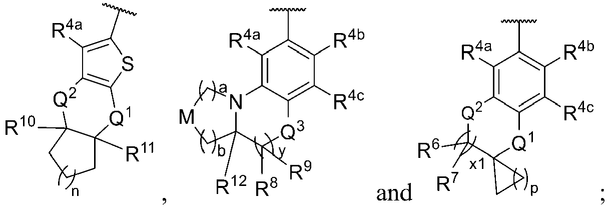
wherein: M is selected from CR⁴R⁴, O, S and NR^{m}; R^{m} is selected from a hydrogen atom, alkyl, haloalkyl, deuterioalkyl, hydroxyalkyl, alkoxyalkyl, aminoalkyl, cycloalkyl, cycloalkylalkyl, heterocyclyl and heterocyclylalkyl; Q¹, Q² and Q³ are the same or different, and are each independently selected from a bond, O, S(O)ᵥ, CR^{4d}R^{4e}, C(O) and NR^{4f}; R^{4a}, R^{4b}, R^{4c}, R^{4d} and R^{4e} are the same or different, and are each independently a hydrogen atom or R⁴; or R^{4d} and R^{4e} together with the carbon atom to which they are attached form cycloalkyl or heterocyclyl, wherein the cycloalkyl or heterocyclyl is optionally substituted with one or more R;
R^{4f} is selected from a hydrogen atom, alkyl, haloalkyl, deuterioalkyl, hydroxyalkyl, alkoxyalkyl, aminoalkyl, cycloalkyl, cycloalkylalkyl, heterocyclyl and heterocyclylalkyl;
R⁶, R⁷, R⁸, R⁹, R¹⁰, R¹¹ and R¹² are the same or different, and are each independently a hydrogen atom or R⁴; or R⁶ and R⁷ together with the carbon atom to which they are attached form cycloalkyl or heterocyclyl, wherein the cycloalkyl or heterocyclyl is optionally substituted with one or more R; or R⁸ and R⁹ together with the carbon atom to which they are attached form cycloalkyl or heterocyclyl, wherein the cycloalkyl or heterocyclyl is optionally substituted with one or more R; or R⁶ and R⁷ on the same carbon atom together form =O; or R⁸ and R⁹ on the same carbon atom together form =O; n is 0, 1, 2, 3 or 4; p is 1, 2, 3, 4, 5 or 6; q is 0, 1, 2, 3 or 4; x is 0, 1, 2, 3, 4 or 5; x1 is 1, 2, 3, 4 or 5; y is 0, 1, 2, 3 or 4; a is 0, 1, 2, 3 or 4; b is 0, 1, 2, 3 or 4; R⁴, R and v are as defined in general formula (I).

In some embodiments of the present disclosure, provided are the compounds represented by general formula (I), general formula (II) or general formula (IV) or the pharmaceutically acceptable salts thereof, wherein R² is selected from 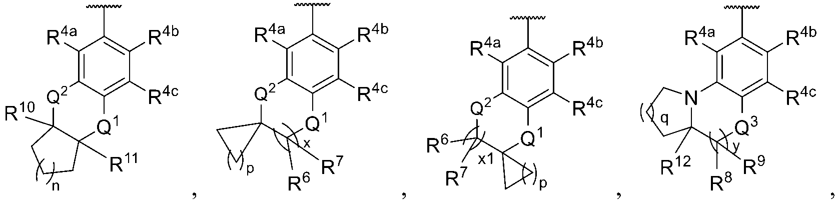 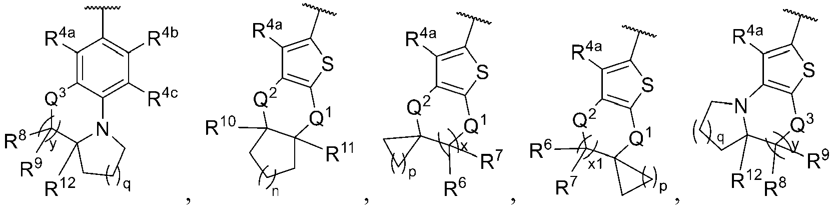 and 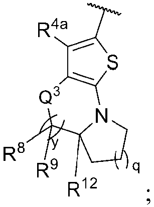 in some embodiments, R² is selected from 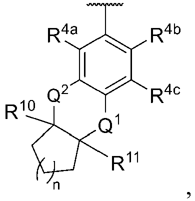 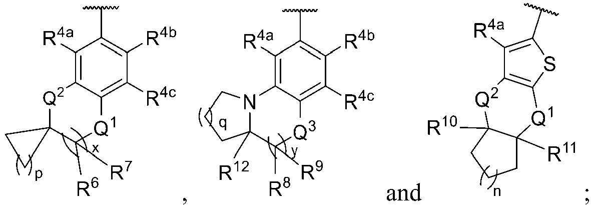 in some embodiments, R² is 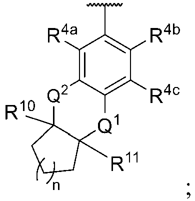
wherein: Q¹, Q² and Q³ are the same or different, and are each independently selected from a bond, O, S(O)ᵥ, CR^{4d}R^{4e}, C(O) and NR^{4f}; R^{4a}, R^{4b}, R^{4c}, R^{4d} and R^{4e} are the same or different, and are each independently a hydrogen atom or R⁴; or R^{4d} and R^{4e} together with the carbon atom to which they are attached form cycloalkyl or heterocyclyl, wherein the cycloalkyl or heterocyclyl is optionally substituted with one or more R;
R^{4f} is selected from a hydrogen atom, alkyl, haloalkyl, deuterioalkyl, hydroxyalkyl, alkoxyalkyl, aminoalkyl, cycloalkyl, cycloalkylalkyl, heterocyclyl and heterocyclylalkyl;
R⁶, R⁷, R⁸, R⁹, R¹⁰, R¹¹ and R¹² are the same or different, and are each independently a hydrogen atom or R⁴; or R⁶ and R⁷ together with the carbon atom to which they are attached form cycloalkyl or heterocyclyl, wherein the cycloalkyl or heterocyclyl is optionally substituted with one or more R; or R⁸ and R⁹ together with the carbon atom to which they are attached form cycloalkyl or heterocyclyl, wherein the cycloalkyl or heterocyclyl is optionally substituted with one or more R; or R⁶ and R⁷ on the same carbon atom together form =O; or R⁸ and R⁹ on the same carbon atom together form =O; n is 0, 1, 2, 3 or 4; p is 1, 2, 3, 4, 5 or 6; q is 0, 1, 2, 3 or 4; x is 0, 1, 2, 3, 4 or 5; x1 is 1, 2, 3, 4 or 5; y is 0, 1, 2, 3 or 4; R⁴, R and v are as defined in general formula (I).

In some embodiments of the present disclosure, provided are the compounds represented by general formula (I), general formula (II), general formula (III), general formula (III-1), general formula (III-2) or general formula (IV) or the pharmaceutically acceptable salts thereof, wherein each R⁴ is the same or different, and is independently selected from a deuterium atom, halogen, hydroxyl, cyano, C₁₋₆ alkyl, C₁₋₆ haloalkyl, C₁₋₆ deuterioalkyl, C₁₋₆ hydroxyalkyl, C₁₋₆ alkoxy C₁₋₆ alkyl, C₁₋₆ alkoxy, C₁₋₆ haloalkoxy, C₁₋₆ deuterioalkoxy, 3- to 8-membered cycloalkyl, 3- to 8-membered cycloalkyloxy, 3- to 8-membered heterocyclyl and 3-to 8-membered heterocyclyloxy; in some embodiments, each R⁴ is the same or different, and is independently selected from halogen, cyano, C₁₋₆ alkyl and C₁₋₆ haloalkyl; in some embodiments, each R⁴ is the same or different, and is independently selected from halogen, C₁₋₆ alkyl and C₁₋₆ haloalkyl; in some embodiments, R⁴ is halogen or cyano; in some embodiments, R⁴ is selected from F, Cl and cyano; in some embodiments, R⁴ is F or cyano.

In some embodiments of the present disclosure, provided are the compounds represented by general formula (I), general formula (II) or general formula (IV) or the pharmaceutically acceptable salts thereof, wherein R^{4a} is cyano.

In some embodiments of the present disclosure, provided are the compounds represented by general formula (III), general formula (III-1) or general formula (III-2) or the pharmaceutically acceptable salts thereof, wherein j is 0 or 1; in some embodiments, j is 0.

In some embodiments of the present disclosure, provided are the compounds represented by general formula (I), general formula (II) or general formula (IV) or the pharmaceutically acceptable salts thereof, wherein R⁶, R⁷, R⁸ and R⁹ are the same or different, and are each independently selected from a hydrogen atom, halogen and C₁₋₆ alkyl; in some embodiments, R⁶, R⁷, R⁸ and R⁹ are each independently a hydrogen atom.

In some embodiments of the present disclosure, provided are the compounds represented by general formula (I), general formula (II) or general formula (IV) or the pharmaceutically acceptable salts thereof, wherein R¹⁰, R¹¹ and R¹² are each independently a hydrogen atom.

In some embodiments of the present disclosure, provided are the compounds represented by general formula (I), general formula (II) or general formula (IV) or the pharmaceutically acceptable salts thereof, wherein x is 0 or 1; in some embodiments, x is 1; in some embodiments, x is 0.

In some embodiments of the present disclosure, provided are the compounds represented by general formula (I), general formula (II) or general formula (IV) or the pharmaceutically acceptable salts thereof, wherein x1 is 1 or 2; in some embodiments, x1 is 1.

In some embodiments of the present disclosure, provided are the compounds represented by general formula (I), general formula (II) or general formula (IV) or the pharmaceutically acceptable salts thereof, wherein y is 0 or 1; in some embodiments, y is 1; in some embodiments, y is 0.

In some embodiments of the present disclosure, provided are the compounds represented by general formula (I), general formula (II) or general formula (IV) or the pharmaceutically acceptable salts thereof, wherein M is selected from NH, O and S; in some embodiments, M is O.

In some embodiments of the present disclosure, provided are the compounds represented by general formula (I), general formula (II) or general formula (IV) or the pharmaceutically acceptable salts thereof, wherein a is 1 or 2; in some embodiments, a is 2.

In some embodiments of the present disclosure, provided are the compounds represented by general formula (I), general formula (II) or general formula (IV) or the pharmaceutically acceptable salts thereof, wherein b is 1 or 2; in some embodiments, b is 1.

In some embodiments of the present disclosure, provided are the compounds represented by general formula (I), general formula (II) or general formula (IV) or the pharmaceutically acceptable salts thereof, wherein a is 2; b is 1.

In some embodiments of the present disclosure, provided are the compounds represented by general formula (I), general formula (II) or general formula (IV) or the pharmaceutically acceptable salts thereof, wherein R^{m} is a hydrogen atom or C₁₋₆ alkyl; in some embodiments, R^{m} is a hydrogen atom.

In some embodiments of the present disclosure, provided are the compounds represented by general formula (I), general formula (II) or general formula (IV) or the pharmaceutically acceptable salts thereof, wherein Q¹ is selected from CH₂, O and a bond; in some embodiments, Q¹ is O or a bond; in some embodiments, Q¹ is O; in some embodiments, Q¹ is a bond.

In some embodiments of the present disclosure, provided are the compounds represented by general formula (I), general formula (II) or general formula (IV) or the pharmaceutically acceptable salts thereof, wherein Q² is selected from CH₂, O and a bond; in some embodiments, Q² is O or a bond; in some embodiments, Q² is O; in some embodiments, Q² is a bond.

In some embodiments of the present disclosure, provided are the compounds represented by general formula (I), general formula (II) or general formula (IV) or the pharmaceutically acceptable salts thereof, wherein Q¹ is a bond or O; Q² is O; in some embodiments, Q¹ is O; Q² is O; in some embodiments, Q¹ is a bond; Q² is O.

In some embodiments of the present disclosure, provided are the compounds represented by general formula (I), general formula (II) or general formula (IV) or the pharmaceutically acceptable salts thereof, wherein Q³ is O.

In some embodiments of the present disclosure, provided are the compounds represented by general formula (I), general formula (II) or general formula (IV) or the pharmaceutically acceptable salts thereof, wherein R² is selected from 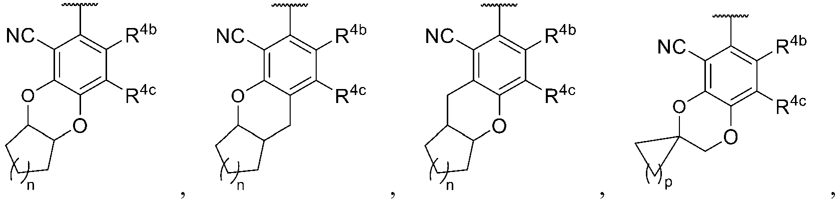 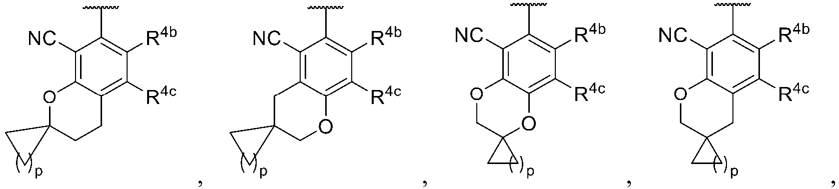 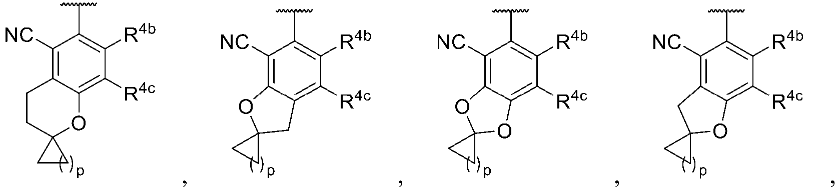 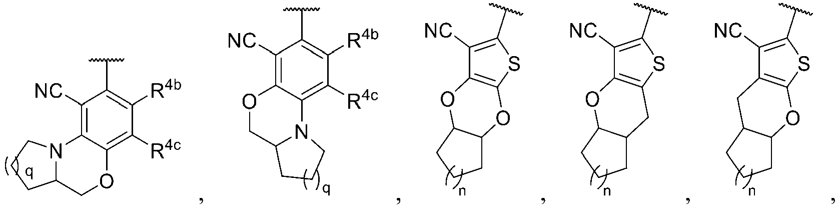 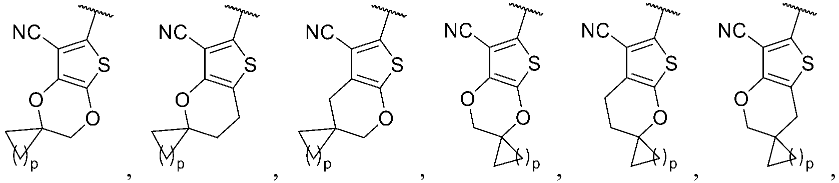 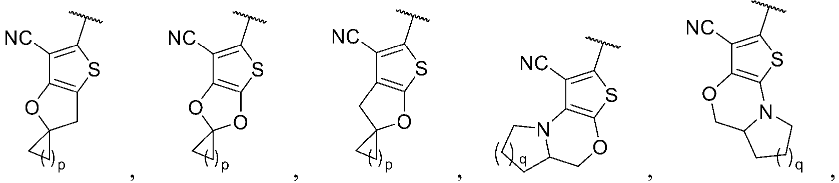 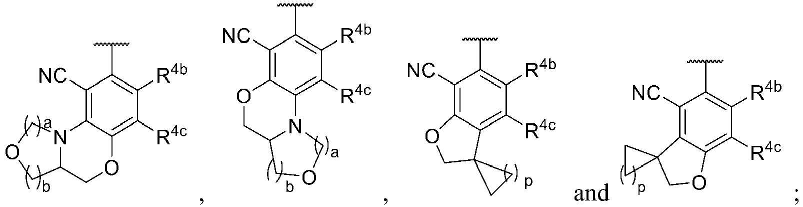 in some embodiments, R² is selected from 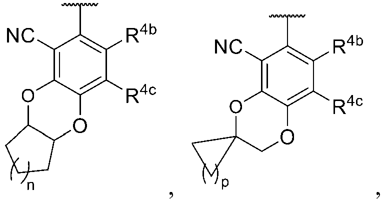 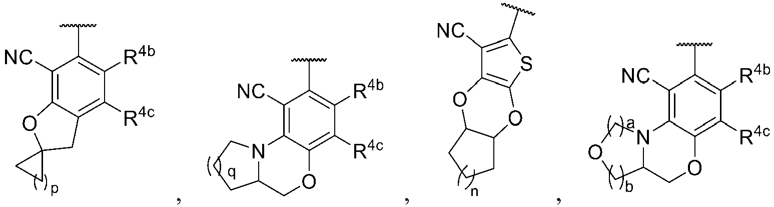 and 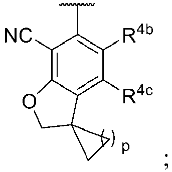 in some embodiments, R² is selected from 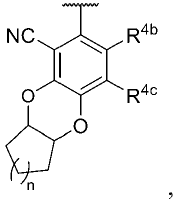 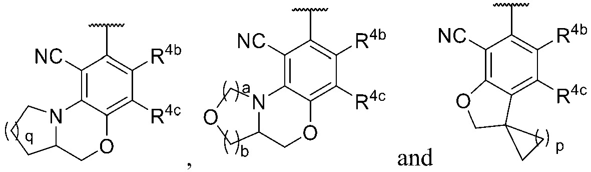 wherein:
n is 0, 1 or 2 (in some embodiments, 0 or 1); p is 1, 2 or 3 (in some embodiments, 1); q is 1 or 2 (in some embodiments, 1); a is 1 or 2 (in some embodiments, 2); b is 1 or 2 (in some embodiments, 1); R^{4b} and R^{4c} are the same or different, and are each independently selected from a hydrogen atom, a deuterium atom, halogen, hydroxyl, cyano, C₁₋₆ alkyl, C₁₋₆ haloalkyl, C₁₋₆ deuterioalkyl, C₁₋₆ hydroxyalkyl, C₁₋₆ alkoxy C₁₋₆ alkyl, C₁₋₆ alkoxy, C₁₋₆ haloalkoxy, C₁₋₆ deuterioalkoxy, 3- to 8-membered cycloalkyl, 3- to 8-membered cycloalkyloxy, 3-to 8-membered heterocyclyl and 3- to 8-membered heterocyclyloxy.

In some embodiments of the present disclosure, provided are the compounds represented by general formula (I), general formula (II) or general formula (IV) or the pharmaceutically acceptable salts thereof, wherein R² is selected from 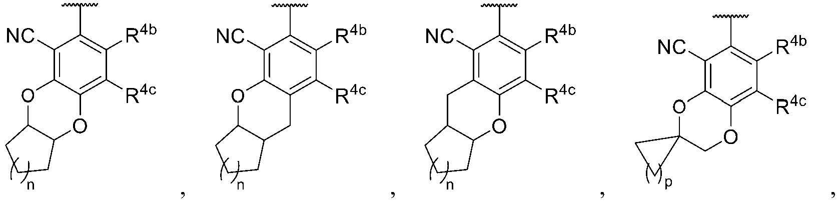 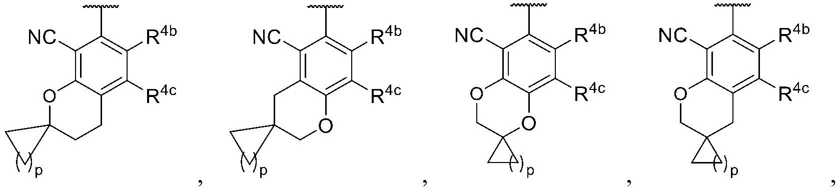 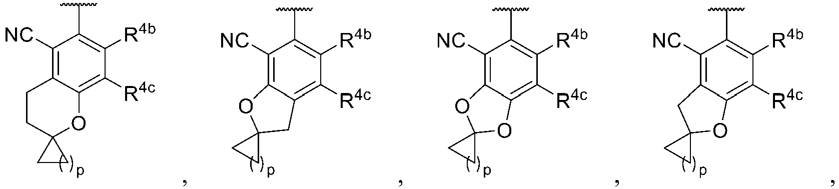 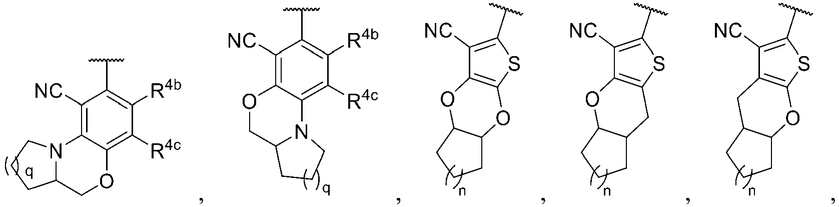 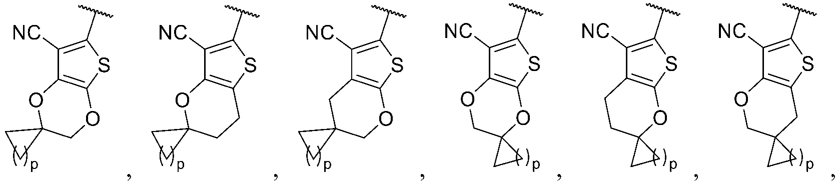 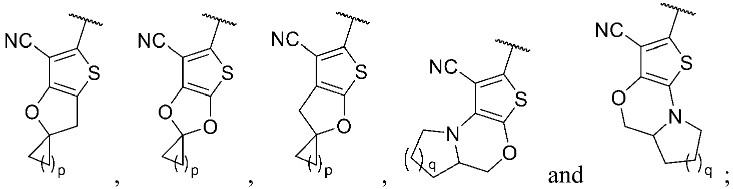 in some embodiments, R² is selected from 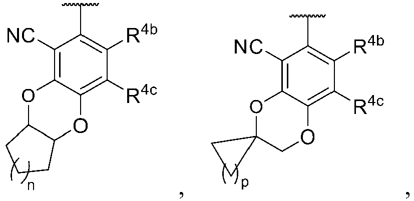 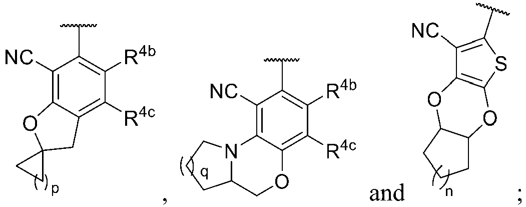 in some embodiments, R² is 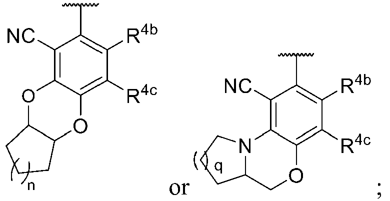 in some embodiments, R² is 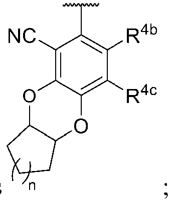 wherein: n is 0, 1 or 2 (in some embodiments, 0 or 1); p is 1, 2 or 3 (in some embodiments, 1); q is 1 or 2 (in some embodiments, 1); R^{4b} and R^{4c} are the same or different, and are each independently selected from a hydrogen atom, a deuterium atom, halogen, hydroxyl, cyano, C₁₋₆ alkyl, C₁₋₆ haloalkyl, C₁₋₆ deuterioalkyl, C₁₋₆ hydroxyalkyl, C₁₋₆ alkoxy C₁₋₆ alkyl, C₁₋₆ alkoxy, C₁₋₆ haloalkoxy, C₁₋₆ deuterioalkoxy, 3- to 8-membered cycloalkyl, 3- to 8-membered cycloalkyloxy, 3-to 8-membered heterocyclyl and 3- to 8-membered heterocyclyloxy.

In some embodiments of the present disclosure, provided are the compounds represented by general formula (I), general formula (II), general formula (III), general formula (III-1), general formula (III-2) or general formula (IV) or the pharmaceutically acceptable salts thereof, wherein R^{4b} is selected from a hydrogen atom, halogen, C₁₋₆ alkyl and C₁₋₆ haloalkyl; in some embodiments, R^{4b} is selected from halogen, C₁₋₆ alkyl and C₁₋₆ haloalkyl; in some embodiments, R^{4b} is halogen; in some embodiments, R^{4b} is F or Cl; in some embodiments, R^{4b} is F.

In some embodiments of the present disclosure, provided are the compounds represented by general formula (I), general formula (II), general formula (III), general formula (III-1), general formula (III-2) or general formula (IV) or the pharmaceutically acceptable salts thereof, wherein R^{4c} is a hydrogen atom or halogen; in some embodiments, R^{4c} is a hydrogen atom; in some embodiments, R^{4c} is halogen; in some embodiments, R^{4c} is a hydrogen atom or Cl.

In some embodiments of the present disclosure, provided are the compounds represented by general formula (I), general formula (II) or general formula (IV) or the pharmaceutically acceptable salts thereof, wherein R^{4d} and R^{4e} are the same or different, and are each independently selected from a hydrogen atom, halogen, C₁₋₆ alkyl and C₁₋₆ haloalkyl; in some embodiments, R^{4d} and R^{4e} are hydrogen atoms.

In some embodiments of the present disclosure, provided are the compounds represented by general formula (I), general formula (II) or general formula (IV) or the pharmaceutically acceptable salts thereof, wherein R^{4f} is a hydrogen atom or C₁₋₆ alkyl; in some embodiments, R^{4f} is a hydrogen atom.

In some embodiments of the present disclosure, provided are the compounds represented by general formula (I), general formula (II), general formula (III), general formula (III-1), general formula (III-2) or general formula (IV) or the pharmaceutically acceptable salts thereof, wherein p is 1, 2 or 3; in some embodiments, p is 1 or 2; in some embodiments, p is 1.

In some embodiments of the present disclosure, provided are the compounds represented by general formula (I), general formula (II), general formula (III), general formula (III-1), general formula (III-2) or general formula (IV) or the pharmaceutically acceptable salts thereof, wherein q is 1 or 2; in some embodiments, q is 1.

In some embodiments of the present disclosure, provided are the compounds represented by general formula (I), general formula (II), general formula (III), general formula (III-1), general formula (III-2) or general formula (IV) or the pharmaceutically acceptable salts thereof, wherein n is 0, 1 or 2; in some embodiments, n is 0 or 1; in some embodiments, n is 0; in some embodiments, n is 1.

In some embodiments of the present disclosure, provided are the compounds represented by general formula (I), general formula (II) or general formula (IV) or the pharmaceutically acceptable salts thereof, wherein R² is selected from 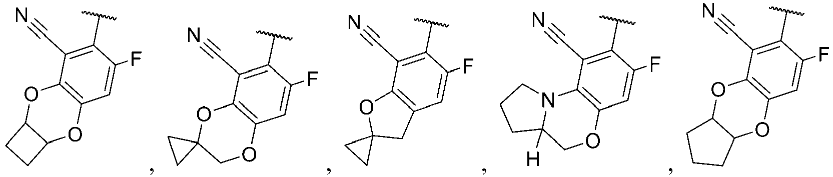 and 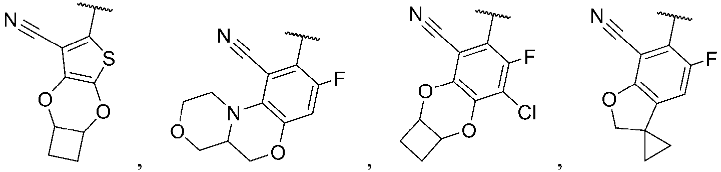 and 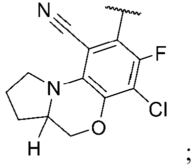 in some embodiments, R² is selected from 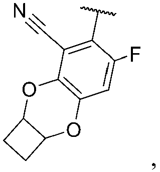 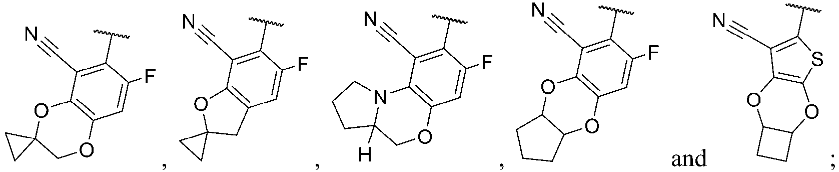 in some embodiments, R² is selected from 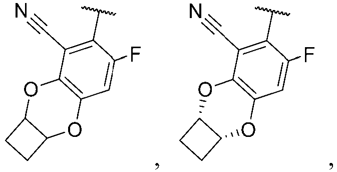 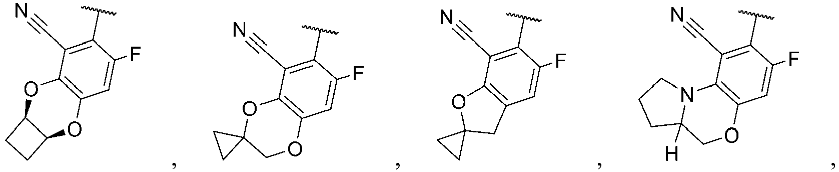 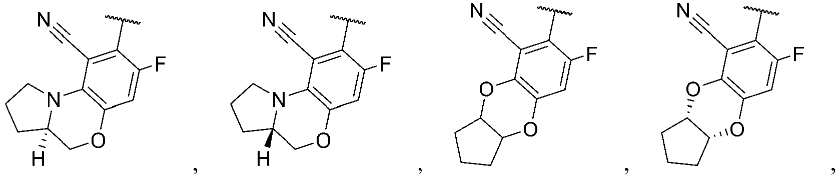 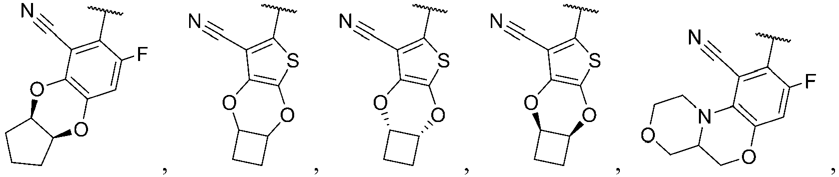 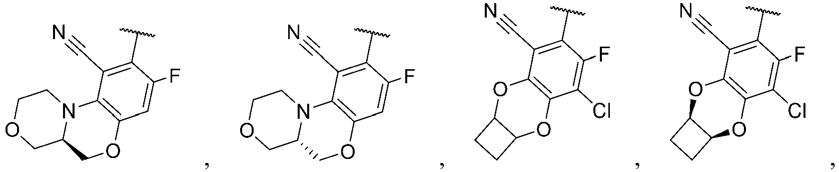 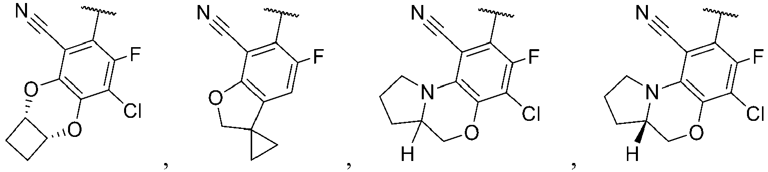 and 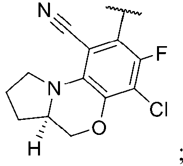 ; in some embodiments, R² is selected from 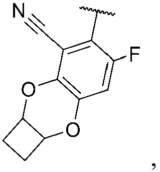 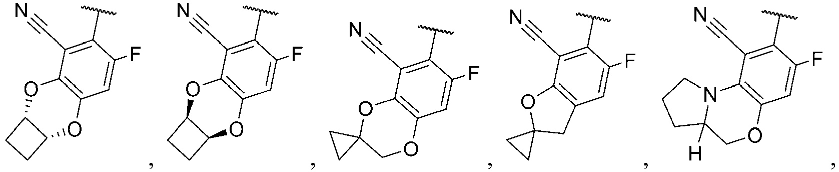 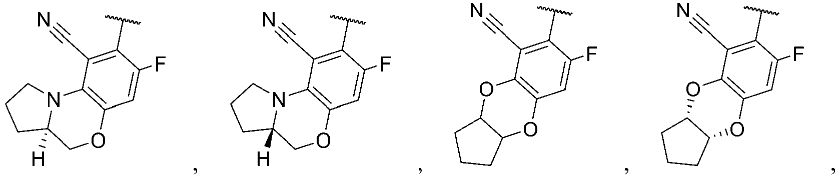 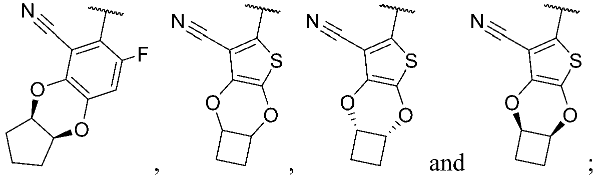 in some embodiments, R² is selected from 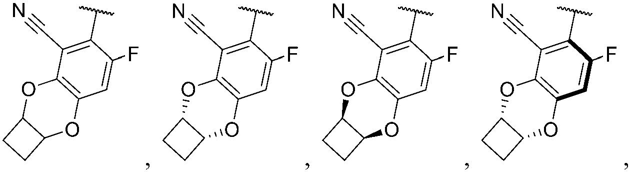 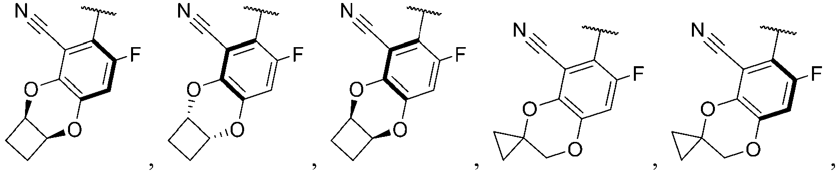 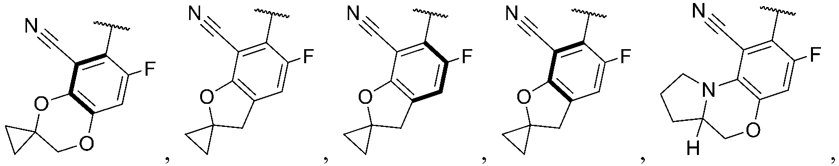 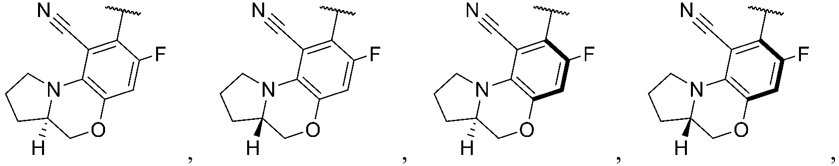 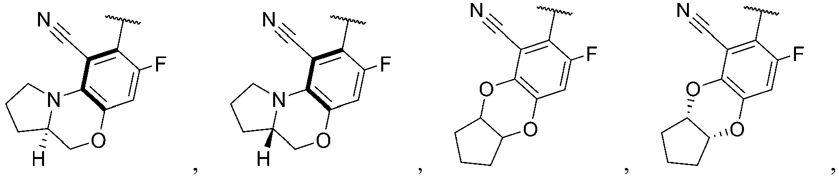 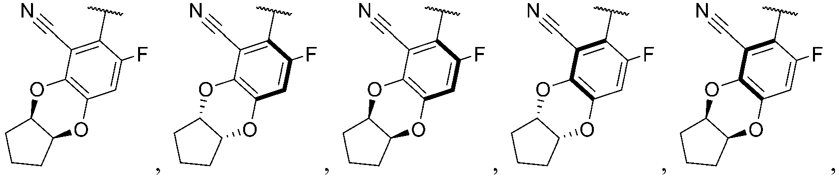 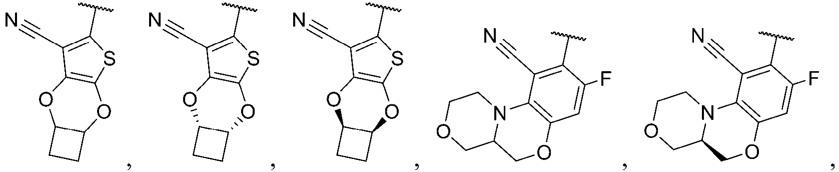 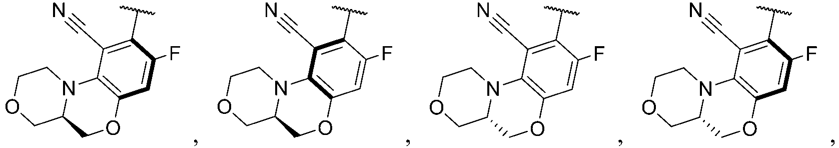 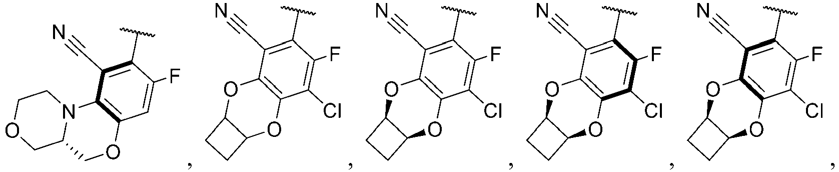 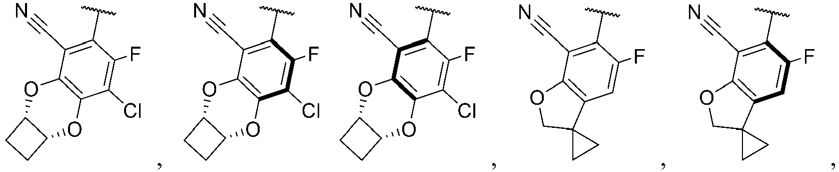 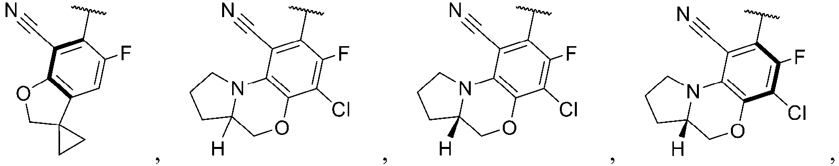 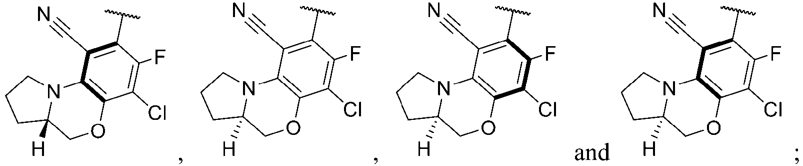 in some embodiments, R² is selected from 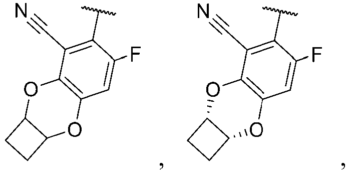 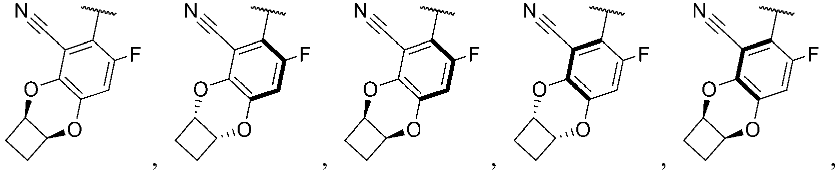 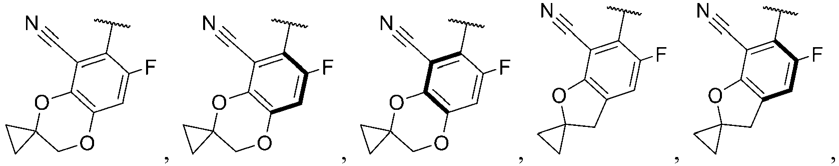 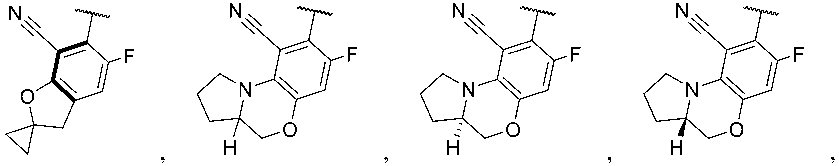 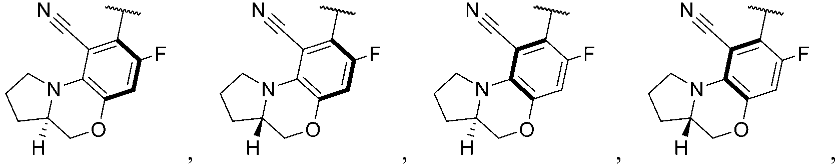 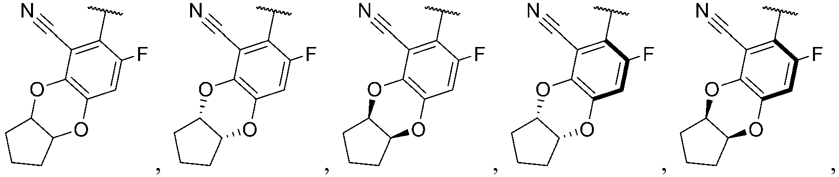 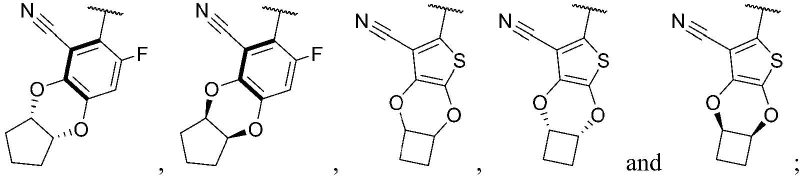 in some embodiments, R² is selected from 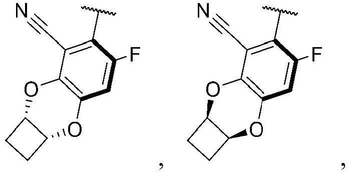 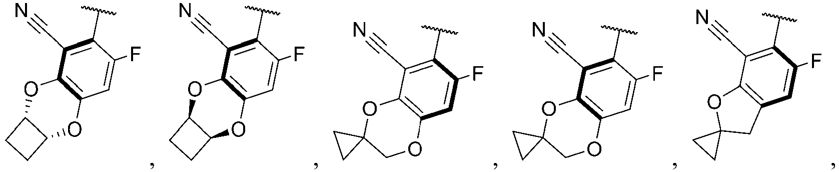 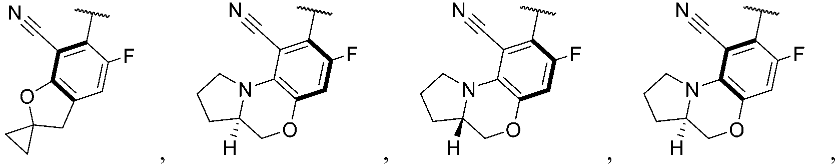 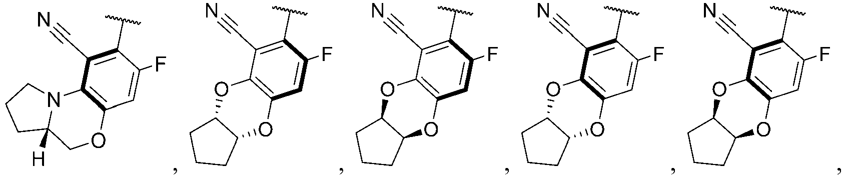 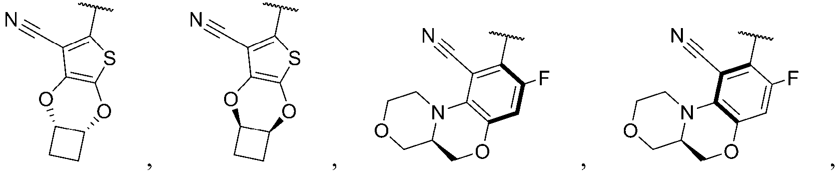 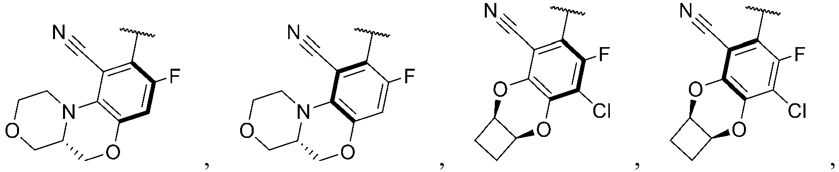 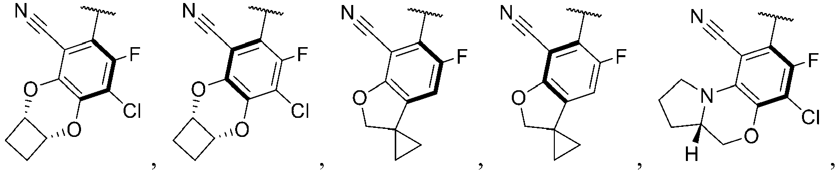 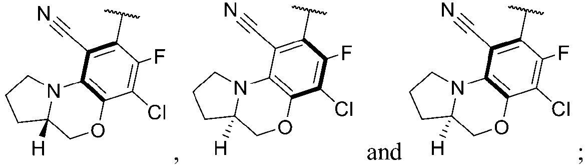 in some embodiments, R² is selected from 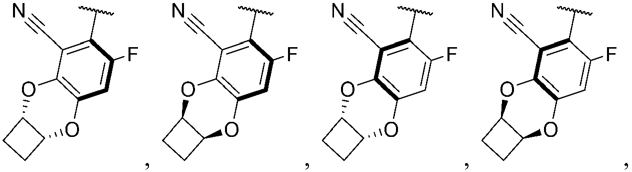 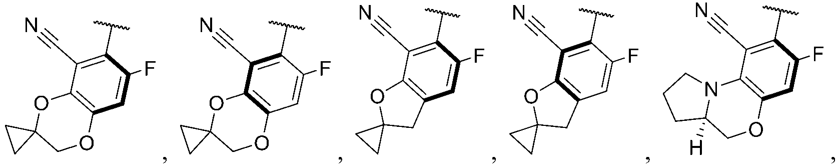 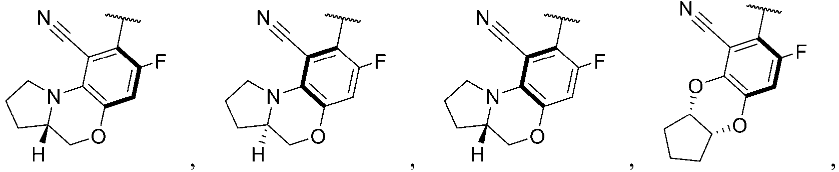 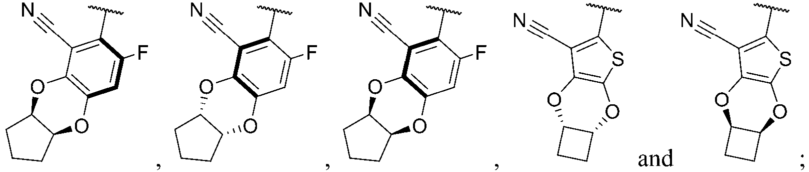 in some embodiments, R² is selected from 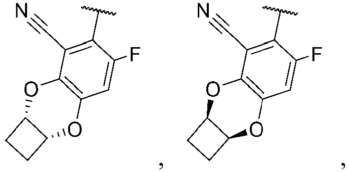 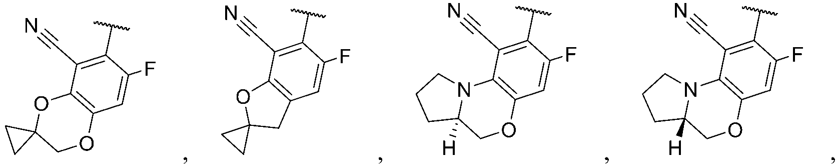 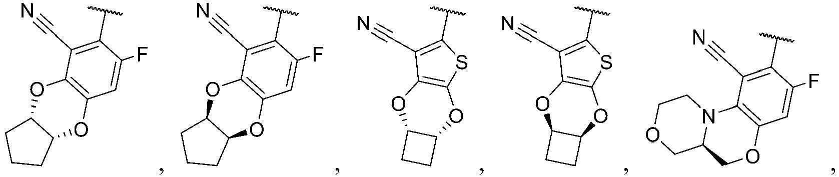 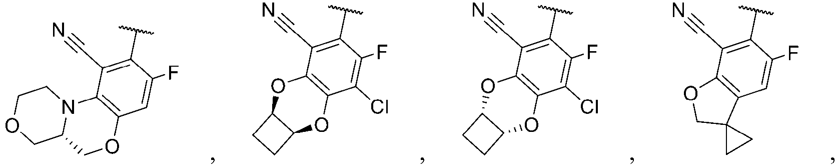 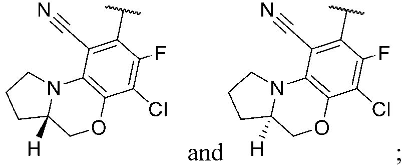 in some embodiments, R² is selected from 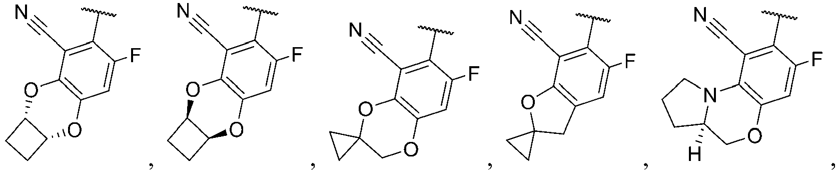 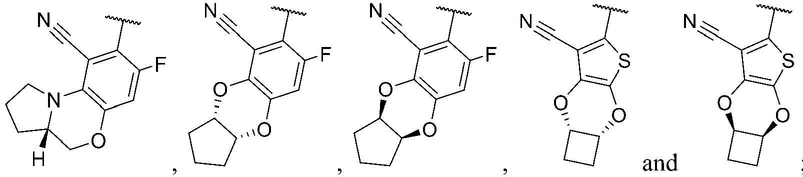 ; in some embodiments, R² is selected from 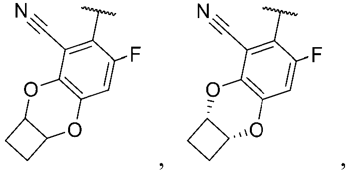 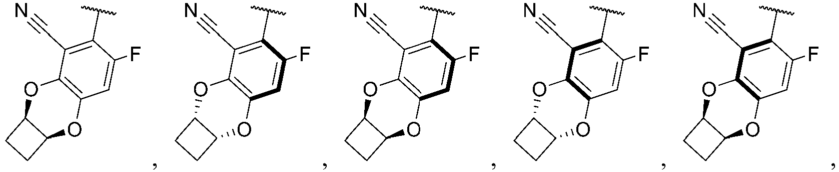 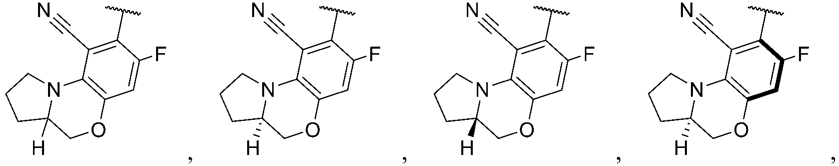 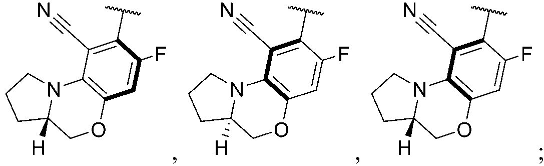 in some embodiments, R² is selected from 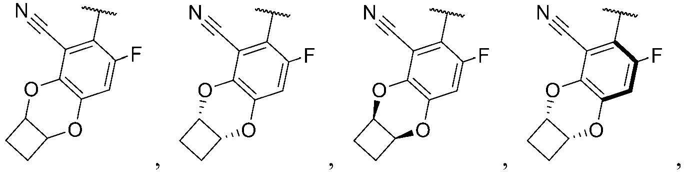 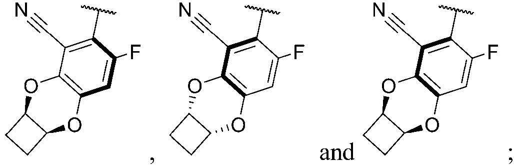 in some embodiments, R² is selected from 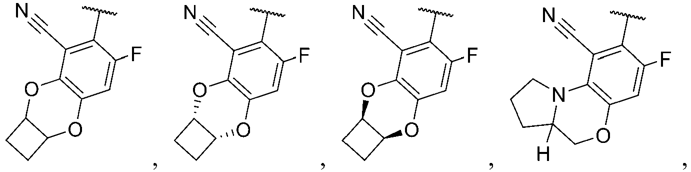 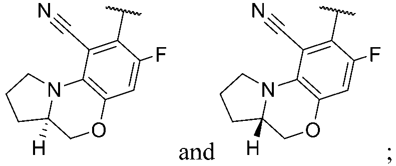 in some embodiments, R² is selected from 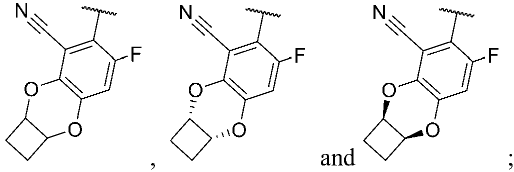 in some embodiments, R² is 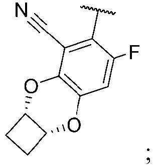 in some embodiments, R² is 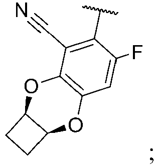 in some embodiments, R² is 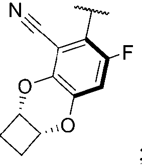 ; in some embodiments, R² is

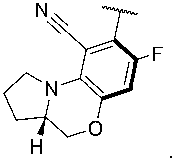

In some embodiments of the present disclosure, provided are the compounds represented by general formula (I), general formula (II), general formula (III), general formula (III-1), general formula (III-2) or general formula (IV) or the pharmaceutically acceptable salts thereof, wherein each R^{3a} is the same or different, and is independently selected from a hydrogen atom, C₁₋₆ alkyl, C₁₋₆ haloalkyl, C₁₋₆ deuterioalkyl and 3- to 8-membered cycloalkyl; in some embodiments, R^{3a} is selected from C₁₋₆ alkyl, C₁₋₆ haloalkyl and C₁₋₆ deuterioalkyl; in some embodiments, R^{3a} is C₁₋₆ alkyl or C₁₋₆ deuterioalkyl; in some embodiments, R^{3a} is C₁₋₆ deuterioalkyl; in some embodiments, R^{3a} is methyl or trideuteriomethyl; in some embodiments, R^{3a} is methyl; in some embodiments, R^{3a} is trideuteriomethyl.

In some embodiments of the present disclosure, provided are the compounds represented by general formula (I), general formula (II), general formula (III), general formula (III-1), general formula (III-2) or general formula (IV) or the pharmaceutically acceptable salts thereof, wherein R⁵ is selected from a hydrogen atom, a deuterium atom, halogen, cyano, C₁₋₆ alkyl, C₁₋₆ haloalkyl, C₁₋₆ deuterioalkyl, C₁₋₆ alkoxy, C₁₋₆ haloalkoxy, C₁₋₆ deuterioalkoxy, 3- to 6-membered cycloalkyl and 3- to 6-membered heterocyclyl; in some embodiments, R⁵ is selected from a hydrogen atom, halogen, cyano, C₁₋₆ alkyl, 3- to 6-membered cycloalkyl and 3- to 6-membered heterocyclyl; in some embodiments, R⁵ is selected from a hydrogen atom, halogen and cyano; in some embodiments, R⁵ is cyano; in some embodiments, R⁵ is a hydrogen atom.

In some embodiments of the present disclosure, provided are the compounds represented by general formula (I), general formula (II), general formula (III), general formula (III-1), general formula (III-2) or general formula (IV) or the pharmaceutically acceptable salts thereof, wherein R¹⁴, R¹⁵ and R¹⁶ at each occurrence are the same or different, and are each independently selected from a hydrogen atom, C₁₋₆ alkyl and 3- to 6-membered cycloalkyl; in some embodiments, R¹⁴, R¹⁵ and R¹⁶ at each occurrence are the same or different, and are each independently a hydrogen atom or C₁₋₆ alkyl; in some embodiments, R¹⁴ and R¹⁵ are hydrogen atoms or C₁₋₆ alkyl; in some embodiments, R¹⁴ and R¹⁵ are hydrogen atoms; in some embodiments, R¹⁶ is C₁₋₆ alkyl.

In some embodiments of the present disclosure, provided are the compounds represented by general formula (I), general formula (II), general formula (III), general formula (III-1), general formula (III-2) or general formula (IV) or the pharmaceutically acceptable salts thereof, wherein R¹⁷ is a hydrogen atom or C₁₋₆ alkyl; in some embodiments, R¹⁷ is a hydrogen atom.

In some embodiments of the present disclosure, provided are the compounds represented by general formula (I), general formula (II), general formula (III), general formula (III-1), general formula (III-2) or general formula (IV) or the pharmaceutically acceptable salts thereof, wherein each R at each occurrence is the same or different, and is independently selected from oxo, a deuterium atom, halogen, cyano, amino, -NH alkyl, -N(alkyl)₂, C₁₋₆ alkyl, C₁₋₆ haloalkyl, C₁₋₆ hydroxyalkyl, C₁₋₆ alkoxy, C₁₋₆ haloalkoxy, 3- to 6-membered cycloalkyl and 3- to 6-membered heterocyclyl; in some embodiments, each R at each occurrence is the same or different, and is independently selected from halogen, C₁₋₆ alkyl, C₁₋₆ haloalkyl, C₁₋₆ alkoxy and C₁₋₆ haloalkoxy; in some embodiments, each R at each occurrence is the same or different, and is independently selected from halogen, C₁₋₆ alkyl and C₁₋₆ haloalkyl.

In some embodiments of the present disclosure, provided is the compound represented by general formula (II) or the pharmaceutically acceptable salt thereof, wherein R^{1a} and R^{1b} are hydrogen atoms; R^{3a} is C₁₋₆ alkyl or C₁₋₆ deuterioalkyl; X¹ is CH;A is CH; R⁵ is a hydrogen atom; R² is selected from 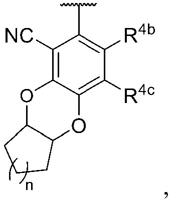 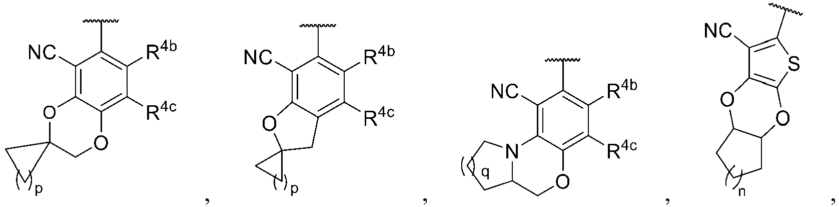 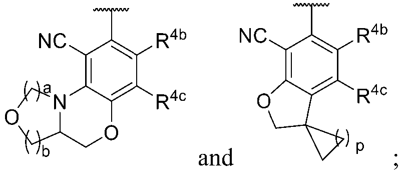 n is 0 or 1;p is 1;q is 1;a is 2;b is 1; R^{4b} is halogen; R^{4c} is a hydrogen atom or halogen.

In some embodiments of the present disclosure, provided is the compound represented by general formula (II) or the pharmaceutically acceptable salt thereof, wherein R^{1a} and R^{1b} are hydrogen atoms; R^{3a} is C₁₋₆ deuterioalkyl; X¹ is CR^{5a}; R^{5a} is selected from a hydrogen atom, halogen and C₁₋₆ alkyl; A is CR^{3b}; R^{3b} is selected from a hydrogen atom, halogen and C₁₋₆ alkyl; R⁵ is selected from a hydrogen atom, halogen and cyano; R² is selected from 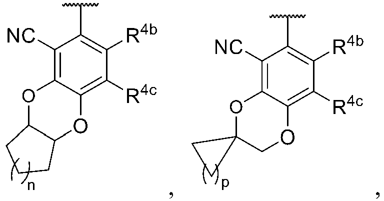 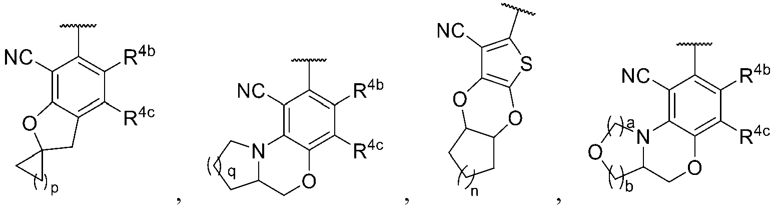 and 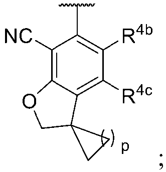 n is 0, 1 or 2; p is 1, 2 or 3; q is 1 or 2;a is 1 or 2;b is 1 or 2; R^{4b} is halogen; R^{4c} is a hydrogen atom or halogen.

In some embodiments of the present disclosure, provided is the compound represented by general formula (II) or the pharmaceutically acceptable salt thereof, wherein R^{1a} and R^{1b} are the same or different, and are each independently a hydrogen atom or a deuterium atom; R^{3a} is C₁₋₆ alkyl or C₁₋₆ deuterioalkyl; X¹ is CR^{5a}; R^{5a} is selected from a hydrogen atom, halogen and C₁₋₆ alkyl; A is CR^{3b}; R^{3b} is selected from a hydrogen atom, halogen and C₁₋₆ alkyl; R⁵ is selected from a hydrogen atom, halogen and cyano; R² is selected from 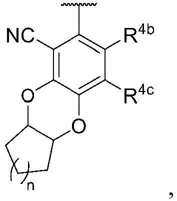 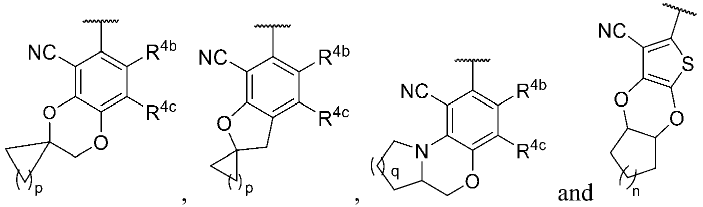 ; n is 0, 1 or 2; p is 1, 2 or 3; q is 1 or 2; R^{4b} is halogen; R^{4c} is a hydrogen atom.

In some embodiments of the present disclosure, provided is the compound represented by general formula (II) or the pharmaceutically acceptable salt thereof, wherein R^{1a} and R^{1b} are the same or different, and are each independently a hydrogen atom or a deuterium atom; R^{3a} is C₁₋₆ deuterioalkyl; X¹ is CR^{5a}; R^{5a} is selected from a hydrogen atom, halogen and C₁₋₆ alkyl; A is CR^{3b}; R^{3b} is selected from a hydrogen atom, halogen and C₁₋₆ alkyl; R⁵ is a hydrogen atom; R² is selected from 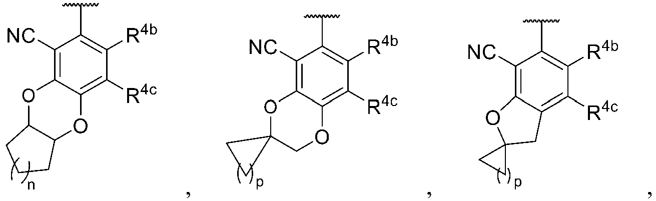 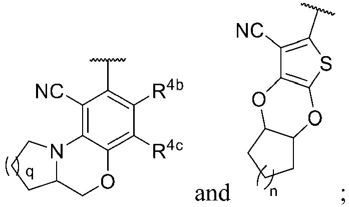 n is 0, 1 or 2; p is 1, 2 or 3; q is 1 or 2; R^{4b} is halogen; R^{4c} is a hydrogen atom.

In some embodiments of the present disclosure, provided are the compounds represented by general formula (III), general formula (III-1) or general formula (III-2) or the pharmaceutically acceptable salts thereof, wherein R^{1a} and R^{1b} are hydrogen atoms; R^{3a} is C₁₋₆ alkyl or C₁₋₆ deuterioalkyl; R⁵ is a hydrogen atom; X¹ is CH; A is CH; R^{4b} is halogen; R^{4c} is a hydrogen atom or halogen; ring D is selected from 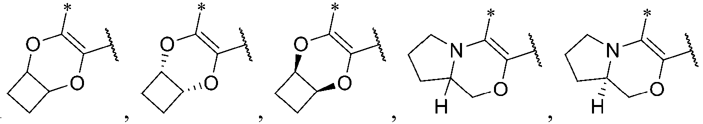 and 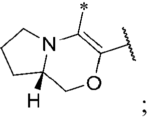 j is 0; the * end is attached to the side proximal to the cyano.

In some embodiments of the present disclosure, provided are the compounds represented by general formula (III), general formula (III-1) or general formula (III-2) or the pharmaceutically acceptable salts thereof, wherein R^{1a} and R^{1b} are hydrogen atoms; R^{3a} is C₁₋₆ deuterioalkyl; R⁵ is selected from a hydrogen atom, halogen and cyano; X¹ is CR^{5a}; R^{5a} is selected from a hydrogen atom, halogen and C₁₋₆ alkyl; A is CR^{3b}; R^{3b} is selected from a hydrogen atom, halogen and C₁₋₆ alkyl; R^{4b} is halogen; R^{4c} is a hydrogen atom or halogen; ring D is selected from 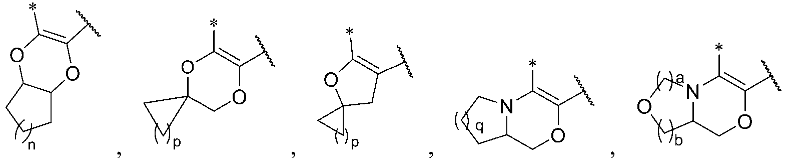 and 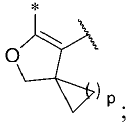 the * end is attached to the side proximal to the cyano; n is 0, 1 or 2; p is 1, 2 or 3; q is 1 or 2; a is 1 or 2; b is 1 or 2; j is 0.

In some embodiments of the present disclosure, provided are the compounds represented by general formula (III), general formula (III-1) or general formula (III-2) or the pharmaceutically acceptable salts thereof, wherein R^{1a} and R^{1b} are the same or different, and are each independently a hydrogen atom or a deuterium atom; R^{3a} is C₁₋₆ alkyl or C₁₋₆ deuterioalkyl; R⁵ is selected from a hydrogen atom, halogen and cyano; X¹ is CR^{5a}; R^{5a} is selected from a hydrogen atom, halogen and C₁₋₆ alkyl; A is CR^{3b}; R^{3b} is selected from a hydrogen atom, halogen and C₁₋₆ alkyl; R^{4b} is halogen; R^{4c} is a hydrogen atom; ring D is selected from 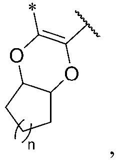 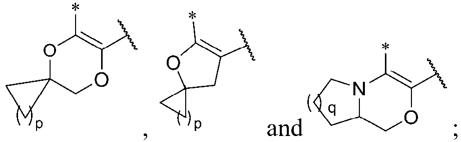 n is 0, 1 or 2; p is 1,2 or 3; q is 1 or 2; R⁴ is selected from halogen, C₁₋₆ alkyl and C₁₋₆ haloalkyl; j is 0 or 1; the * end is attached to the side proximal to the cyano, and ring D can be substituted with R⁴ at any substitutable position.

In some embodiments of the present disclosure, provided are the compounds represented by general formula (III), general formula (III-1) or general formula (III-2) or the pharmaceutically acceptable salts thereof, wherein R^{1a} and R^{1b} are hydrogen atoms; R^{3a} is C₁₋₆ deuterioalkyl; R⁵ is a hydrogen atom; X¹ is CH; A is CH; R^{4b} is halogen; R^{4c} is a hydrogen atom; ring D is selected from 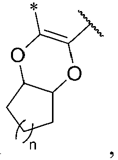 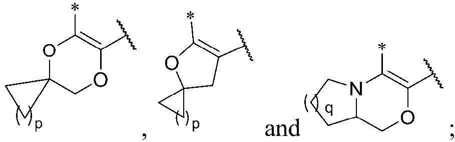 n is 0 or 1; p is 1; q is 1 or 2; j is 0; the * end is attached to the side proximal to the cyano.

In some embodiments of the present disclosure, provided is the compound represented by general formula (IV) or the pharmaceutically acceptable salt thereof, wherein R^{3a} is C₁₋₆ alkyl or C₁₋₆ deuterioalkyl; R⁵ is a hydrogen atom; R² is selected from 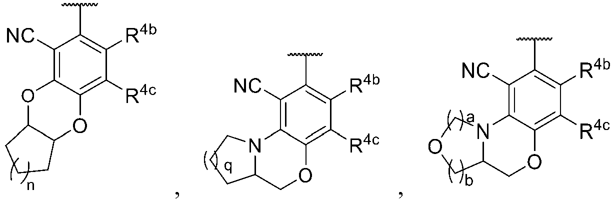 and 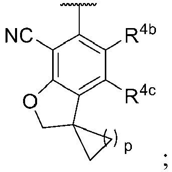 n is 0; p is 1; q is 1; a is 2; b is 1; R^{4b} is halogen; R^{4c} is a hydrogen atom or halogen. In some embodiments of the present disclosure, provided is the compound represented by general formula (IV) or the pharmaceutically acceptable salt thereof, wherein R^{3a} is C₁₋₆ deuterioalkyl; R⁵ is a hydrogen atom; R² is selected from 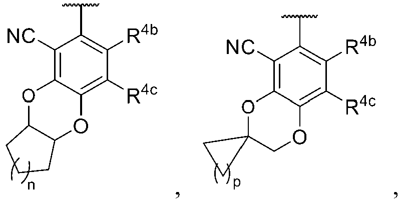 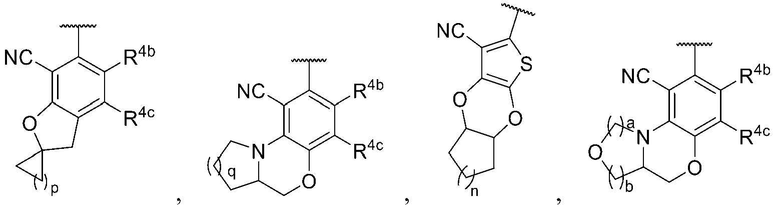 and 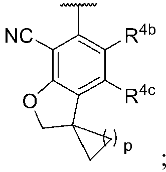 n is 0, 1 or 2; p is 1, 2 or 3; q is 1 or 2; a is 1 or 2; b is 1 or 2; R^{4b} is halogen; R^{4c} is a hydrogen atom or halogen.

In some embodiments of the present disclosure, provided is the compound represented by general formula (IV) or the pharmaceutically acceptable salt thereof, wherein R^{3a} is C₁₋₆ alkyl or C₁₋₆ deuterioalkyl; R⁵ is selected from a hydrogen atom, halogen and cyano; R² is selected from 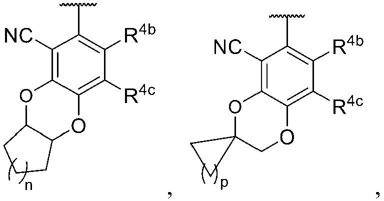 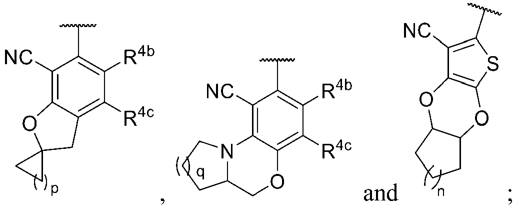 n is 0, 1 or 2; p is 1, 2 or 3; q is 1 or 2; R^{4b} is halogen; R^{4c} is a hydrogen atom.

In some embodiments of the present disclosure, provided is the compound represented by general formula (IV) or the pharmaceutically acceptable salt thereof, wherein R^{3a} is C₁₋₆ deuterioalkyl; R⁵ is a hydrogen atom; R² is selected from 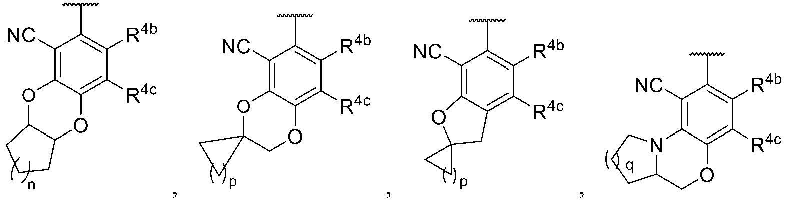 and 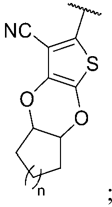 n is 0, 1 or 2; p is 1, 2 or 3; q is 1 or 2; R^{4b} is halogen; R^{4c} is a hydrogen atom.

In some embodiments of the present disclosure, provided is the compound represented by general formula (IV) or the pharmaceutically acceptable salt thereof, wherein R^{3a} is C₁₋₆ deuterioalkyl; R⁵ is a hydrogen atom; R² is 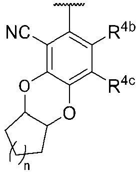 or 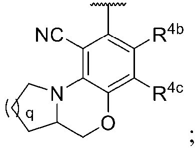 n is 0, 1 or 2; q is 1 or 2; R^{4b} is halogen; R^{4c} is a hydrogen atom or halogen.

**Table A. Typical compounds of the present disclosure, including but not limited to:**

| Compound No. | Compound structure | Name |
|---|---|---|
| 1 | 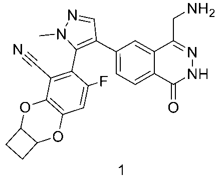 | 5-(4-(4-(aminomethyl)-1-oxo-1,2-dihydrophthalazin-6-yl)-1-methyl-1*H-*pyrazol-5-yl)-6-fluoro-1,2,2*a*,8*a-*tetrahydrobenzo[*b*]cyclobuta[*e*][1,4]dioxi ne-4-carbonitrile **1** |
| 1-p1 | 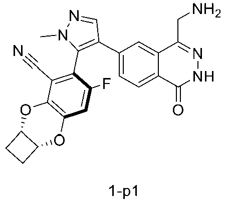 | (*M*)-(2*aS*,8*aR*)-5-(4-(4-(aminomethyl)-1-oxo-1,2-dihydrophthalazin-6-yl)-1-methyl-1*H*-pyrazol-5-yl)-6-fluoro-1,2,2*a*,8*a-*tetrahydrobenzo[*b*]cyclobuta[*e*][1,4]dioxi ne-4-carbonitrile **1-p1** |
| 1-p2 | 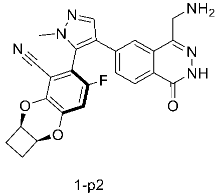 | (*M*)-(2*aR*,8*aS*)-5-(4-(4-(aminomethyl)-1-oxo-1,2-dihydrophthalazin-6-yl)-1-methyl-1*H*-pyrazol-5-yl)-6-fluoro-1,2,2*a,*8*a-*tetrahydrobenzo[*b*]cyclobuta[*e*][1,4]dioxi ne-4-carbonitrile **1-p2** |
| 1-p3 | 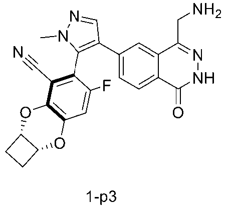 | (*P*)-(2*aS*,8*aR*)-5-(4-(4-(aminomethyl)-1-oxo-1,2-dihydrophthalazin-6-yl)-1-methyl-1*H*-pyrazol-5-yl)-6-fluoro-*1*,2,2*a*,8*a-*tetrahydrobenzo[*b*]cyclobuta[*e*][1,4]dioxi ne-4-carbonitrile **1-p3** |
| 1-p4 | 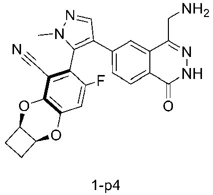 | (*P*)-(2*aR*,8*aS*)-5-(4-(4-(aminomethyl)-1-oxo-1,2-dihydrophthalazin-6-yl)-1-methyl-1*H*-pyrazol-5-yl)-6-fluoro-1,2,2*a*,8*a-*tetrahydrobenzo[*b*]cyclobuta[*e*][1,4]dioxi ne-4-carbonitrile **1-p4** |
| 2 | 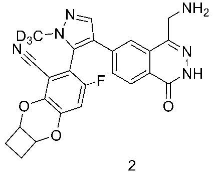 | 5-(4-(4-(aminomethyl)-1-oxo-1,2-dihydrophthalazin-6-yl)-1-(methyl-*d₃*)-1*H*-pyrazol-5-yl)-6-fluoro-1,2,2*a*,8*a-*tetrahydrobenzo[*b*]cyclobuta[*e*][1,4]dioxi ne-4-carbonitrile **2** |
| 2-p1 | 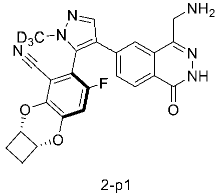 | (*M*)-(2*aS*,8*aR*)-5-(4-(4-(aminomethyl)-1-oxo-1,2-dihydrophthalazin-6-yl)-1-(methyl-*d₃*)-1*H*-pyrazol-5-yl)-6-fluoro-1,2,2*a*,8*a-*tetrahydrobenzo[*b*]cyclobuta[*e*][1,4]dioxi ne-4-carbonitrile **2-p1** |
| 2-p2 | 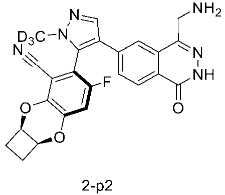 | (*M*)-(2*aR*,8*aS*)-5-(4-(4-(aminomethyl)-1-oxo-1,2-dihydrophthalazin-6-yl)-1-(methyl-*d₃*)-1*H*-pyrazol-5-yl)-6-fluoro-*1*,2,2*a*,8*a-*tetrahydrobenzo[*b*]cyclobuta[*e*][1,4]dioxi ne-4-carbonitrile **2-p2** |
| 2-p3 | 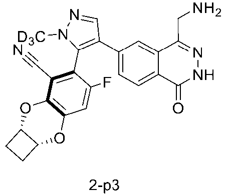 | (*P*)-(2*aS*,8*aR*)-5-(4-(4-(aminomethyl)-1-oxo-1,2-dihydrophthalazin-6-yl)-1-(methyl-*d₃*)-1*H*-pyrazol-5-yl)-6-fluoro-1,2,2*a,*8*a-*tetrahydrobenzo[*b*]cyclobuta[*e*][1,4]dioxi ne-4-carbonitrile **2-p3** |
| 2-p4 | 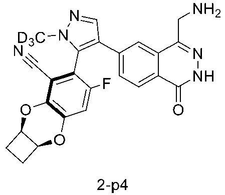 | (*P*)-(2*aR*,8*aS*)-5-(4-(4-(aminomethyl)-1-oxo-1,2-dihydrophthalazin-6-yl)-1-(methyl-*d₃*)-1*H*-pyrazol-5-yl)-6-fluoro-1,2,2*a*,8*a-*tetrahydrobenzo[*b*]cyclobuta[*e*][1,4]dioxi ne-4-carbonitrile **2-p4** |
| 3 | 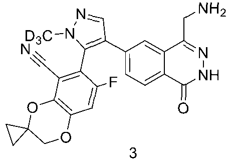 | 7-(4-(4-(aminomethyl)-1-oxo-1,2-dihydrophthalazin-6-yl)-1-(methyl-*d₃*)-1H-pyrazol-5-yl)-6-fluoro-3*H-*spiro[benzo[*b*][1,4]dioxine-2,1'-cyclopropane]-8-carbonitrile **3** |
| 3-p1 | 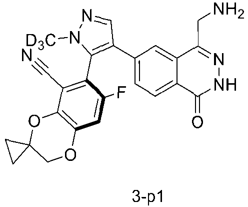 | (*M*)-7-(4-(4-(aminomethyl)-1-oxo-1,2-dihydrophthalazin-6-yl)-1-(methyl-*d₃*)-1*H*-pyrazol-5-yl)-6-fluoro-3H-spiro[benzo[*b*][1,4]dioxine-2,1'-cyclopropane]-8-carbonitrile **3-p1** |
| 3-p2 | 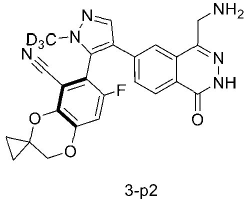 | (*P*)-7-(4-(4-(aminomethyl)-1-oxo-1,2-dihydrophthalazin-6-yl)-1-(methyl-*d₃*)-1*H*-pyrazol-5-yl)-6-fluoro-3H-spiro[benzo[*b*][1,4]dioxine-2,1'-cyclopropane]-8-carbonitrile **3-p2** |
| 4 | 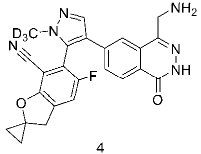 | 6-(4-(4-(aminomethyl)-1-oxo-1,2-dihydrophthalazin-6-yl)-1-(methyl-*d₃*)-1H-pyrazol-5-yl)-5-fluoro-3*H-*spiro[benzofuran-2,1'-cyclopropane]-7-carbonitrile **4** |
| 4-p1 | 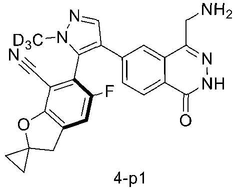 | (*M*)-6-(4-(4-(aminomethyl)-1-oxo-1,2-dihydrophthalazin-6-yl)-1-(methyl-*d₃*)-1*H*-pyrazol-5-yl)-5-fluoro-3*H-*spiro[benzofuran-2,1'-cyclopropane]-7-carbonitrile **4-p1** |
| 4-p2 | 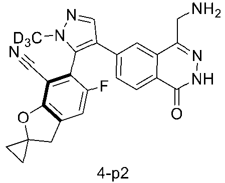 | (*P*)-6-(4-(4-(aminomethyl)-1-oxo-1,2-dihydrophthalazin-6-yl)-1-(methyl-*d₃*)-1H-pyrazol-5-yl)-5-fluoro-3*H-*spiro[benzofuran-2,1'-cyclopropane]-7-carbonitrile **4-p2** |
| | 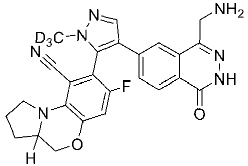 | 8-(4-(4-(aminomethyl)-1-oxo-1,2-dihydrophthalazin-6-yl)-1-(methyl-*d₃*)-1*H*-pyrazol-5-yl)-7-fluoro-2,3,3a,4-tetrahydro-1H-benzo[*b*]pyrrolo[1,2-*d*][1,4]oxazine-9-carbonitrile |
| 5 | 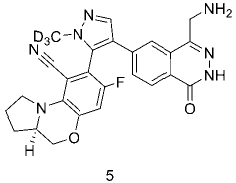 | (*R)*-8-(4-(4-(aminomethyl)-1-oxo-1,2-dihydrophthalazin-6-yl)-1-(methyl-*d₃*)-1*H*-pyrazol-5-yl)-7-fluoro-2,3,3a,4-tetrahydro-1H-benzo[*b*]pyrrolo[1,2-*d*][1,4]oxazine-9-carbonitrile **5** |
| 5-p1 | 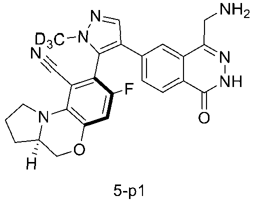 | (*M*)-(*R*)-8-(4-(4-(aminomethyl)-1-oxo-1,2-dihydrophthalazin-6-yl)-1-(methyl-*d₃*)-1*H*-pyrazol-5-yl)-7-fluoro-2,3,3a,4-tetrahydro-1H-benzo[b]pyrrolo[1,2-*d*][1,4]oxazine-9-carbonitrile **5-p1** |
| 5-p2 | 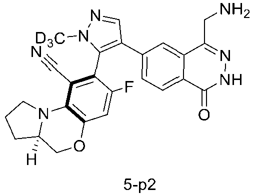 | (*P*)-(*R*)-8-(4-(4-(aminomethyl)-1-oxo-1,2-dihydrophthalazin-6-yl)-1-(methyl-*d₃*)-1*H*-pyrazol-5-yl)-7-fluoro-2,3,3a,4-tetrahydro-1H-benzo[*b*]pyrrolo[1,2-*d*][1,4]oxazine-9-carbonitrile **5-p2** |
| 6 | 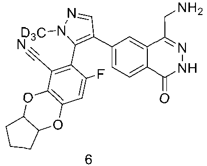 | 6-(4-(4-(aminomethyl)-1-oxo-1,2-dihydrophthalazin-6-yl)-1-(methyl-*d₃*)-1*H*-pyrazol-5-yl)-7-fluoro-2,3,3a,9a-tetrahydro-1*H-*benzo[*b*]cyclopenta[*e*][1,4]dioxine-5-carbonitrile 6 |
| 6-p1 | 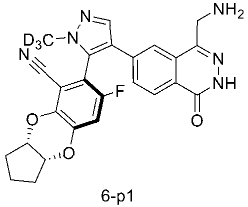 | (*M*)-(3*aS*,9*aR*)-6-(4-(4-(aminomethyl)-1-oxo-1,2-dihydrophthalazin-6-yl)-1-(methyl-*d₃*)-1*H*-pyrazol-5-yl)-7-fluoro-2,3,3*a*,9*a*-tetrahydro-1*H-*benzo[*b*]cyclopenta[*e*][1,4]dioxine-5-carbonitrile **6-p1** |
| 6-p2 | 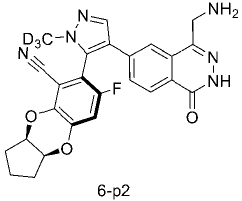 | (*M*)-(3*aR*,9*aS*)-6-(4-(4-(aminomethyl)-1-oxo-1,2-dihydrophthalazin-6-yl)-1-(methyl-*d₃*)-1*H*-pyrazol-5-yl)-7-fluoro-2,3,3*a*,9*a*-tetrahydro-1*H-*benzo[*b*]cyclopenta[*e*][1,4]dioxine-5-carbonitrile **6-p2** |
| 6-p3 | 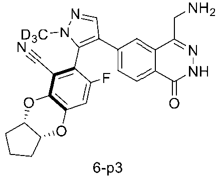 | (*P*)-(3*aS*,9*aR*)-6-(4-(4-(aminomethyl)-1-oxo-1,2-dihydrophthalazin-6-yl)-1-(methyl-*d₃*)-1*H*-pyrazol-5-yl)-7-fluoro-2,3,3*a*,9*a*-tetrahydro-1*H-*benzo[*b*]cyclopenta[*e*][1,4]dioxine-5-carbonitrile **6-p3** |
| 6-p4 | 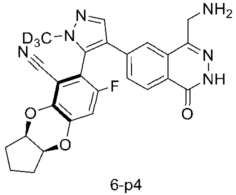 | (*P*)-(3*aR*,9*aS*)-6-(4-(4-(aminomethyl)-1-oxo-1,2-dihydrophthalazin-6-yl)-1-(methyl-*d₃*)-1*H*-pyrazol-5-yl)-7-fluoro-2,3,3*a*,9*a-*tetrahydro-1*H-*benzo[*b*]cyclopenta[*e*][1,4]dioxine-5-carbonitrile **6-p4** |
| 7 | 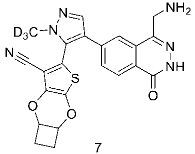 | 2-(4-(4-(aminomethyl)-1-oxo-1,2-dihydrophthalazin-6-yl)-1-(methyl-*d3*)-1*H*-pyrazol-5-yl)-4*a*,5,6,6*a-*tetrahydrocyclobuta[*b*]thieno[2,3-*e*][1,4]dioxine-3-carbonitrile 7 |
| 7-p1 | 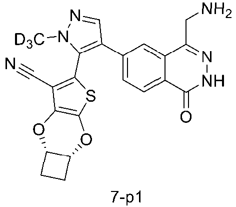 | (4*aS*,6*aR*)-2-(4-(4-(aminomethyl)-1-oxo-1,2-dihydrophthalazin-6-yl)-1-(methyl-*d3*)-1*H-*pyrazol-5-yl)-4*a*,5,6,6*a-*tetrahydrocyclobuta[*b*]thieno[2,3-*e*][1,4]dioxine-3-carbonitrile **7-pl** |
| 7-p2 | 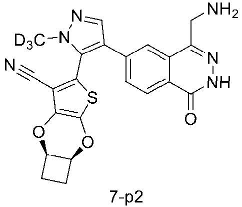 | (4*aR*,6*aS*)-2-(4-(4-(aminomethyl)-1-oxo-1,2-dihydrophthalazin-6-yl)-1-(methyl-*d3*)-1*H*-pyrazol-5-yl)-4*a*,5,6,6*a-*tetrahydrocyclobuta[*b*]thieno[2,3-*e*][1,4]dioxine-3-carbonitrile **7-p2** |
| 8 | 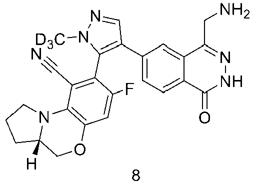 | (*S*)-8-(4-(4-(aminomethyl)-1-oxo-1,2-dihydrophthalazin-6-yl)-1-(methyl-*d₃*)-1*H*-pyrazol-5-yl)-7-fluoro-2,3,3a,4-tetrahydro-1*H*-benzo[*b*]pyrrolo[1,2-*d*][1,4]oxazine-9-carbonitrile **8** |
| 8-p1 | 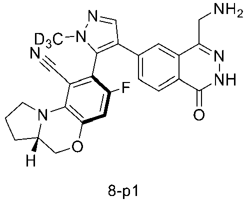 | (*M*)-(*S*)-8-(4-(4-(aminomethyl)-1-oxo-1,2-dihydrophthalazin-6-yl)-1-(methyl-*d₃*)-1*H*-pyrazol-5-yl)-7-fluoro-2,3,3a,4-tetrahydro-1*H*-benzo[*b*]pyrrolo[1,2-*d*][1,4]oxazine-9-carbonitrile **8-pl** |
| 8-p2 | 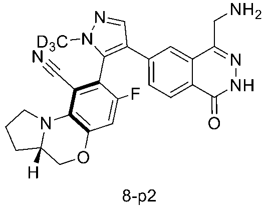 | (*P*)-(*S*)-8-(4-(4-(aminomethyl)-1-oxo-1,2-dihydrophthalazin-6-yl)-1-(methyl-*d₃*)-1*H*-pyrazol-5-yl)-7-fluoro-2,3,3*a*,4-tetrahydro-1*H*-benzo[*b*]pyrrolo[1,2-*d*][1,4]oxazine-9-carbonitrile **8-p2** |
| | 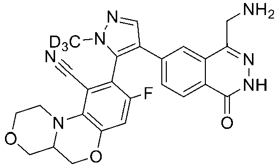 | 9-(4-(4-(aminomethyl)-1-oxo-1,2-dihydrophthalazin-6-yl)-1-(methyl-*d₃*)-1*H*-pyrazol-5-yl)-8-fluoro-1,2,4*a*,5-tetrahydro-4*H*-benzo[*b*][1,4]oxazino[4,3-*d*][1,4]oxazine-10-carbonitrile |
| **9** | 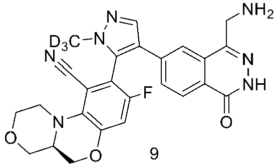 | (*R*)-9-(4-(4-(aminomethyl)-1-oxo-1,2-dihydrophthalazin-6-yl)-1-(methyl-*d₃*)-1*H*-pyrazol-5-yl)-8-fluoro-1,2,4*a*,5-tetrahydro-4*H*-benzo[*b*][1,4]oxazino[4,3-*d*][1,4]oxazine-10-carbonitrile **9** |
| **9-pl** | 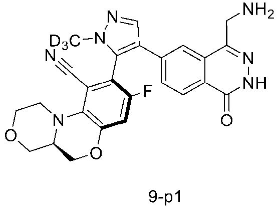 | (*M*)-(*R*)-9-(4-(4-(aminomethyl)-1-oxo-1,2-dihydrophthalazin-6-yl)-1-(methyl-*d₃*)-1*H*-pyrazol-5-yl)-8-fluoro-1,2,4*a*,5-tetrahydro-4*H*-benzo[*b*][1,4]oxazino[4,3-*d*][1,4]oxazine-10-carbonitrile **9-pl** |
| **9-p2** | 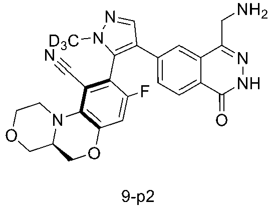 | (*P*)-(*R*)-9-(4-(4-(aminomethyl)-1-oxo-1,2-dihydrophthalazin-6-yl)-1-(methyl-*d₃*)-1*H*-pyrazol-5-yl)-8-fluoro-1,2,4*a*,5-tetrahydro-4*H*-benzo[*b*][1,4]oxazino[4,3-*d*][1,4]oxazine-10-carbonitrile **9-p2** |
| **10** | 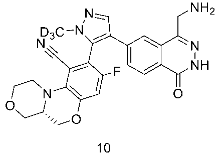 | (*S*)-9-(4-(4-(aminomethyl)-1-oxo-1,2-dihydrophthalazin-6-yl)-1-(methyl-*d₃*)-1*H*-pyrazol-5-yl)-8-fluoro-1,2,4*a*,5-tetrahydro-4*H*-benzo[*b*][1,4]oxazino[4,3-*d*][1,4]oxazine-10-carbonitrile **10** |
| | 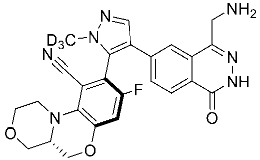 | (*M*)-(*S*)-9-(4-(4-(aminomethyl)-1-oxo-1,2-dihydrophthalazin-6-yl)-1-(methyl-*d₃*)-1*H*-pyrazol-5-yl)-8-fluoro-1,2,4*a*,5-tetrahydro-4*H*-benzo[*b*][1,4]oxazino[4,3-*d*][1,4]oxazine-10-carbonitrile |
| | 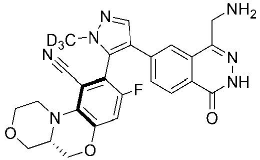 | (*P*)-(*S*)-9-(4-(4-(aminomethyl)-1-oxo-1,2-dihydrophthalazin-6-yl)-1-(methyl-*d₃*)-1*H*-pyrazol-5-yl)-8-fluoro-1,2,4*a*,5-tetrahydro-4*H*-benzo[*b*][1,4]oxazino[4,3-*d*][1,4]oxazine-10-carbonitrile |
| | 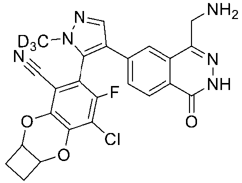 | 5-(4-(4-(aminomethyl)-1-oxo-1,2-dihydrophthalazin-6-yl)-1-(methyl-*d₃*)-1*H*-pyrazol-5-yl)-7-chloro-6-fluoro-1,2,2*a*,8*a-*tetrahydrobenzo[*b*]cyclobuta[*e*][1,4]dioxi ne-4-carbonitrile |
| | 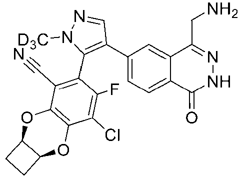 | (2*aR*,8*aS*)-5-(4-(4-(aminomethyl)-1-oxo-1,2-dihydrophthalazin-6-yl)-1-(methyl-*d₃*)-1*H*-pyrazol-5-yl)-7-chloro-6-fluoro-1,2,2*a,*8*a-*tetrahydrobenzo[*b*]cyclobuta[*e*][1,4]dioxi ne-4-carbonitrile |
| **11-p1** | 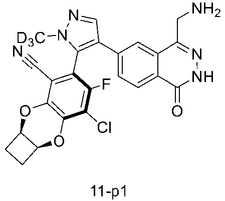 | (*M*)-(2*aR*,8*aS*)-5-(4-(4-(aminomethyl)-1-oxo-1,2-dihydrophthalazin-6-yl)-1-(methyl-*d₃*)-1*H*-pyrazol-5-yl)-7-chloro-6-fluoro-1,2,2*a*,8*a-*tetrahydrobenzo[*b*]cyclobuta[*e*][1,4]dioxi ne-4-carbonitrile **11-p1** |
| **11-p2** | 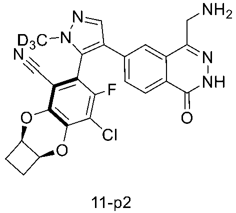 | (*P*)-(2*aR*,8*aS*)-5-(4-(4-(aminomethyl)-1-oxo-1,2-dihydrophthalazin-6-yl)-1-(methyl-*d₃*)-1*H*-pyrazol-5-yl)-7-chloro-6-fluoro-1,2,2*a*,8*a-*tetrahydrobenzo[*b*]cyclobuta[*e*][1,4]dioxi ne-4-carbonitrile **11-p2** |
| | 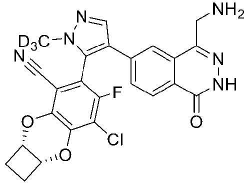 | (2*aS*,8*aR*)-5-(4-(4-(aminomethyl)-1-oxo-1,2-dihydrophthalazin-6-yl)-1-(methyl-*d₃*)-1*H*-pyrazol-5-yl)-7-chloro-6-fluoro-1,2,2*a*,8*a-*tetrahydrobenzo[*b*]cyclobuta[*e*][1,4]dioxi ne-4-carbonitrile |
| **11-p3** | 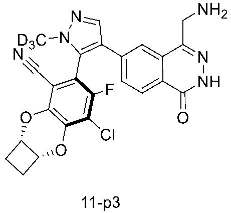 | (*M*)-(2*aS*,8*aR*)-5-(4-(4-(aminomethyl)-1-oxo-1,2-dihydrophthalazin-6-yl)-1-(methyl-*d₃*)-1*H*-pyrazol-5-yl)-7-chloro-6-fluoro-1,2,2*a*,8*a-*tetrahydrobenzo[*b*]cyclobuta[*e*][1,4]dioxi ne-4-carbonitrile **11-p3** |
| **11-p4** | 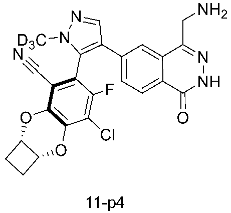 | (*P*)-(2*aS*,8*aR*)-5-(4-(4-(aminomethyl)-1-oxo-1,2-dihydrophthalazin-6-yl)-1-(methyl-*d₃*)-1*H*-pyrazol-5-yl)-7-chloro-6-fluoro-1,2,2*a*,8*a-*tetrahydrobenzo[*b*]cyclobuta[*e*][1,4]dioxi ne-4-carbonitrile **11-p4** |
| 12 | 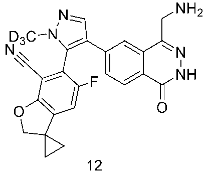 | 6-(4-(4-(aminomethyl)-1-oxo-1,2-dihydrophthalazin-6-yl)-1-(methyl-*d₃*)-1*H*-pyrazol-5-yl)-5-fluoro-2*H-*spiro[benzofuran-3,1'-cyclopropane]-7-carbonitrile **12** |
| | 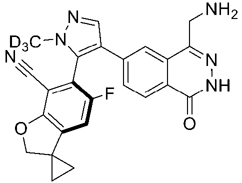 | (*M*)-6-(4-(4-(aminomethyl)-1-oxo-1,2-dihydrophthalazin-6-yl)-1-(methyl-*d₃*)-1*H*-pyrazol-5-yl)-5-fluoro-2*H-*spiro[benzofuran-3,1'-cyclopropane]-7-carbonitrile |
| | 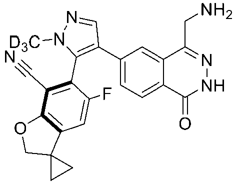 | (*P*)-6-(4-(4-(aminomethyl)-1-oxo-1,2-dihydrophthalazin-6-yl)-1-(methyl-*d₃*)-1*H*-pyrazol-5-yl)-5-fluoro-2*H-*spiro[benzofuran-3,1'-cyclopropane]-7-carbonitrile |
| | 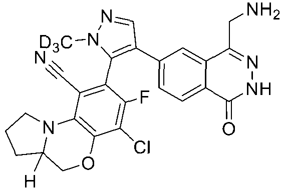 | 8-(4-(4-(aminomethyl)-1-oxo-1,2-dihydrophthalazin-6-yl)-1-(methyl-*d₃*)-1*H*-pyrazol-5-yl)-6-chloro-7-fluoro-2,3,3a,4-tetrahydro-1*H-*benzo[*b*]pyrrolo[1,2-*d*][1,4]oxazine-9-carbonitrile |
| **13** | 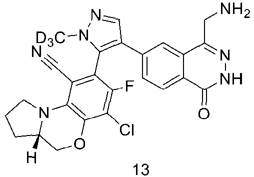 | (*S*)-8-(4-(4-(aminomethyl)-1-oxo-1,2-dihydrophthalazin-6-yl)-1-(methyl-*d₃*)-1*H*-pyrazol-5-yl)-6-chloro-7-fluoro-2,3,3a,4-tetrahydro-1*H-*benzo[*b*]pyrrolo[1,2-*d*][1,4]oxazine-9-carbonitrile **13** |
| **13-p1** | 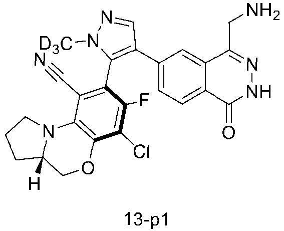 | (*M*)-(*S*)-8-(4-(4-(aminomethyl)-1-oxo-1,2-dihydrophthalazin-6-yl)-1-(methyl-*d₃*)-1*H*-pyrazol-5-yl)-6-chloro-7-fluoro-2,3,3*a*,4-tetrahydro-1*H-*benzo[*b*]pyrrolo[1,2-*d*][1,4]oxazine-9-carbonitrile **13-p1** |
| **13-p2** | 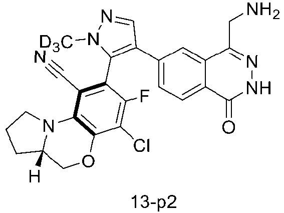 | (*P*)-(*S*)-8-(4-(4-(aminomethyl)-1-oxo-1,2-dihydrophthalazin-6-yl)-1-(methyl-*d₃*)-1*H*-pyrazol-5-yl)-6-chloro-7-fluoro-2,3,3*a*,4-tetrahydro-1*H-*benzo[*b*]pyrrolo[1,2-*d*][1,4]oxazine-9-carbonitrile **13-p2** |
| | 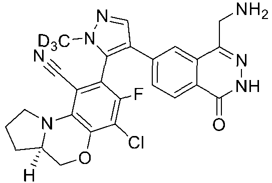 | (*R*)-8-(4-(4-(aminomethyl)-1-oxo-1,2-dihydrophthalazin-6-yl)-1-(methyl-*d₃*)-1*H*-pyrazol-5-yl)-6-chloro-7-fluoro-2,3,3a,4-tetrahydro-1*H-*benzo[*b*]pyrrolo[1,2-*d*][1,4]oxazine-9-carbonitrile |
| | 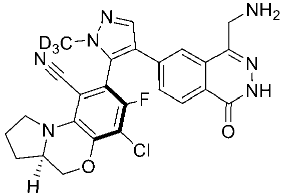 | (*M*)-(*R*)-8-(4-(4-(aminomethyl)-1-oxo-1,2-dihydrophthalazin-6-yl)-1-(methyl-*d₃*)-1*H*-pyrazol-5-yl)-6-chloro-7-fluoro-2,3,3*a*,4-tetrahydro-1*H-*benzo[*b*]pyrrolo[1,2-*d*][1,4]oxazine-9-carbonitrile |
| | 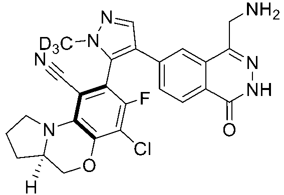 | (*P*)-(*R*)-8-(4-(4-(aminomethyl)-1-oxo-1,2-dihydrophthalazin-6-yl)-1-(methyl-*d₃*)-1*H*-pyrazol-5-yl)-6-chloro-7-fluoro-2,3,3*a*,4-tetrahydro-1*H-*benzo[*b*]pyrrolo[1,2-*d*][1,4]oxazine-9-carbonitrile |

Another aspect of the present disclosure relates to a compound represented by general formula (IA) or a salt thereof: 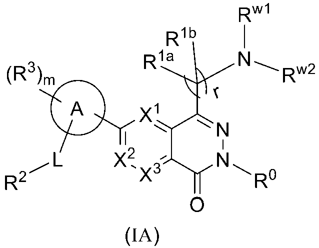 wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 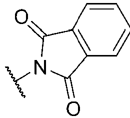 );
ring A, L, X¹, X², X³, R⁰, R^{1a}, R^{1b}, R², R³, m and r are as defined in general formula (I).

Another aspect of the present disclosure relates to a compound represented by general formula (IIA) or a salt thereof: wherein: 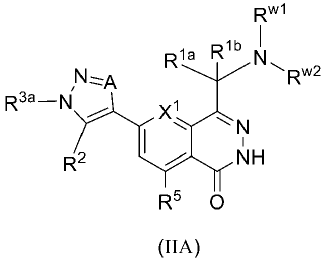
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 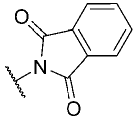 );
A, X¹, R^{1a}, R^{1b}, R², R^{3a} and R⁵ are as defined in general formula (II).

Another aspect of the present disclosure relates to a compound represented by general formula (IIIA) or a salt thereof: 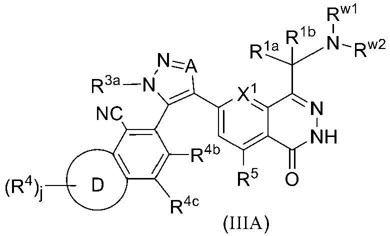 wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 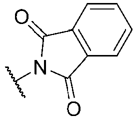 );
ring D, A, X¹, R^{1a}, R^{1b}, R^{3a}, R⁴, R^{4b}, R^{4c}, R⁵ and j are as defined in general formula (III).

Another aspect of the present disclosure relates to a compound represented by general formula (III-1A) or general formula (III-2A) or a salt thereof: 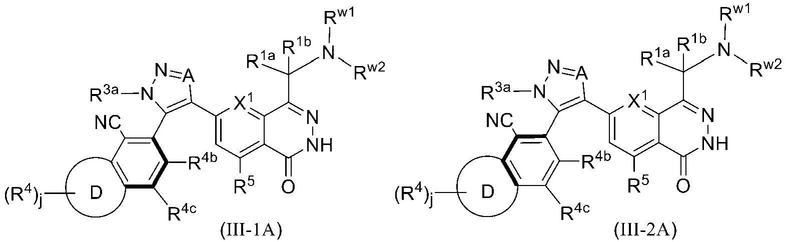 wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 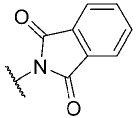 );
ring D, A, X¹, R^{1a}, R^{1b}, R^{3a}, R⁴, R^{4b}, R^{4c}, R⁵ and j are as defined in general formula (III-1) or general formula (III-2).

Another aspect of the present disclosure relates to a compound represented by general formula (IVA) or a salt thereof: 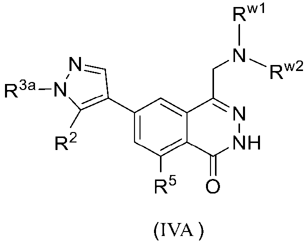 wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 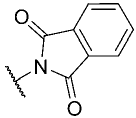 );
R², R^{3a} and R⁵ are as defined in general formula (IV).

**Table B. Typical intermediate compounds of the present disclosure, including but not limited to:**

| Compound No. | Compound structure | Name |
|---|---|---|
| 11 | 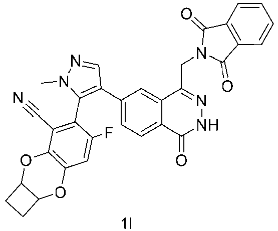 | *S*-(4-(4-((1,3-dioxoisoindolin-2-yl)methyl)-1-oxo-1,2-dihydrophthalazin-6-yl)-1-methyl-1*H-*pyrazol-5-yl)-6-fluoro-1,2,2*a*,8*a-*tetrahydrobenzo[*b*]cyclobuta[e][1,4] dioxine-4-carbonitrile **1l** |
| 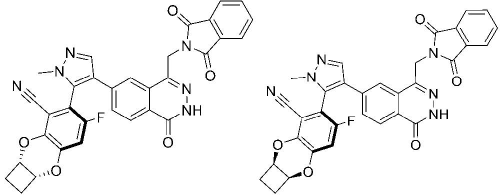 | | |
| 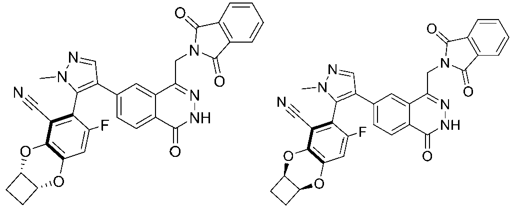 | | |
| 2d | 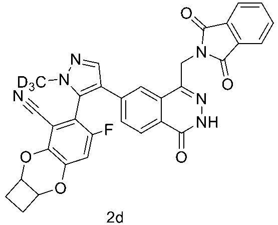 | 5-(4-(4-((1,3-dioxoisoindolin-2-yl)methyl)-1-oxo-1,2-dihydrophthalazin-6-yl)-1-(methyl-*d₃*)-1*H*-pyrazol-5-yl)-6-fluoro-1,2,2*a*,8*a-*tetrahydrobenzo[*b*]cyclobuta[e][1,4] dioxine-4-carbonitrile **2d** |
| 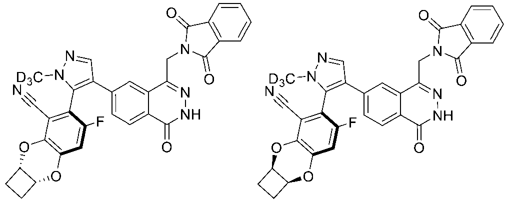 | | |
| 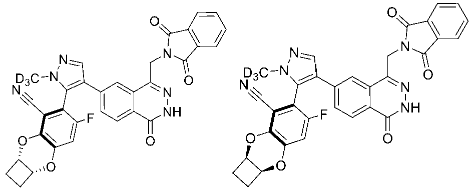 | | |
| 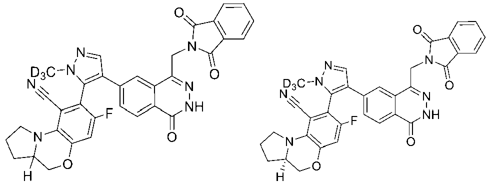 | | |
| 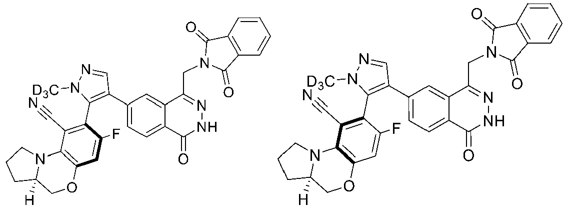 | | |
| 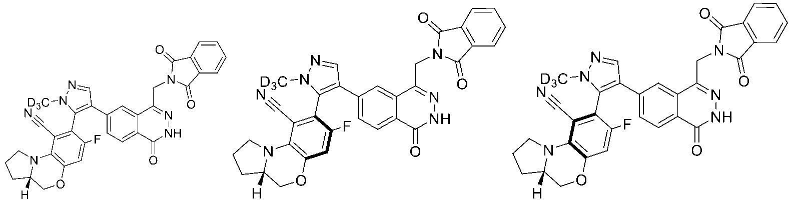 | | |
| 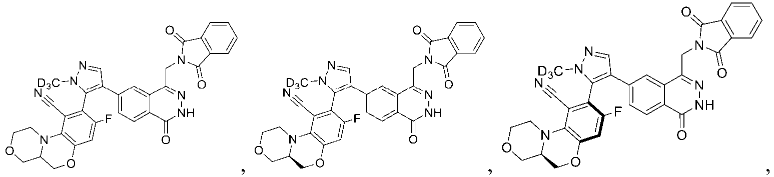 | | |
| 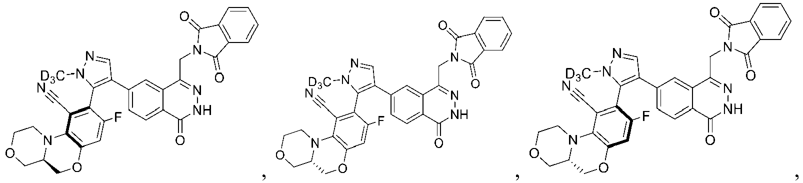 | | |
| 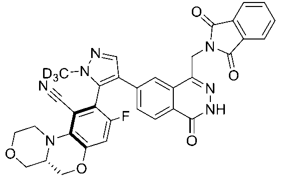 | | |
| 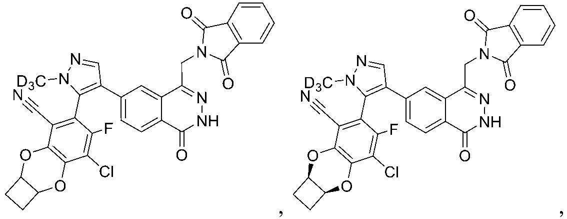 | | |
| 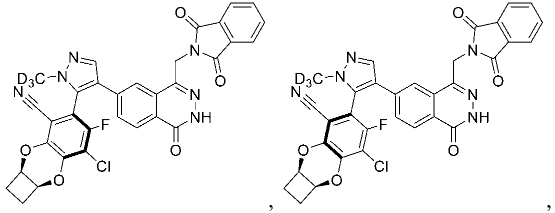 | | |
| 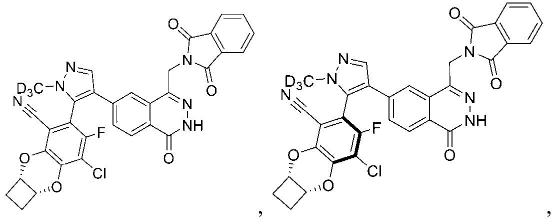 | | |
| 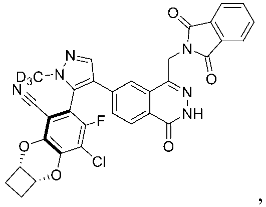 | | |
| 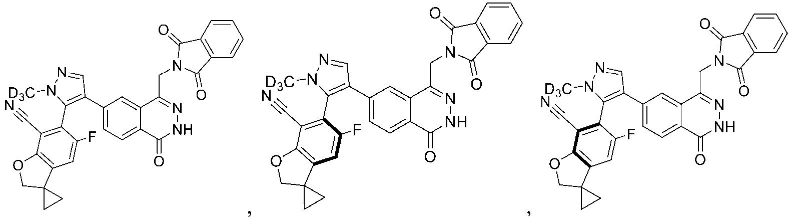 | | |

| 13e | 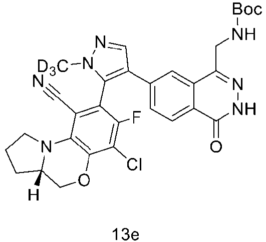 | Tert-butyl (*S*)-((7-(5-(6-chloro-9-cyano-7-fluoro-2,3,3*a*,4-tetrahydro-1*H*-benzo[*b*]pyrrolo[1,2-*d*][1,4]oxazin-8-yl)-1-(methyl-*d₃*)-1*H*-pyrazol-4-yl)-4-oxo-3,4-dihydrophthalazin-1-yl)methyl)carbamate 13e |
|---|---|---|
| 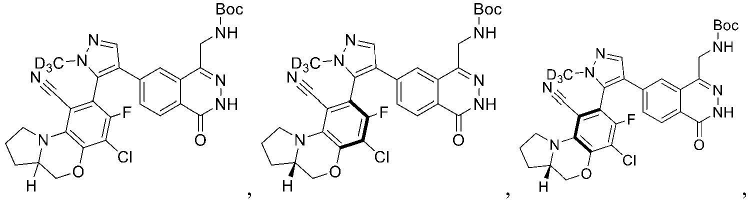 | | |
| 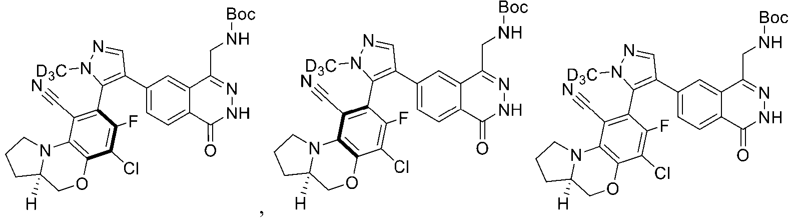 | | |

Another aspect of the present disclosure relates to a method for preparing the compound represented by general formula (I) or the pharmaceutically acceptable salt thereof, wherein the method comprises: 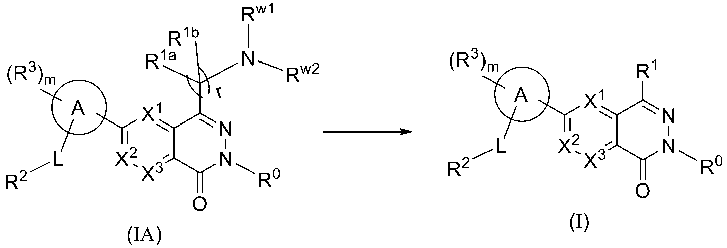 removing an amino protecting group from a compound represented by general formula (IA) or a salt thereof to obtain the compound represented by general formula (I) or the pharmaceutically acceptable salt thereof,
wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 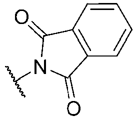 );
R¹ is-(CR^{1a}R^{1b})r-NH₂;
ring A, L, X¹, X², X³, R⁰, R^{1a}, R^{1b}, R², R³, m and r are as defined in general formula (I).

Another aspect of the present disclosure relates to a method for preparing the compound represented by general formula (II) or the pharmaceutically acceptable salt thereof, wherein the method comprises: 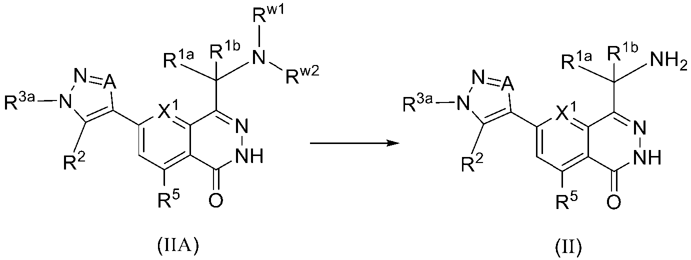 removing an amino protecting group from a compound represented by general formula (IIA) or a salt thereof to obtain the compound represented by general formula (II) or the pharmaceutically acceptable salt thereof,
wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 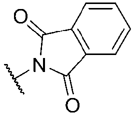 );
A, X¹, R^{1a}, R^{1b}, R², R^{3a} and R⁵ are as defined in general formula (II).

Another aspect of the present disclosure relates to a method for preparing the compound represented by general formula (III) or the pharmaceutically acceptable salt thereof, wherein the method comprises: 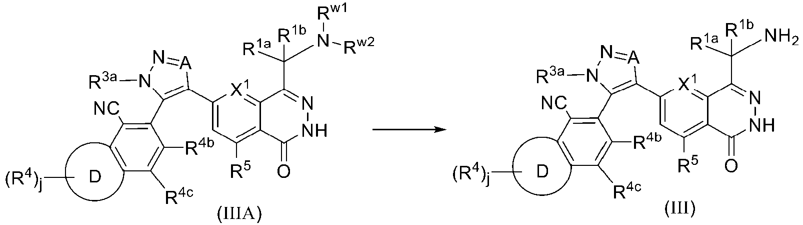 removing an amino protecting group from a compound represented by general formula (IIIA) or a salt thereof to obtain the compound represented by general formula (III) or the pharmaceutically acceptable salt thereof,
wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 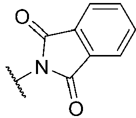 );
ring D, A, X¹, R^{1a}, R^{1b}, R^{3a}, R⁴, R^{4b}, R^{4c}, R⁵ and j are as defined in general formula (III).

Another aspect of the present disclosure relates to a method for preparing the compound represented by general formula (III-1) or the pharmaceutically acceptable salt thereof, wherein the method comprises: 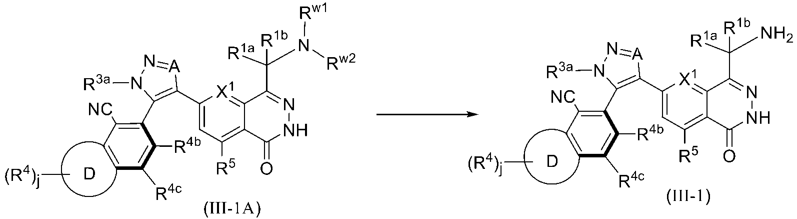 removing an amino protecting group from a compound represented by general formula (III-1A) or a salt thereof to obtain the compound represented by general formula (III-1) or the pharmaceutically acceptable salt thereof,
wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 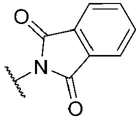 );
ring D, A, X¹, R^{1a}, R^{1b}, R^{3a}, R⁴, R^{4b}, R^{4c}, R⁵ and j are as defined in general formula (III-1).

Another aspect of the present disclosure relates to a method for preparing the compound represented by general formula (III-2) or the pharmaceutically acceptable salt thereof, wherein the method comprises: 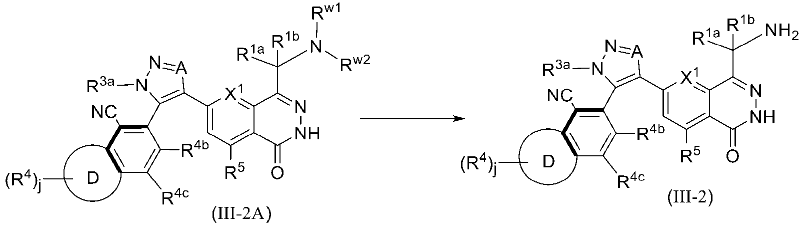 removing an amino protecting group from a compound represented by general formula (III-2A) or a salt thereof to obtain the compound represented by general formula (III-2) or the pharmaceutically acceptable salt thereof,
wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 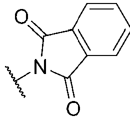 );
ring D, A, X¹, R^{1a}, R^{1b}, R^{3a}, R⁴, R^{4b}, R^{4c}, R⁵ and j are as defined in general formula (III-2).

Another aspect of the present disclosure relates to a method for preparing the compound represented by general formula (IV) or the pharmaceutically acceptable salt thereof, wherein the method comprises: 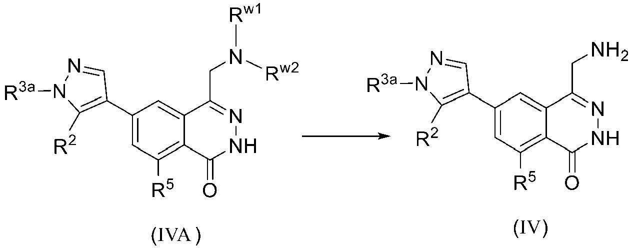 removing an amino protecting group from a compound represented by general formula (IVA) or a salt thereof to obtain the compound represented by general formula (IV) or the pharmaceutically acceptable salt thereof,
wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 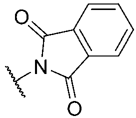 );
R², R^{3a} and R⁵ are as defined in general formula (IV).

Another aspect of the present disclosure relates to a method for preparing the compounds represented by general formula (III-1) and general formula (III-2) or the pharmaceutically acceptable salts thereof, wherein the method comprises: 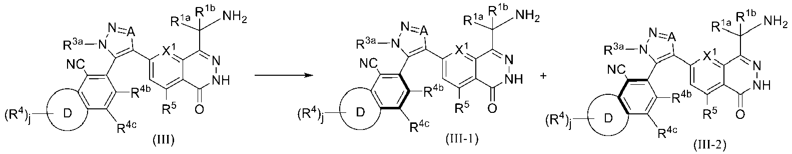 subjecting the compound represented by general formula (III) or the pharmaceutically acceptable salt thereof to a resolution, so as to obtain the compounds represented by general formula (III-1) and general formula (III-2) or the pharmaceutically acceptable salts thereof,
wherein:
ring D, A, X¹, R^{1a}, R^{1b}, R^{3a}, R⁴, R^{4b}, R^{4c}, R⁵ and j are as defined in general formula (III-1) or general formula (III-2).

Another aspect of the present disclosure relates to a pharmaceutical composition, wherein the pharmaceutical composition comprises the compounds represented by general formula (I), general formula (II), general formula (III), general formula (III-1), general formula (III-2) or general formula (IV) and the compounds shown in Table A or the pharmaceutically acceptable salts thereof according to the present disclosure, and one or more pharmaceutically acceptable carriers, diluents or excipients.

The present disclosure further relates to the use of the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same in the preparation of a PRMT5 inhibitor.

The present disclosure further relates to the use of the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same in the preparation of a PRMT5-MTA complex inhibitor.

The present disclosure further relates to the use of the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same in the preparation of a medicament for treating and/or preventing an MTAP-deficient and/or MTA-accumulation associated disease.

The present disclosure further relates to the use of the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same in the preparation of a medicament for treating and/or preventing a PRMT5-mediated disease or condition.

The present disclosure further relates to the use of the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same in the preparation of a medicament for treating and/or preventing cancer.

The present disclosure further relates to a method for inhibiting PRMT5, wherein the method comprises administering to a patient in need thereof a therapeutically effective amount of the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same.

The present disclosure further relates to a method for inhibiting a PRMT5-MTA complex, wherein the method comprises administering to a patient in need thereof a therapeutically effective amount of the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same.

The present disclosure further relates to a method for treating and/or preventing an MTAP-deficient and/or MTA-accumulation associated disease, wherein the method comprises administering to a patient in need thereof a therapeutically effective amount of the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same.

The present disclosure further relates to a method for treating and/or preventing a PRMT5-mediated disease or condition, wherein the method comprises administering to a patient in need thereof an inhibitory effective amount of the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same.

The present disclosure further relates to a method for treating and/or preventing cancer, wherein the method comprises administering to a patient in need thereof a therapeutically effective amount of the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same.

The present disclosure further relates to the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same, for use as a medicament.

The present disclosure further relates to the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same, for use as a PRMT5 inhibitor.

The present disclosure further relates to the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same, for use as a PRMT5-MTA complex inhibitor.

The present disclosure further relates to the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same, for use in the inhibition of PRMT5.

The present disclosure further relates to the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same, for use in the inhibition of a PRMT5-MTA complex.

The present disclosure further relates to the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same, for use in the treatment and/or prevention of an MTAP-deficient and/or MTA-accumulation associated disease.

The present disclosure further relates to the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same, for use in the treatment and/or prevention of a PRMT5-mediated disease or condition.

The present disclosure further relates to the compounds represented by general formula (I), general formula (II), general formula (III), general formula (III-1), general formula (III-2) or general formula (IV) and the compounds shown in Table A or the pharmaceutically acceptable salts thereof, or the pharmaceutical composition comprising same, for use in the treatment and/or prevention of cancer.

In some embodiments, the PRMT5-mediated disease or condition described in the present disclosure is cancer.

In some embodiments, the MTAP-deficient and/or MTA-accumulation associated disease described in the present disclosure is cancer.

In some embodiments, the cancer described in the present disclosure is selected from lung cancer (such as non-small cell lung cancer), kidney cancer, liver cancer (such as liver cell carcinoma), head and neck cancer, oesophageal cancer (also known as oesophageal carcinoma), lymphoma (such as diffuse large B cell lymphoma, Hodgkin lymphoma, non-Hodgkin lymphoma, mantle cell lymphoma, lymphoid malignancies of T-cell or B-cell origin, and follicular lymphoma), spongioblastoma, glioblastoma multiforme, colorectal cancer (such as colon cancer and rectal cancer), malignant peripheral nerve sheath tumour (MPNST, also known as malignant schwannoma), melanoma, stomach cancer, pancreatic cancer, cholangiocarcinoma, bladder cancer, breast cancer, ovarian cancer, vaginal cancer, cervical cancer, endometrial cancer, prostate cancer, testicular cancer, seminoma, myeloma (such as multiple myeloma), leukaemia (such as acute leukaemia, chronic leukaemia, myeloid leukaemia, myelofibrosis, and erythroleukaemia), acoustic neuroma, basal cell carcinoma, brain cancer, bronchial cancer, sarcoma (such as chondrosarcoma, soft tissue sarcoma, fibrosarcoma, leiomyosarcoma, liposarcoma, lymphangiosarcoma, myxosarcoma, osteogenic sarcoma, rhabdomyosarcoma, and Ewing sarcoma), choriocarcinoma, craniopharyngioma, cystadenocarcinoma, haemangioendothelioma, ependymoma, epithelial carcinoma, glioma, astrocytoma, hemangioblastoma, medulloblastoma, meningioma, mesothelioma, neuroblastoma, bone cancer, nasopharyngeal carcinoma, oral cancer, laryngeal cancer, thyroid cancer, retinoblastoma, skin cancer, squamous cell carcinoma (such as head and neck squamous cell carcinoma), synovial sarcoma, hidradenocarcinoma and myelodysplastic syndrome; in some embodiments, the cancer is selected from ovarian cancer, lymphoma, pancreatic cancer, bladder cancer, stomach cancer, colorectal cancer, cholangiocarcinoma, mesothelioma, malignant peripheral nerve sheath tumour (MPNST), glioblastoma multiforme and lung cancer; in some embodiments, the cancer is selected from lung cancer, mesothelioma, malignant peripheral nerve sheath tumour (MPNST) and pancreatic cancer; in some embodiments, the cancer is selected from liver cancer, breast cancer, skin cancer, bladder cancer, pancreatic cancer, and head and neck cancer.

In some embodiments, the cancer described in the present disclosure is MTAP-related cancer.

In some embodiments, the lung cancer described in the present disclosure is non-small cell lung cancer.

In another aspect, the cancer described in the present disclosure is selected from the group consisting of:
heart: sarcoma (angiosarcoma, fibrosarcoma, rhabdomyosarcoma, or liposarcoma), myxoma, rhabdomyoma, fibroma, lipoma and teratoma;
lung: lung bronchogenic carcinoma (squamous cell carcinoma, small cell undifferentiated carcinoma, large cell undifferentiated carcinoma, or adenocarcinoma), alveolar (bronchiolar) carcinoma, bronchial adenoma, sarcoma, lymphoma, chondromatous hamartoma, and mesothelioma;
gastrointestinal tract: oesophagus (squamous cell carcinoma, adenocarcinoma, leiomyosarcoma, or lymphoma), stomach (carcinoma, lymphoma, or leiomyosarcoma), pancreas (ductal adenocarcinoma, insulinoma, glucagonoma, gastrinoma, carcinoid tumour, or VIPoma), small intestine (adenocarcinoma, lymphoma, carcinoid tumour, Kaposi sarcoma, leiomyoma, haemangioma, lipoma, neurofibroma, or fibroma), large intestine (adenocarcinoma, tubular adenoma, villous adenoma, hamartoma, or leiomyoma);
urogenital tract: kidneys (adenocarcinoma, Wilms tumour (nephroblastoma), lymphoma, or leukaemia), bladder and urethra (squamous cell carcinoma, transitional cell carcinoma, or adenocarcinoma), prostate (adenocarcinoma, or sarcoma), testes (seminoma, teratoma, embryonal carcinoma, teratoma, choriocarcinoma, sarcoma, stromal cell carcinoma, fibroma, fibroadenoma, adenomatoid tumour, or lipoma);
liver: liver cancer (liver cell carcinoma), cholangiocarcinoma, hepatoblastoma, angiosarcoma, hepatocellular adenoma, or haemangioma;
biliary tract: gallbladder carcinoma, ampullary carcinoma, or cholangiocarcinoma;
bone: osteoblastic sarcoma (osteosarcoma), fibrosarcoma, malignant fibrous histiocytoma, chondrosarcoma, Ewing sarcoma, malignant lymphoma (reticular cell sarcoma), multiple myeloma, malignant giant cell tumour, chordoma, osteochronfroma (osteocartilaginous exostosis), benign chondroma, chondroblastoma, chondromyxofibroma, osteoid osteoma and giant cell tumour;
nervous system: skull (osteoma, haemangioma, granuloma, xanthoma, or osteitis deformans), meninges (meningioma, meningiosarcoma, or gliomatosis), brain (astrocytoma, medulloblastoma, glioma, ependymoma, germ cell tumour, glioblastoma multiforme, oligodendroglioma, schwannoma (such as malignant peripheral nerve sheath tumour), retinoblastoma, or congenital tumours), spinal neurofibroma, meningioma, glioma, or sarcoma;
gynaecological: uterus (endometrial cancer), cervix (cervical cancer, or pre-tumour cervical dysplasia), ovary (ovarian cancer (serous cystadenocarcinoma, mucinous cystadenocarcinoma, or unclassified carcinoma), granulosa-thecal cell tumour, Sertoli-stromal cell tumour, dysgerminoma, or malignant teratoma), vulva (squamous cell carcinoma, intraepithelial carcinoma, adenocarcinoma, fibrosarcoma, or melanoma), vagina (clear cell carcinoma, squamous cell carcinoma, botryoid sarcoma (embryonal rhabdomyosarcoma), fallopian tube (carcinoma);
haematological: blood (myeloid leukaemia (acute and chronic), acute lymphoblastic leukaemia, chronic lymphocytic leukaemia, myeloproliferative diseases, multiple myeloma, or myelodysplastic syndrome), Hodgkin disease, or non-Hodgkin lymphoma (malignant lymphoma);
skin: malignant melanoma, basal cell carcinoma, squamous cell carcinoma, Kaposi sarcoma, hydatidiform mole, lipoma, haemangioma, dermatofibroma, keloid, or psoriasis;
and adrenal glands: neuroblastoma.

In certain embodiments, the cancer is diffuse large B-cell lymphoma (DLBCL).

The active compound can be formulated into a form suitable for administration via any appropriate route, and the compositions of the present disclosure can be formulated using conventional methods with one or more pharmaceutically acceptable carriers. Therefore, the active compounds of the present disclosure can be formulated into various dosage forms for oral administration, injection (e.g., intravenous injection, intramuscular injection, or subcutaneous injection), inhalation, or insufflation. The compounds of the present disclosure can also be formulated into dosage forms such as tablets, hard or soft capsules, aqueous or oily suspensions, emulsions, injectable solutions, dispersible powders or granules, suppositories, lozenges or syrups.

As a general guide, in some embodiments, the active compound is presented in a unit dosage form, or in a manner that allows the patient to self-administer a single dose. The compounds or compositions of the present disclosure can be presented in unit dosage forms such as tablets, capsules, cachets, bottled oral solutions, powders, granules, lozenges, suppositories, reconstitutable powders, or liquid preparations. An appropriate unit dose can be 0.1-1000 mg.

In addition to the active compound, the pharmaceutical composition of the present disclosure can contain one or more auxiliary materials selected from: fillers (diluents), binders, wetting agents, disintegrants, excipients, etc. Depending on the administration method, the composition can contain 0.1% to 99% by weight of the active compound.

In some embodiments, the unit dose of the pharmaceutical composition is 0.001 mg-1000 mg.

In certain embodiments, the pharmaceutical composition comprises 0.01%-99.99% of the aforementioned compound or a pharmaceutically acceptable salt thereof, or an isotopic substitute thereof, on the basis of the total weight of the composition. In certain embodiments, the pharmaceutical composition comprises 0.1%-99.9% of the aforementioned compound or a pharmaceutically acceptable salt thereof, or an isotopic substitute thereof. In certain embodiments, the pharmaceutical composition comprises 0.5%-99.5% of the aforementioned compound or a pharmaceutically acceptable salt thereof, or an isotopic substitute thereof. In certain embodiments, the pharmaceutical composition comprises 1%-99% of the aforementioned compound or a pharmaceutically acceptable salt thereof, or an isotopic substitute thereof. In certain embodiments, the pharmaceutical composition comprises 2%-98% of the aforementioned compound or a pharmaceutically acceptable salt thereof, or an isotopic substitute thereof.

In certain embodiments, the pharmaceutical composition comprises 0.01%-99.99% of the pharmaceutically acceptable excipient, on the basis of the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

The tablet contains an active ingredient, and a non-toxic, pharmaceutically acceptable excipient suitable for compounding into a tablet. These excipients can be inert excipients, granulators, disintegrants, binders, and lubricants. These tablets can be uncoated, or coated using known techniques to mask the taste of the drug or provide a sustained-release effect over an extended period by delaying disintegration and absorption in the gastrointestinal tract.

Oral preparations can also be provided as soft gelatine capsules in which the active ingredient is mixed with an inert solid diluent or in which the active ingredient is mixed with a water-soluble carrier or an oil-based vehicle.

The aqueous suspension contains an active substance, and an excipient suitable for compounding into an aqueous suspension. Such excipients are suspending agents, dispersants, or wetting agents. The aqueous suspension can also contain one or more preservatives, one or more colourants, one or more flavouring agents, and one or more sweeteners.

The oil suspension can be prepared by suspending active ingredients in vegetable oil or mineral oil. The oil suspension can contain thickeners. The above-described sweeteners and flavouring agents can be added to provide a palatable preparation. These compositions can be preserved by adding antioxidants.

The pharmaceutical composition of the present disclosure can also be in the form of an oil-in-water emulsion. The oil phase can be vegetable oil, mineral oil, or a mixture thereof. Suitable emulsifiers can be naturally occurring phospholipids, and emulsions can also contain sweeteners, flavouring agents, preservatives, and antioxidants. Such preparations can also contain demulcents, preservatives, colourants, and antioxidants.

The pharmaceutical composition of the present disclosure can be in the form of a sterile injectable aqueous solution. Acceptable vehicles or solvents that can be used include water, Ringer's solution, and isotonic sodium chloride solution. Sterile injectable preparations can be sterile injectable oil-in-water microemulsions in which the active ingredient is dissolved in the oil phase. The injectable solutions or microemulsions can be injected into the patient's bloodstream through local high-volume injection. Alternatively, the solutions and microemulsions are preferably administered in a manner that maintains a constant circulating concentration of the compounds of the present disclosure. To maintain such a constant concentration, a continuous intravenous delivery device can be used. An example of such a device is the Deltec CADD-PLUS. TM. 5400 intravenous injection pump.

The pharmaceutical composition of the present disclosure can be in the form of a sterile injectable water or an oil suspension for intramuscular and subcutaneous administration. The suspension can be prepared using the above-described suitable dispersants or wetting agents and suspending agents according to known techniques. Sterile injectable preparations can also be sterile injectable solutions or suspensions prepared in non-toxic diluents or solvents that are acceptable for parenteral administration. Furthermore, sterile fixed oils can conveniently be used as solvents or suspension media. For this purpose, any blended fixed oil can be used. In addition, fatty acids can also be used in the preparation of injections.

The compounds of the present disclosure can be administered in suppository form for rectal administration. These pharmaceutical compositions can be prepared by mixing the drug with a suitable, non-irritating excipient that is solid at normal temperatures but liquid in the rectum, and thus dissolves in the rectum to release the drug.

The compounds of the present disclosure can be prepared by adding water to form water-dispersible powders and granules. These pharmaceutical compositions can be prepared by mixing the active ingredient with a dispersant or a wetting agent, a suspending agent, or one or more preservatives.

As will be appreciated by a person skilled in the art, the administration dosage of the drug depends on a variety of factors, including, but not limited to: the activity of the specific compound used, the patient's age, weight, health status, behaviour and diet, administration time, administration method, excretion rate, combination of drugs, and severity of disease. Furthermore, the optimal treatment method, such as the mode of treatment, the daily dosage of the compound, or the type of pharmaceutically acceptable salts, can be validated by conventional therapeutic protocols.

### Description of Terminology

Unless stated to the contrary, the terms used in the description and claims have the following meanings.

The term "alkyl" refers to a saturated, straight or branched aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkyl). In some embodiments, the alkyl has 1 to 12 carbon atoms (i.e., C₁₋₁₂ alkyl). In some embodiments, the alkyl has 1 to 6 carbon atoms (i.e., C₁₋₆ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, etc. The alkyl can be substituted or unsubstituted. When substituted, the alkyl can be substituted at any available point of attachment, and in some embodiments, the substituents are selected from one or more of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkylene" refers to a divalent alkyl group, wherein the alkyl, as defined above, has 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkylene). In some embodiments, the alkylene has 1 to 12 carbon atoms (i.e., C₁₋₁₂ alkylene). In some embodiments, the alkylene has 1 to 6 carbon atoms (i.e., C₁₋₆ alkylene). Non-limiting examples include: -CH₂-, -CH(CH₃)-, -C(CH₃)₂-, -CH₂CH₂-, -CH(CH₂CH₃)-, -CH₂CH(CH₃)-, -CH₂C(CH₃)₂-, -CH₂CH₂CH₂-, -CH₂CH₂CH₂CH₂-, etc. The alkylene can be substituted or unsubstituted. When substituted, the alkylene can be substituted at any available point of attachment, and in some embodiments, the substituents are selected from one or more of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkenyl" refers to an alkyl group containing at least one carbon-carbon double bond in the molecule, wherein the alkyl, as defined above, has 2 to 12 (e.g., 2, 3, 4, 5, 6, 7, 8, 9, 10, 11, or 12) carbon atoms (i.e., C₂₋₁₂ alkenyl). In some embodiments, the alkenyl has 2 to 6 carbon atoms (i.e., C₂₋₆ alkenyl). Non-limiting examples include: ethenyl, propenyl, isopropenyl, butenyl, etc. The alkenyl can be substituted or unsubstituted. When substituted, the alkenyl can be substituted at any available point of attachment, and in some embodiments, the substituents are selected from one or more of a D atom, alkoxy, halogen, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkynyl" refers to an alkyl group containing at least one carbon-carbon triple bond in the molecule, wherein the alkyl, as defined above, has 2 to 12 (e.g., 2, 3, 4, 5, 6, 7, 8, 9, 10, 11, or 12) carbon atoms (i.e., C₂₋₁₂ alkynyl). In some embodiments, the alkynyl has 2 to 6 carbon atoms (i.e., C₂₋₆ alkynyl). Non-limiting examples include: ethynyl, propynyl, butynyl, pentynyl, hexynyl, etc. The alkynyl can be substituted or unsubstituted. When substituted, the alkynyl can be substituted at any available point of attachment, and in some embodiments, the substituents are selected from one or more of a D atom, alkoxy, halogen, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkoxy" refers to -O-(alkyl), wherein the alkyl is as defined above. Non-limiting examples include: methoxy, ethoxy, propoxy, butoxy, etc. The alkoxy can be substituted or unsubstituted. When substituted, the alkoxy can be substituted at any available point of attachment, and in some embodiments, the substituents are selected from one or more of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "cycloalkyl" refers to a saturated or partially unsaturated monocyclic all-carbon ring (i.e., monocyclic cycloalkyl) or polycyclic system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). In some embodiments, the cycloalkyl has 3 to 12 ring atoms (i.e., 3- to 12-membered cycloalkyl). In some embodiments, the cycloalkyl has 3 to 8 ring atoms (i.e., 3- to 8-membered cycloalkyl). In some embodiments, the cycloalkyl has 3 to 6 ring atoms (i.e., 3- to 6-membered cycloalkyl).

Non-limiting examples of the monocyclic cycloalkyl include: cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.

The polycyclic cycloalkyl includes: spirocycloalkyl, fused cycloalkyl and bridged cycloalkyl.

The bicyclic cycloalkyl includes: monospirocycloalkyl, bicyclic fused cycloalkyl and bicyclic bridged cycloalkyl.

The term "spirocycloalkyl" refers to a polycyclic system in which the rings share one carbon atom (called spiro atom), wherein the ring can contain one or more double bonds, or the ring can contain one or more heteroatoms selected from nitrogen, oxygen and sulphur (the nitrogen can optionally be oxidised, i.e., to form nitrogen oxides; and the sulphur can optionally be oxidised, i.e., to form sulphoxides or sulphones, but excluding -O-O-, -O-S- or -S-S-), provided that at least one all-carbon ring is present and the point of attachment is on the all-carbon ring. The spirocycloalkyl has 5 to 20 (e.g., 5, 6, 7, 8, 9, 10, 11, 12, 13, 14, 15, 16, 17, 18, 19, or 20) ring atoms (i.e., 5- to 20-membered spirocycloalkyl). In some embodiments, the spirocycloalkyl has 6 to 14 ring atoms (i.e., 6- to 14-membered spirocycloalkyl). In some embodiments, the spirocycloalkyl has 7 to 10 ring atoms (i.e., 7- to 10-membered spirocycloalkyl). The spirocycloalkyl includes monospirocycloalkyl and polyspirocycloalkyl (such as dispirocycloalkyl); in some embodiments, the spirocycloalkyl is monospirocycloalkyl or dispirocycloalkyl; and in some embodiments, the spirocycloalkyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospirocycloalkyl. Non-limiting examples include: 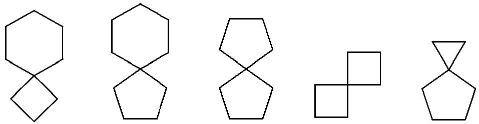 (the point of attachment can be at any position); 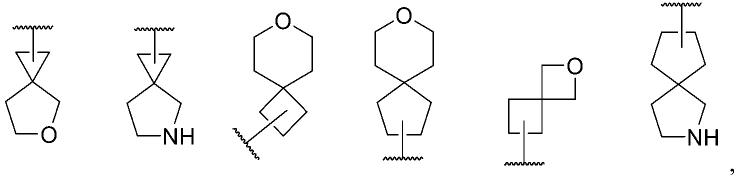 etc. Taking the ring D of the present disclosure as an example, non-limiting examples of monospirocycloalkyl further include 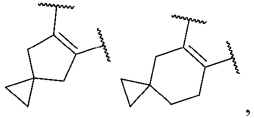 etc.

The term "fused cycloalkyl" refers to a polycyclic system in which the rings share two adjacent carbon atoms, formed by fusing monocyclic cycloalkyl with one or more monocyclic cycloalkyl, or by fusing monocyclic cycloalkyl with one or more of heterocyclyl, aryl, or heteroaryl, wherein the point of attachment is on the monocyclic cycloalkyl, and the ring can contain one or more double bonds. The fused cycloalkyl has 5 to 20 (e.g., 5, 6, 7, 8, 9, 10, 11, 12, 13, 14, 15, 16, 17, 18, 19, or 20) ring atoms (i.e., 5- to 20-membered fused cycloalkyl). In some embodiments, the fused cycloalkyl has 6 to 14 ring atoms (i.e., 6- to 14-membered fused cycloalkyl). In some embodiments, the fused cycloalkyl has 7 to 10 ring atoms (i.e., 7- to 10-membered fused cycloalkyl). The fused cycloalkyl includes bicyclic fused cycloalkyl and polycyclic fused cycloalkyl (such as tricyclic fused cycloalkyl and tetracyclic fused cycloalkyl); in some embodiments, the fused cycloalkyl is bicyclic fused cycloalkyl or tricyclic fused cycloalkyl; and in some embodiments, the fused cycloalkyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicyclic fused cycloalkyl. Non-limiting examples include: 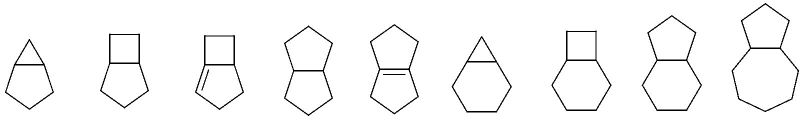 (the point of attachment can be at any position); 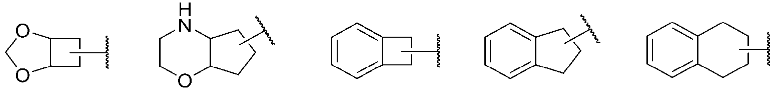 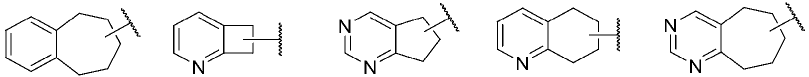 etc. Taking the ring D of the present disclosure as an example, non-limiting examples of bicyclic fused cycloalkyl further include 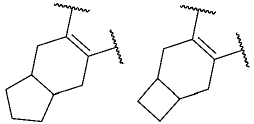 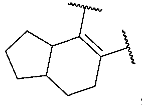 , etc.

The term "bridged cycloalkyl" refers to an all-carbon polycyclic system in which the rings share two non-adjacent carbon atoms, wherein the ring can contain one or more double bonds. The bridged cycloalkyl has 5 to 20 (e.g., 5, 6, 7, 8, 9, 10, 11, 12, 13, 14, 15, 16, 17, 18, 19, or 20) carbon atoms (i.e., 5- to 20-membered bridged cycloalkyl). In some embodiments, the bridged cycloalkyl has 6 to 14 carbon atoms (i.e., 6- to 14-membered bridged cycloalkyl). In some embodiments, the bridged cycloalkyl has 7 to 10 carbon atoms (i.e., 7- to 10-membered bridged cycloalkyl). The bridged cycloalkyl includes bicyclic bridged cycloalkyl and polycyclic bridged cycloalkyl (such as tricyclic bridged cycloalkyl and tetracyclic bridged cycloalkyl), and in some embodiments, the bridged cycloalkyl is bicyclic bridged cycloalkyl or tricyclic bridged cycloalkyl. Non-limiting examples include: 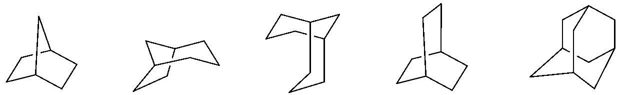 (the point of attachment can be at any position). Taking the ring D of the present disclosure as an example, non-limiting examples of bicyclic bridged cycloalkyl further include 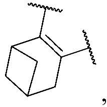 etc.

The cycloalkyl can be substituted or unsubstituted. When substituted, the cycloalkyl can be substituted at any available point of attachment, and in some embodiments, the substituents are selected from one or more of a D atom, halogen, alkyl, alkoxy, haloalkyl, haloalkoxy, cycloalkyloxy, heterocyclyloxy, hydroxyl, hydroxyalkyl, oxo, cyano, amino, nitro, cycloalkyl, heterocyclyl, aryl and heteroaryl.

The term "tricyclic cycloalkyl" refers to a ring system containing 3 rings, wherein the point of attachment is on the all-carbon ring. Taking the R² of the present disclosure as an example, non-limiting examples of "tricyclic cycloalkyl" include 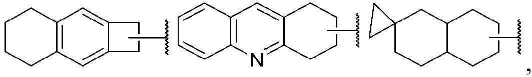 etc.

The term "heterocyclyl" refers to a saturated or partially unsaturated monocyclic heterocycle (i.e., monocyclic heterocyclyl) or polycyclic heterocyclic system (i.e., polycyclic heterocyclyl), wherein the ring contains at least one (e.g., 1, 2, 3 or 4) heteroatoms selected from nitrogen, oxygen and sulphur (the nitrogen can optionally be oxidised, i.e., to form nitrogen oxides; and the sulphur can optionally be oxidised, i.e., to form sulphoxides or sulphones, but excluding -O-O-, -O-S- or -S-S-). The heterocyclyl has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl). In some embodiments, the heterocyclyl has 3 to 12 ring atoms (i.e., 3- to 12-membered heterocyclyl). In some embodiments, the heterocyclyl has 3 to 8 ring atoms (i.e., 3- to 8-membered heterocyclyl). In some embodiments, the heterocyclyl has 3 to 6 ring atoms (i.e., 3- to 6-membered heterocyclyl). In some embodiments, the heterocyclyl has 5 or 6 ring atoms (i.e., 5- or 6-membered heterocyclyl).

Non-limiting examples of the monocyclic heterocycly include: pyrrolidyl, dihydropyrrolyl, dihydrofuryl, tetrahydropyranyl, 1,2,3,6-tetrahydropyridyl, piperidyl, piperazinyl, morpholinyl, thiomorpholinyl, homopiperazinyl, etc.

The polycyclic heterocyclyl includes spiroheterocyclyl, fused heterocyclyl and bridged heterocyclyl. The term "spiroheterocyclyl" refers to a polycyclic heterocyclic system in which the rings share one atom (called spiro atom), wherein the ring can contain one or more double bonds, and the ring contains at least one (e.g., 1, 2, 3 or 4) heteroatoms selected from nitrogen, oxygen and sulphur (the nitrogen can optionally be oxidised, i.e., to form nitrogen oxides; and the sulphur can optionally be oxidised, i.e., to form sulphoxides or sulphones, but excluding - O-O-, -O-S- or -S-S-), provided that at least one monocyclic heterocycly is present and the point of attachment is on the monocyclic heterocycly. The spiroheterocyclyl has 5 to 20 (e.g., 5, 6, 7, 8, 9, 10, 11, 12, 13, 14, 15, 16, 17, 18, 19, or 20) ring atoms (i.e., 5- to 20-membered spiroheterocyclyl). In some embodiments, the spiroheterocyclyl has 6 to 14 ring atoms (i.e., 6- to 14-membered spiroheterocyclyl). In some embodiments, the spiroheterocyclyl has 7 to 10 ring atoms (i.e., 7- to 10-membered spiroheterocyclyl). In some embodiments, the spiroheterocyclyl has 7 to 9 ring atoms (i.e., 7- to 9-membered spiroheterocyclyl). In some embodiments, the spiroheterocyclyl has 7 or 8 ring atoms (i.e., 7- or 8-membered spiroheterocyclyl). The spiroheterocyclyl includes monospiroheterocyclyl and polyspiroheterocyclyl (such as dispiroheterocyclyl); in some embodiments, the spiroheterocyclyl is monospiroheterocyclyl or dispiroheterocyclyl; and in some embodiments, the spiroheterocyclyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospiroheterocyclyl. Non-limiting examples include: 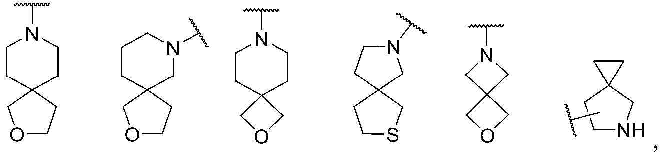 etc. Taking the ring D of the present disclosure as an example, non-limiting examples of monospiroheterocyclyl further include 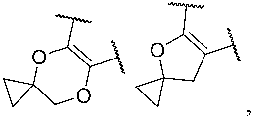 etc.

The term "fused heterocyclyl" refers to a polycyclic heterocyclic system in which the rings share two adjacent atoms, wherein the ring can contain one or more double bonds, and the ring contains at least one (e.g., 1, 2, 3 or 4) heteroatoms selected from nitrogen, oxygen and sulphur (the nitrogen can optionally be oxidised, i.e., to form nitrogen oxides; and the sulphur can optionally be oxidised, i.e., to form sulphoxides or sulphones, but excluding -O-O-, -O-S- or - S-S-), formed by fusing monocyclic heterocyclyl with one or more monocyclic heterocyclyl, or by fusing monocyclic heterocyclyl with one or more of cycloalkyl, aryl or heteroaryl, wherein the point of attachment is on the monocyclic heterocycly. The fused heterocyclyl has 5 to 20 (e.g., 5, 6, 7, 8, 9, 10, 11, 12, 13, 14, 15, 16, 17, 18, 19, or 20) ring atoms (i.e., 5- to 20-membered fused heterocyclyl). In some embodiments, the fused heterocyclyl has 6 to 14 ring atoms (i.e., 6- to 14-membered fused heterocyclyl). In some embodiments, the fused heterocyclyl has 7 to 10 ring atoms (i.e., 7- to 10-membered fused heterocyclyl). In some embodiments, the fused heterocyclyl has 7 to 9 ring atoms (i.e., 7- to 9-membered fused heterocyclyl). In some embodiments, the fused heterocyclyl has 8 or 9 ring atoms (i.e., 8- or 9-membered fused heterocyclyl). The fused heterocyclyl includes bicyclic and polycyclic fused heterocyclyl (such as tricyclic fused heterocyclyl and tetracyclic fused heterocyclyl); in some embodiments, the fused heterocyclyl is bicyclic fused heterocyclyl or tricyclic fused heterocyclyl; in some embodiments, the fused heterocyclyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicyclic fused heterocyclyl. Non-limiting examples include: 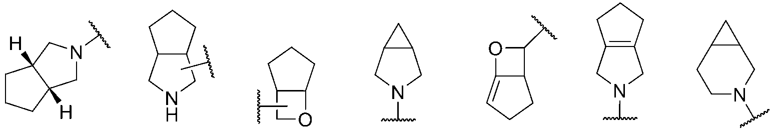 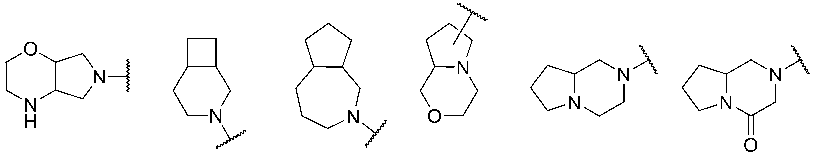 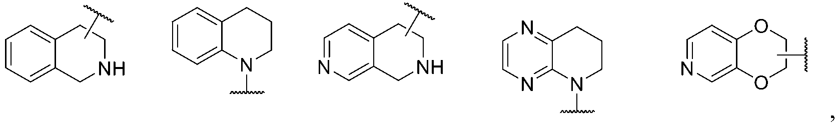 etc. Taking the ring D of the present disclosure as an example, non-limiting examples of bicyclic fused heterocyclyl further include 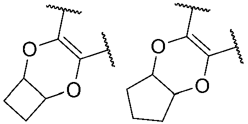 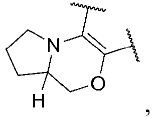 etc.

The term "bridged heterocyclyl" refers to a polycyclic heterocyclic system in which the rings share two non-adjacent atoms, wherein the ring can contain one or more double bonds, and the ring contains at least one (e.g., 1, 2, 3 or 4) heteroatoms selected from nitrogen, oxygen and sulphur (the nitrogen can optionally be oxidised, i.e., to form nitrogen oxides; and the sulphur can optionally be oxidised, i.e., to form sulphoxides or sulphones, but excluding -O-O-, -O-S- or - S-S-). The bridged heterocyclyl has 5 to 20 (e.g., 5, 6, 7, 8, 9, 10, 11, 12, 13, 14, 15, 16, 17, 18, 19, or 20) ring atoms (i.e., 5- to 20-membered bridged heterocyclyl). In some embodiments, the bridged heterocyclyl has 6 to 14 ring atoms (i.e., 6- to 14-membered bridged heterocyclyl). In some embodiments, the bridged heterocyclyl has 7 to 10 ring atoms (i.e., 7- to 10-membered bridged heterocyclyl). Based on the number of constituent rings, the bridged heterocyclyl can be classified into bicyclic bridged heterocyclyl and polycyclic bridged heterocyclyl (such as tricyclic bridged heterocyclyl and tetracyclic bridged heterocyclyl); and in some embodiments, the bridged heterocyclyl is bicyclic bridged heterocyclyl or tricyclic bridged heterocyclyl. Non-limiting examples include: 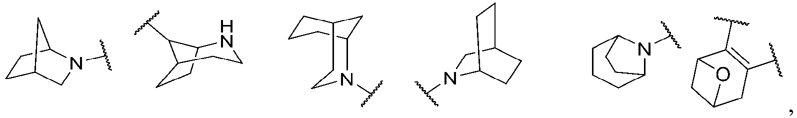 etc. Taking the ring D of the present disclosure as an example, non-limiting examples of bicyclic bridged heterocyclyl further include 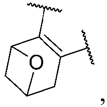 etc.

The "bicyclic heterocyclyl" includes bicyclic fused heterocyclyl, monospiroheterocyclyl and bicyclic bridged heterocyclyl; in some embodiments, the bicyclic heterocyclyl is 7- to 9-membered bicyclic heterocyclyl; in some embodiments, the bicyclic heterocyclyl is 8- or 9-membered bicyclic heterocyclyl; and in some embodiments, the bicyclic heterocyclyl is 8-membered bicyclic heterocyclyl. Taking the ring D of the present disclosure as an example, non-limiting examples of "bicyclic heterocyclyl" include:

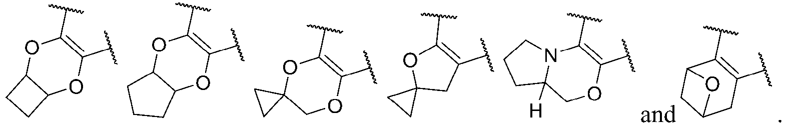

The term "tricyclic heterocyclyl" refers to a ring system containing 3 rings, wherein the point of attachment is on the monocyclic heterocyclic ring. Taking the R² of the present disclosure as an example, non-limiting examples of "tricyclic heterocyclyl" include 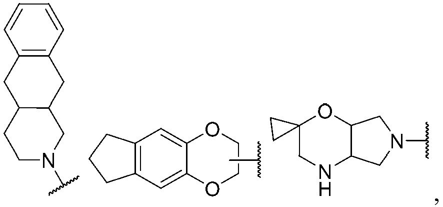 etc.

The heterocyclyl can be substituted or unsubstituted. When substituted, the heterocyclyl can be substituted at any available point of attachment, and in some embodiments, the substituents are selected from one or more of a D atom, halogen, alkyl, alkoxy, haloalkyl, haloalkoxy, cycloalkyloxy, heterocyclyloxy, hydroxyl, hydroxyalkyl, oxo, cyano, amino, nitro, cycloalkyl, heterocyclyl, aryl and heteroaryl.

The term "aryl" refers to a monocyclic all-carbon aromatic ring (i.e., monocyclic aryl) or polycyclic aromatic ring system (i.e., polycyclic aryl) having a conjugated π-electron system. The aryl has 6 to 22 (e.g., 6, 7, 8, 9, 10, 11, 12, 13, 14, 15, 16, 17, 18, 19, 20, 21, or 22) ring atoms (i.e., 6- to 22-membered aryl). In some embodiments, the aryl has 6 to 10 ring atoms (i.e., 6- to 10-membered aryl) or has 10 to 14 ring atoms (i.e., 10- to 14-membered aryl); in some embodiments, the aryl has 11 to 13 ring atoms (i.e., 11- to 13-membered aryl); in some embodiments, the aryl has 12 ring atoms (i.e., 12-membered aryl). the aryl is monocyclic aryl, such as phenyl. Non-limiting examples of the polycyclic aryl include: naphthyl, anthryl, phenanthryl, etc. The polycyclic aryl further includes phenyl fused with one or more of heterocyclyl or cycloalkyl, or naphthyl fused with one or more of heterocyclyl or cycloalkyl, wherein the point of attachment is on the phenyl or naphthyl. In such cases, the number of ring atoms still represents the number of ring atoms in the polycyclic aromatic ring system (in some embodiments, bicyclic aryl or tricyclic aryl), and non-limiting examples include: 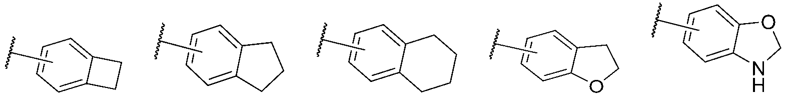 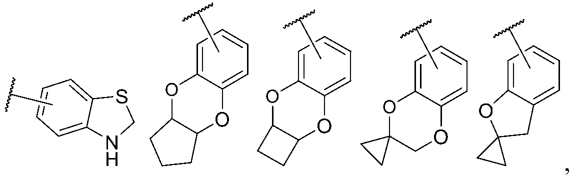 etc. Taking the ring D of the present application as an example, non-limiting examples of "bicyclic aryl" further include: 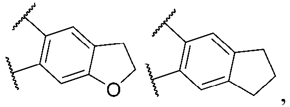 etc.

The term "tricyclic aryl" refers to a ring system containing 3 rings, wherein the point of attachment is on the phenyl or naphthyl. In some embodiments, the tricyclic aryl has 10 to 14 ring atoms (i.e., 10- to 14-membered tricyclic aryl); in some embodiments, the tricyclic aryl has 11 to 13 ring atoms (i.e., 11- to 13-membered tricyclic aryl); in some embodiments, the tricyclic aryl has 12 ring atoms (i.e., 12-membered tricyclic aryl); Taking the R² of the present disclosure as an example, non-limiting examples of "tricyclic aryl" include: 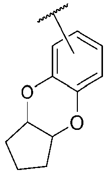 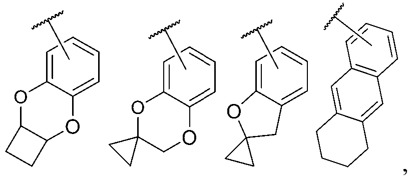 etc.

The aryl can be substituted or unsubstituted. When substituted, the aryl can be substituted at any available point of attachment, and in some embodiments, the substituents are selected from one or more of a D atom, halogen, alkyl, alkoxy, haloalkyl, haloalkoxy, cycloalkyloxy, heterocyclyloxy, hydroxyl, hydroxyalkyl, oxo, cyano, amino, nitro, cycloalkyl, heterocyclyl, aryl and heteroaryl.

The term "heteroaryl" refers to a monocyclic heteroaromatic ring (i.e., monocyclic heteroaryl) or polycyclic heteroaromatic ring system (i.e., polycyclic heteroaryl) having a conjugated π-electron system, wherein the ring contains at least one (e.g., 1, 2, 3 or 4) heteroatoms selected from nitrogen, oxygen and sulphur (the nitrogen can optionally be oxidised, i.e., to form nitrogen oxides; and the sulphur can optionally be oxidised, i.e., to form sulphoxides or sulphones, but excluding -O-O-, -O-S- or -S-S-). The heteroaryl has 5 to 22 (e.g., 5, 6, 7, 8, 9, 10, 11, 12, 13, 14, 15, 16, 17, 18, 19, 20, 21, or 22) ring atoms (i.e., 5- to 22-membered heteroaryl). In some embodiments, the heteroaryl has 5 to 10 ring atoms (i.e., 5- to 10-membered heteroaryl) or has 9 to 14 ring atoms (i.e., 9- to 14-membered heteroaryl); in some embodiments, the heteroaryl has 11 or 12 ring atoms (i.e., 11- or 12-membered heteroaryl); in some embodiments, the heteroaryl has 11 ring atoms (i.e., 11-membered heteroaryl); in some embodiments, the heteroaryl has 5 or 6 ring atoms (i.e., 5- or 6-membered heteroaryl); in some embodiments, the heteroaryl is 5-membered heteroaryl.

Non-limiting examples of the monocyclic heteroaryl include: furyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkyl pyrrolyl, pyridyl, pyrimidyl, pyridonyl, N-alkylpyridone (such as 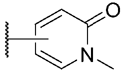 ), pyrazinyl, pyridazinyl, etc.

Non-limiting examples of the polycyclic heteroaryl (in some embodiments, 9-to 14-membered heteroaryl) include: indolyl, indazolyl, quinolyl, isoquinolyl, quinoxalinyl, phthalazinyl, benzoimidazolyl, benzothienyl, quinazolinyl, benzothiazolyl, carbazolyl, etc. The polycyclic heteroaryl further includes monocyclic heteroaryl fused with one or more aryl, wherein the point of attachment is on the aromatic ring. In such cases, the number of ring atoms still represents the number of ring atoms in the polycyclic heteroaromatic ring system. The polycyclic heteroaryl further includes monocyclic heteroaryl fused with one or more of cycloalkyl or heterocyclyl, wherein the point of attachment is on the monocyclic heteroaromatic ring. In such cases, the number of ring atoms still represents the number of ring atoms in the polycyclic heteroaromatic ring system (in some embodiments, the polycyclic heteroaryl is bicyclic heteroaryl or tricyclic heteroaryl). Non-limiting examples include: 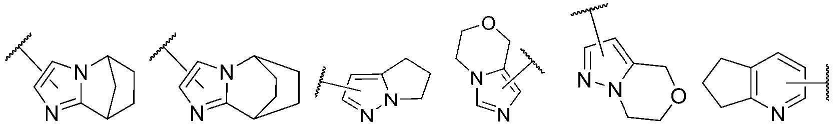 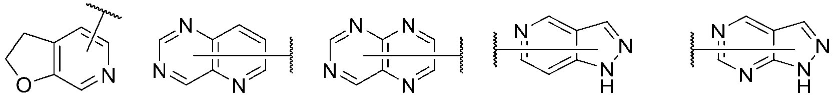 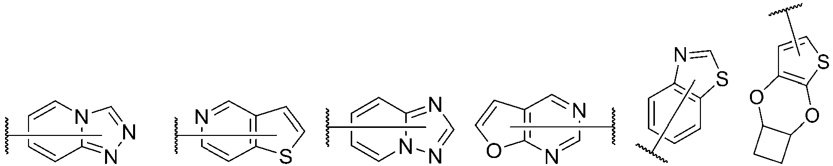 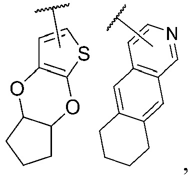 etc. Taking the ring D of the present application as an example, non-limiting examples of "bicyclic heteroaryl" further include: 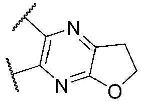 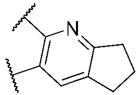 etc.

The term "tricyclic heteroaryl" refers to a ring system containing 3 rings, wherein the point of attachment is on the monocyclic heteroaromatic ring. In some embodiments, the "tricyclic heteroaryl" has 9 to 14 ring atoms (i.e., 9- to 14-membered tricyclic heteroaryl); in some embodiments, the tricyclic heteroaryl has 11 or 12 ring atoms (i.e., 11- or 12-membered tricyclic heteroaryl); in some embodiments, the tricyclic heteroaryl has 11 ring atoms (i.e., 12-membered tricyclic heteroaryl). Taking the R² of the present disclosure as an example, non-limiting examples of "tricyclic heteroaryl" include: 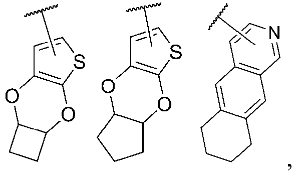 etc.

The heteroaryl can be substituted or unsubstituted. When substituted, the heteroaryl can be substituted at any available point of attachment, and in some embodiments, the substituents are selected from one or more of a D atom, halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "amino protecting group" refers to a readily removable group introduced onto an amino to keep the amino intact during reactions occurring at other sites of the molecule. Non-limiting examples include: (trimethylsilyl)ethoxymethyl, tetrahydropyranyl, tert-butoxycarbonyl (Boc), benzyloxycarbonyl (Cbz), fluorenylmethyloxycarbonyl (Fmoc), allyloxycarbonyl (Alloc), trimethylsilylethoxycarbonyl (Teoc), methoxycarbonyl, ethoxycarbonyl, phthaloyl (Pht), p-toluenesulphonyl (Tos), trifluoroacetyl (Tfa), triphenylmethyl (Trt), 2,4-dimethoxybenzyl (DMB), acetyl, benzyl, allyl, p-methoxybenzyl, etc.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "cycloalkylalkyl" refers to an alkyl group substituted with one or more cycloalkyl groups, wherein the alkyl and cycloalkyl are as defined above.

The term "heterocyclylalkyl" refers to an alkyl group substituted with one or more heterocyclyl groups, wherein the alkyl and heterocyclyl are as defined above.

The term "arylalkyl" refers to an alkyl group substituted with one or more aryl groups, wherein the alkyl and aryl are as defined above, such as -CH₂Ph.

The term "heteroarylalkyl" refers to an alkyl group substituted with one or more heteroaryl groups, wherein the alkyl and heteroaryl are as defined above. The term "alkylthio" refers to alkyl-S-, wherein the alkyl is as defined above.

The term "haloalkyl" refers to an alkyl group substituted with one or more halogen, wherein the alkyl is as defined above.

The term "haloalkoxy" refers to an alkoxy group substituted with one or more halogen, wherein the alkoxy is as defined above, such as-OCF₃.

The term "deuterioalkyl" refers to an alkyl group substituted with one or more deuterium atoms, wherein the alkyl is as defined above, such as -CD₃.

The term "hydroxyalkyl" refers to an alkyl group substituted with one or more hydroxyl groups, wherein the alkyl is as defined above, such as -CH₂OH.

The term "deuterioalkoxy" refers to an alkoxy group substituted with one or more deuterium atoms, wherein the alkoxy is as defined above, such as -OCD₃.

The term "alkoxyalkyl" refers to an alkyl group substituted with one or more alkoxy groups, wherein the alkyl and alkoxy are as defined above, such as - CH₂OCH₃.

The term "aminoalkyl" refers to an alkyl group substituted with one or more amino groups, wherein the alkyl is as defined above, such as -CH₂NH₂.

The term "methylidene" refers to=CH₂.

The term "halogen" refers to fluorine, chlorine, bromine or iodine.

The term "hydroxyl" refers to -OH.

The term "mercapto" refers to -SH.

The term "amino" refers to -NH₂.

The term "cyano" refers to -CN.

The term "nitro" refers to -NO₂.

The term "oxo" or "oxo group" refers to "=O".

The term "carbonyl" refers to C=O.

The term "carboxyl" refers to -C(O)OH.

The term "carboxylate ester group" refers to -C(O)O(alkyl), - C(O)O(cycloalkyl), (alkyl)C(O)O- or (cycloalkyl)C(O)O-, wherein the alkyl and cycloalkyl are as defined above.

The compounds of the present disclosure can exist in specific stereoisomeric forms. The term "stereoisomer" refers to isomers with the same structure but different spatial arrangements of atoms. It includes cis- and trans- (or *Z*- and *E*-) isomers, (-)- and (+)-isomers, (*R*)- and (*S*)-enantiomers, diastereomers, (*D*)- and (*L*)-isomers, tautomers, atropisomer, conformational isomers and mixtures thereof (such as mixtures of racemates and diastereomers). The substituents in the compounds of the present disclosure can contain additional asymmetric atoms. All these stereoisomer and mixtures thereof are encompassed within the scope of the present disclosure. Optically active (-)- and (+)-isomers, (*R*)- and (*S*)-enantiomers, and (*D*)- and (*L*)-isomers can be prepared by using chiral synthesis, chiral reagents or other conventional techniques. An isomer of a compound of the present disclosure can be prepared by using asymmetric synthesis or chiral auxiliaries. Alternatively, where the molecule contains a basic functional group (such as amino) or an acidic functional group (such as carboxyl), diastereomeric salts can be formed with an appropriate optically active acid or base, followed by resolution of the diastereomers using conventional methods well known in the art to obtain the pure isomers. In addition, the separation of enantiomers and diastereomers is usually performed by chromatography.

The configuration of atropisomers can be described using the nomenclature (M)- and (P)- to indicate the relative positions of substituents (see, for example, WO 2022192745 A1 and J. Med. Chem. 2022, 65, 3, 1749-1766).

Non-limiting examples of the atropisomers include 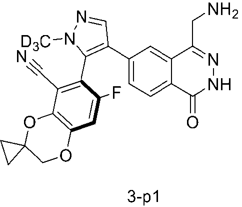 and 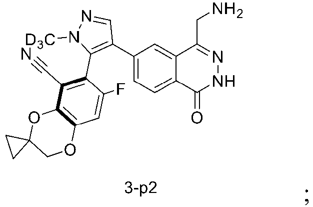 and 5-p1 and 5-p2 ( 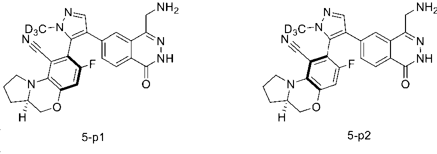 ).

In the chemical structures of the compounds of the present disclosure, the bond " 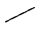 " represents an unspecified configuration; that is, if the chemical structure exists as stereoisomers, the bond " 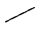 " can be " 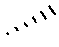 " or " 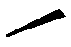 ", or both the configurations of " 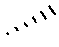 " and " 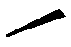 " are involved. For all carbon-carbon double bonds, both the *Z*- and *E*-forms are included, even if only one configuration is named.

The compounds of the present disclosure can also exist as different tautomeric forms, and all such forms are encompassed within the scope of the present disclosure. The term "tautomer" or "tautomeric form" refers to a structural isomer that exists in equilibrium and is easily transformed from one isomeric form to another. All possible tautomers are included, whether they exist as a single isomeric form or as a mixture in any proportion of the tautomers. Non-limiting examples include: keto-enols, imine-enamines, lactam-lactim, etc.

For example, the compound should be understood to encompass either of the following structures or a mixture of both tautomers:

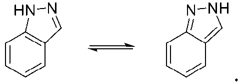

All tautomeric forms fall within the scope of the present disclosure, and the naming of the compound does not exclude any tautomer.

The compounds of the present disclosure include all suitable isotopic derivatives thereof. The term "isotopic derivative" refers to a compound in which at least one atom is replaced by an atom having the same atomic number but a different atomic mass. Examples of isotopes that can be introduced into the compounds of the present disclosure include stable and radioactive isotopes of hydrogen, carbon, nitrogen, oxygen, phosphorus, sulphur, fluorine, chlorine, bromine, iodine, etc., such as ²H (deuterium, D),³H (tritium, T), ¹¹C, ¹³C, ¹⁴C, ¹⁵N, ¹⁷O, ¹⁸O, ³²p, ³³p, ³³S, ³⁴S, ³⁵S, ³⁶S, ¹⁸F, ³⁶Cl, ⁸²Br, ¹²³I, ¹²⁴I, ¹²⁵I, ¹²⁹I and ¹³¹I. In some embodiments, the isotope is deuterium.

Deuterated drugs offer advantages over their non-deuterated counterparts, such as reduced toxicity and side effects, increased drug stability, enhanced efficacy, and prolonged biological half-life. All isotopic variations of the compounds of the present disclosure, whether radioactive or not, are encompassed within the scope of the present disclosure. Each available hydrogen atom attached to a carbon atom can be independently replaced by a deuterium atom, where such deuterium replacement can be partial or complete, and partial deuterium replacement means that at least one hydrogen atom is replaced by at least one deuterium atom.

For the compounds of the present disclosure, when a position is specifically designated as "deuterium" or "D," it should be understood that the position has a deuterium abundance at least 1000 times greater than the natural abundance of deuterium (which is 0.015%) (i.e., at least 15% deuterium incorporation). In some embodiments, the deuterium abundance in each designated deuterium atom is at least 1000 times greater than the natural abundance of deuterium (i.e., at least 15% deuterium incorporation). In some embodiments, the deuterium abundance in each designated deuterium atom is at least 2000 times greater than the natural abundance of deuterium (i.e., at least 30% deuterium incorporation). In some embodiments, the deuterium abundance in each designated deuterium atom is at least 3000 times greater than the natural abundance of deuterium (i.e., at least 45% deuterium incorporation). In some embodiments, the deuterium abundance in each designated deuterium atom is at least 3340 times greater than the natural abundance of deuterium (i.e., at least 50.1% deuterium incorporation). In some embodiments, the deuterium abundance in each designated deuterium atom is at least 3500 times greater than the natural abundance of deuterium (i.e., at least 52.5% deuterium incorporation). In some embodiments, the deuterium abundance in each designated deuterium atom is at least 4000 times greater than the natural abundance of deuterium (i.e., at least 60% deuterium incorporation). In some embodiments, the deuterium abundance in each designated deuterium atom is at least 4500 times greater than the natural abundance of deuterium (i.e., at least 67.5% deuterium incorporation). In some embodiments, the deuterium abundance in each designated deuterium atom is at least 5000 times greater than the natural abundance of deuterium (i.e., at least 75% deuterium incorporation). In some embodiments, the deuterium abundance in each designated deuterium atom is at least 5500 times greater than the natural abundance of deuterium (i.e., at least 82.5% deuterium incorporation). In some embodiments, the deuterium abundance in each designated deuterium atom is at least 6000 times greater than the natural abundance of deuterium (i.e., at least 90% deuterium incorporation). In some embodiments, the deuterium abundance in each designated deuterium atom is at least 6333.3 times greater than the natural abundance of deuterium (i.e., at least 95% deuterium incorporation). In some embodiments, the deuterium abundance in each designated deuterium atom is at least 6466.7 times greater than the natural abundance of deuterium (i.e., at least 97% deuterium incorporation). In some embodiments, the deuterium abundance in each designated deuterium atom is at least 6600 times greater than the natural abundance of deuterium (i.e., at least 99% deuterium incorporation). In some embodiments, the deuterium abundance in each designated deuterium atom is at least 6633.3 times greater than the natural abundance of deuterium (i.e., at least 99.5% deuterium incorporation).

The term "optionally" or "optional" means that the event or circumstance subsequently described may but does not necessarily occur, encompassing both the case where it occurs and the case where it does not occur. For example, "alkyl optionally substituted with halogen or cyano" includes both the case where the alkyl is substituted with halogen or cyano and the case where the alkyl is not substituted with halogen or cyano.

The term "substitution" or "substituted" means that one or more hydrogen atoms in a group, in some embodiments 1 to 6 hydrogen atoms, and in some embodiments 1 to 3 hydrogen atoms, are each independently substituted with a corresponding number of substituents. A person skilled in the art would have been able to determine the possible or impossible substitutions (by experiment or theory) without undue effort. For example, an amino or hydroxyl group having free hydrogen may be unstable when bonded to a carbon atom bearing an unsaturated bond (e.g., in alkene).

The term "pharmaceutical composition" means a mixture containing one or more compounds described herein or the pharmaceutically acceptable salts thereof together with other chemical components, and additional components such as pharmaceutically acceptable carriers and excipients. The purpose of the pharmaceutical compositions is to facilitate administration to living organisms and facilitate the absorption of active ingredients to exert biological activity.

The term "pharmaceutically acceptable salt" refers to a salt of the compound of the present disclosure, which can be selected from an inorganic salt or an organic salt. Such salts are safe and effective when used in mammals, and possess the expected biological activity. The salts can be prepared either during the final separation and purification of the compound, or separately by reacting suitable functional groups with appropriate bases or acids. The bases commonly used to form pharmaceutically acceptable salts include inorganic bases such as sodium hydroxide and potassium hydroxide, and organic bases such as ammonia. The acids commonly used to form pharmaceutically acceptable salts include inorganic acids and organic acids.

With respect to drugs or pharmacologically active agents, the term "therapeutically effective amount" refers to an amount of the drug or agent sufficient to achieve or at least partially achieve the desired effect. Determination of the therapeutically effective amount, varying from person to person, depends on the age and general condition of a subject and also depends on a specific active substance. The appropriate therapeutically effective amount in individual cases can be determined by a person skilled in the art according to routine experiments.

As used herein, the term "pharmaceutically acceptable" refers to those compounds, materials, compositions and/or dosage forms that are, within the scope of sound medical judgment, suitable for use in contact with patient tissues without excessive toxicity, irritation, allergic response, or other problems or complications, commensurate with a reasonable benefit/risk ratio, and effective for the intended use.

As used herein, the singular forms "a," "an," and "the" include plural referents and vice versa, unless otherwise clearly indicated in the context.

When the term "about" is applied to parameters such as pH, concentration and temperature, it indicates that the parameter can vary by ±10%, and in some embodiments, within ±5%. As will be understood by a person skilled in the art, numerical values for non-critical parameters are generally provided for illustrative purposes only and not as limitations.

### Methods for synthesising compounds of the present disclosure

In order to achieve the objectives of the present disclosure, the following technical solutions are adopted:

### Scheme 1

Provided is a method for preparing the compound represented by general formula (I) or the pharmaceutically acceptable salt thereof according to the present disclosure, wherein the method comprises: 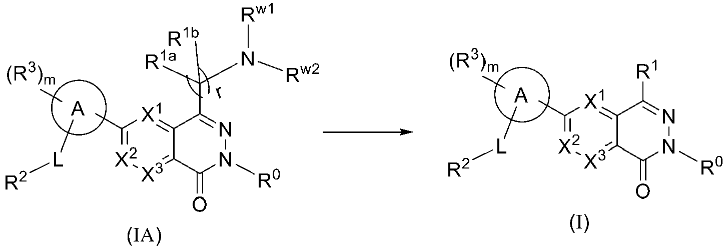 under the action of a deprotecting agent, removing an amino protecting group from a compound represented by general formula (IA) or a salt thereof to obtain the compound represented by general formula (I) or the pharmaceutically acceptable salt thereof,
wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 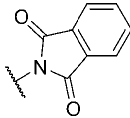 );
R¹ is-(CR^{1a}R^{1b})r-NH₂;
ring A, L, X¹, X², X³, R⁰, R^{1a}, R^{1b}, R², R³, m and r are as defined in general formula (I).

### Scheme 2

Provided is a method for preparing the compound represented by general formula (II) or the pharmaceutically acceptable salt thereof according to the present disclosure, wherein the method comprises: 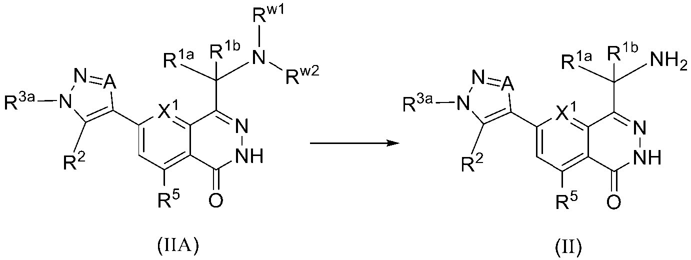 under the action of a deprotecting agent, removing an amino protecting group from a compound represented by general formula (IIA) or a salt thereof to obtain the compound represented by general formula (II) or the pharmaceutically acceptable salt thereof,
wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 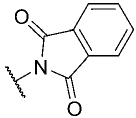 );
A, X¹, R^{1a}, R^{1b}, R², R^{3a} and R⁵ are as defined in general formula (II).

### Scheme 3

Provided is a method for preparing the compound represented by general formula (III) or the pharmaceutically acceptable salt thereof according to the present disclosure, wherein the method comprises: 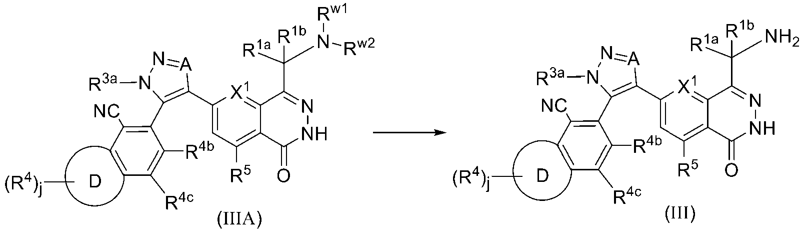 under the action of a deprotecting agent, removing an amino protecting group from a compound represented by general formula (IIIA) or a salt thereof to obtain the compound represented by general formula (III) or the pharmaceutically acceptable salt thereof,
wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 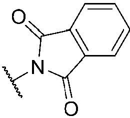 );
ring D, A, X¹, R^{1a}, R^{1b}, R^{3a}, R⁴, R^{4b}, R^{4c}, R⁵ and j are as defined in general formula (III).

### Scheme 4

Provided is a method for preparing the compound represented by general formula (III-1) or the pharmaceutically acceptable salt thereof according to the present disclosure, wherein the method comprises: 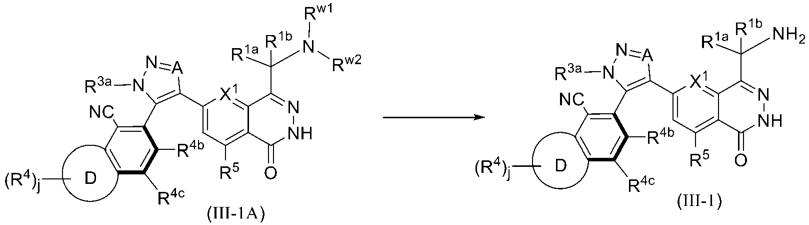 under the action of a deprotecting agent, removing an amino protecting group from a compound represented by general formula (III-1A) or a salt thereof to obtain the compound represented by general formula (III-1) or the pharmaceutically acceptable salt thereof,
wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 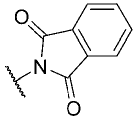 );
ring D, A, X¹, R^{1a}, R^{1b}, R^{3a}, R⁴, R^{4b}, R^{4c}, R⁵ and j are as defined in general formula (III-1).

### Scheme 5

Provided is a method for preparing the compound represented by general formula (III-2) or the pharmaceutically acceptable salt thereof according to the present disclosure, wherein the method comprises: 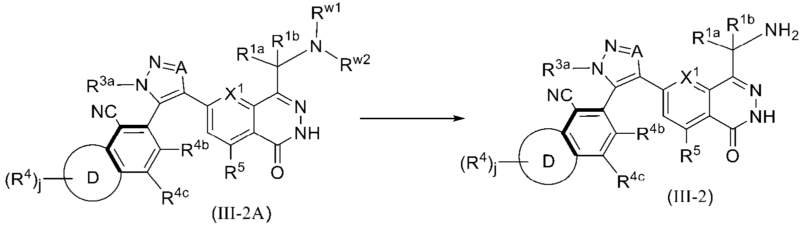 under the action of a deprotecting agent, removing an amino protecting group from a compound represented by general formula (III-2A) or a salt thereof to obtain the compound represented by general formula (III-2) or the pharmaceutically acceptable salt thereof,
wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 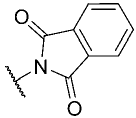 );
ring D, A, X¹, R^{1a}, R^{1b}, R^{3a}, R⁴, R^{4b}, R^{4c}, R⁵ and j are as defined in general formula (III-2).

### Scheme 6

Provided is a method for preparing the compound represented by general formula (IV) or the pharmaceutically acceptable salt thereof according to the present disclosure, wherein the method comprises: 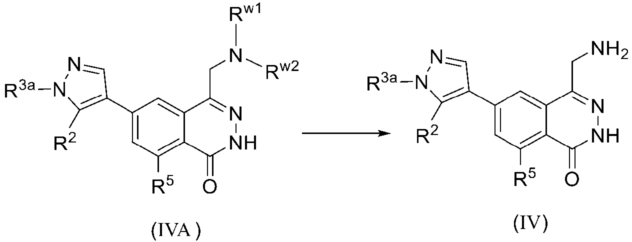 under the action of a deprotecting agent, removing an amino protecting group from a compound represented by general formula (IVA) or a salt thereof to obtain the compound represented by general formula (IV) or the pharmaceutically acceptable salt thereof,
wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido (i.e., R^{w1} and R^{w2} together with the nitrogen atom to which they are attached form 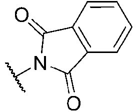 );
R², R^{3a} and R⁵ are as defined in general formula (IV).

### Scheme 7

Provided is a method for preparing the compounds represented by general formula (III-1) and general formula (III-2) or the pharmaceutically acceptable salts thereof according to the present disclosure, wherein the method comprises: 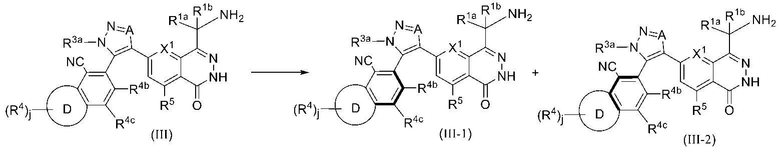 subjecting the compound represented by general formula (III) or the pharmaceutically acceptable salt thereof to a resolution, so as to obtain the compounds represented by general formula (III-1) and general formula (III-2) or the pharmaceutically acceptable salts thereof,
wherein:
ring D, A, X¹, R^{1a}, R^{1b}, R^{3a}, R⁴, R^{4b}, R^{4c}, R⁵ and j are as defined in general formula (III-1) or general formula (III-2).

In the above-described synthetic schemes 1 to 6, the amino protecting group is, in some embodiments, Boc.

In the above-described synthetic schemes 1 to 6, when R^{w1} and R^{w2} together with the nitrogen atom to which they are attached form phthalimido, the deprotection reaction for removing the amino protecting group can be carried out in the presence of a deprotecting agent such as hydrazine, hydrazine hydrate, sodium borohydride, or concentrated HCl, with hydrazine hydrate being employed in some embodiments.

In some embodiments, the above-described reactions are carried out in solvents, including but not limited to: N-methylpyrrolidone, ethylene glycol dimethyl ether, acetic acid, methanol, ethanol, acetonitrile, n-butanol, toluene, tetrahydrofuran, dichloromethane, petroleum ether, ethyl acetate, n-hexane, dimethyl sulphoxide, 1,4-dioxane, water, N,N-dimethylformamide, N,N-dimethylacetamide, 1,2-dibromoethane and mixtures thereof.

### Brief Description of the Drawings

FIG. 1 shows the efficacy data of compound 2-p1 against Lu99 xenografts in NUNU nude mice.
FIG. 2 shows the effect of compound 2-p1 on the body weight of NUNU nude mice.

### Detailed Description of Embodiments

The present disclosure will be further described below in conjunction with examples, but these examples are not intended to limit the scope of the present disclosure.

### Example

The structures of the compounds are determined by nuclear magnetic resonance (NMR) or /and mass spectrometry (MS). The NMR shift (δ) is given in the unit of 10⁻⁶ (ppm). NMR determination is performed by Bruker AVANCE NEO 500M nuclear magnetic resonance spectrometer; the solvents for determination are deuterated dimethyl sulphoxide (DMSO-*d₆*), deuterated chloroform (CDCl₃) and deuterated methanol (CD₃OD); and the internal standard is tetramethylsilane (TMS).

MS determination is performed by Agilent 1200 /1290 DAD- 6110/6120 Quadrupole MS liquid chromatography-mass spectrometer (manufacturer: Agilent, MS model: 6110/6120 Quadrupole MS); waters ACQuity UPLC-QD/SQD (manufacturer: waters, MS model: waters ACQuity Qda Detector/waters SQ Detector); and THERMO Ultimate 3000-Q Exactive (manufacturer: THERMO, MS model: THERMO Q Exactive).

High-performance liquid chromatography (HPLC) analysis is carried out by Agilent HPLC 1200DAD, Agilent HPLC 1200VWD and Waters HPLC e2695-2489 high-performance liquid chromatographs.

Chiral HPLC analysis is determined by Agilent 1260 DAD high-performance liquid chromatograph.

Preparative high-performance liquid chromatography is carried out by Waters 2545-2767, Waters 2767-SQ Detecor2, Shimadzu LC-20AP and Gilson GX-281 preparative chromatographs.

Chiral preparative chromatography is carried out by Shimadzu LC-20AP preparative chromatograph.

The CombiFlash flash preparative instrument used is Combiflash Rf200 (TELEDYNE ISCO).

The silica gel plate for thin layer chromatography is Yantai Huanghai HSGF254 or Qingdao GF254 silica gel plate, the specification of the silica gel plate used for thin layer chromatography (TLC) is 0.15 mm to 0.2 mm, and the specification of the silica gel plate used for separating and purifying products by thin layer chromatography is 0.4 mm to 0.5 mm.

For silica gel column chromatography, Yantai Huanghai silica gel 200-300 mesh silica gel is generally used as a carrier.

The determination of the average kinase inhibition rate and IC₅₀ value is performed using NovoStar microplate reader (BMG Company, Germany).

Known starting materials of the present disclosure may be synthesised using or according to methods known in the art, or may be purchased from ABCR GmbH & Co. KG, Acros Organics, Aldrich Chemical Company, Accela ChemBio Inc., Chembee Chemicals, and other companies.

Unless otherwise specified, the reactions in the examples can be carried out under argon atmosphere or nitrogen atmosphere.

The argon atmosphere or nitrogen atmosphere means that the reaction bottle is connected to an argon or nitrogen balloon with a volume of about 1 L.

The hydrogen atmosphere means that the reaction bottle is connected to a hydrogen balloon with a volume of about 1 L.

Parr 3916EKX hydrogenator, Qinglan QL-500 hydrogen generator or HC2-SS hydrogenator are used for the pressurised hydrogenation reaction.

The hydrogenation reaction usually comprises the steps of vacuumising and charging with hydrogen, and the operation is repeated for 3 times.

CEM Discover-S 908860 microwave reactor is used for the microwave reaction.

Unless otherwise specified in the examples, a solution refers to an aqueous solution.

Unless otherwise specified in the examples, the reaction temperature refers to room temperature, which is 20°C to 30°C.

The progress of the reaction in the examples is monitored by means of thin layer chromatography (TLC). The developing solvent used in the reaction, the eluent system of column chromatography used in the purification of compounds and the developing solvent system of thin layer chromatography include: A: dichloromethane/methanol, B: n-hexane/ethyl acetate, wherein the volume ratio of the solvents is adjusted according to the polarity of compounds, and a small quantity of basic reagents (e.g., triethylamine) or acidic reagents (e.g., acetic acid) can be added for further adjustment.

### Example 1

5-(4-(4-(aminomethyl)-1-oxo-1,2-dihydrophthalazin-6-yl)-1-methyl-1*H*-pyrazol-5-yl)-6-fluoro-1,2,2*a*,8*a*-tetrahydrobenzo[*b*]cyclobuta[*e*][1,4]dioxine-4-carbonitrile **1** (*M*)-(2*aS*,8*aR*)-5-(4-(4-(aminomethyl)-1-oxo-1,2-dihydrophthalazin-6-yl)-1-methyl-1*H*-pyrazol-5-yl)-6-fluoro-1,2,2*a*,8*a-*tetrahydrobenzo[*b*]cyclobuta[e][1,4]dioxine-4-carbonitrile **1-p1** (*M*)-(2*a*R,8*aS*)-5-(4-(4-(aminomethyl)-1-oxo-1,2-dihydrophthalazin-6-yl)-1-methyl-1*H*-pyrazol-5-yl)-6-fluoro-1,2,2*a*,8*a-*tetrahydrobenzo[*b*]cyclobuta[*e*][1,4]dioxine-4-carbonitrile **1-p2** (*P*)-(2*aS*,8*aR*)-5-(4-(4-(aminomethyl)-1-oxo-1,2-dihydrophthalazin-6-yl)-1-methyl-1*H*-pyrazol-5-yl)-6-fluoro-1,2,2*a*,8*a-*tetrahydrobenzo[*b*]cyclobuta[e][1,4]dioxine-4-carbonitrile **1-p3** (*P*)-(2*aR*,8*aS*)-5-(4-(4-(aminomethyl)-1-oxo-1,2-dihydrophthalazin-6-yl)-1-methyl-1*H*-pyrazol-5-yl)-6-fluoro-1,2,2*a*,8*a-*tetrahydrobenzo[b]cyclobuta[*e*][1,4]dioxine-4-carbonitrile **1-p4**

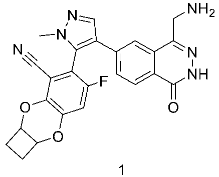

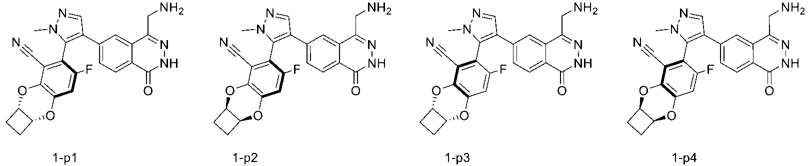

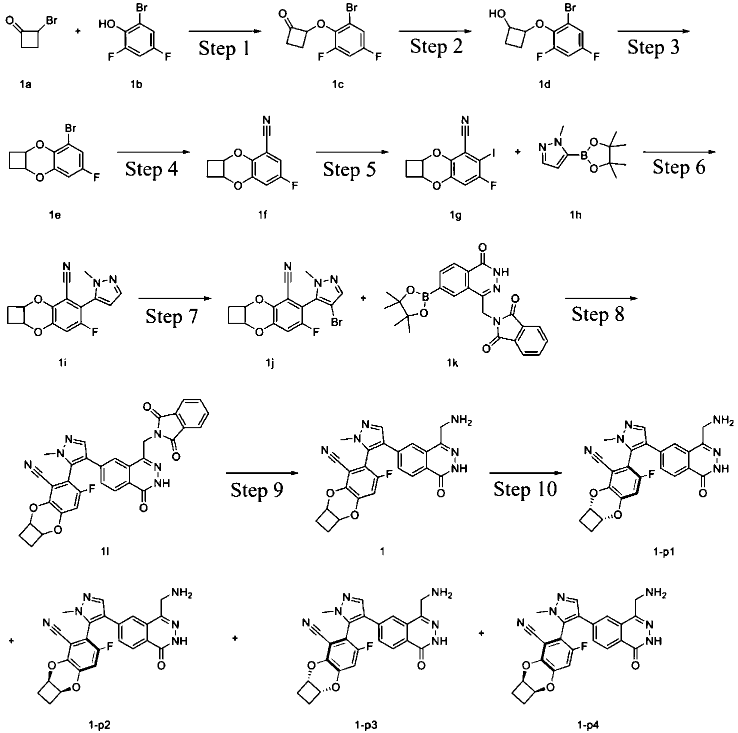

### Step 1

### 2-(2-bromo-4,6-difluorophenoxy)cyclobutan-1-one 1c

2-bromo-4,6-difluorophenol **1b** (473 g, 2.04 mol, Jiangsu Aikon) was dissolved in *N*,*N*-dimethylformamide (3000 mL). Sodium carbonate (997.14 g, 9.41 mol) and 2-bromocyclobutan-1-one **1a** (410.08 g, 2.34 mol, prepared according to the method disclosed in patent application "WO 2018013770", description, page 53, for intermediate I-07A) were added. The mixture was stirred at 80°C for 2 hours. The reaction solution was cooled to room temperature and filtered. The filter cake was washed with 4000 mL of ethyl acetate, and the filtrate was collected. 4000 mL of water was added. The organic phase was separated, and the aqueous phase was extracted once with 500 mL of ethyl acetate. The organic phases were combined, washed with a saturated sodium chloride solution, dried over anhydrous sodium sulphate, filtered to remove the drying agent and then concentrated under reduced pressure. The residue was purified by silica gel column chromatography with eluent system B to obtain the title compound **1c** (260 g, yield: 46%).

MS m/z (ESI): 276.9 [M+1].

### Step 2

### 2-(2-bromo-4,6-difluorophenoxy)cyclobutan-1-ol 1d

Compound **1c** (240 g, 822.92 mmol) was dissolved in tetrahydrofuran (1500 mL), and the mixture was cooled to-70°C. A solution of tri-sec-butylborohydride in tetrahydrofuran (905 mL, 1 M) was added dropwise, and the mixture was stirred for 1 hour while maintaining the temperature. The reaction was quenched by dropwise adding 500 mL of water, and then 500 mL of 1% sodium hydroxide solution was added. The mixture was extracted with ethyl acetate (500 mL×2). The organic phases were combined, dried over anhydrous sodium sulphate, filtered to remove the drying agent and then concentrated under reduced pressure to obtain the crude title compound **1d** (272 g), which was used directly in the next reaction without purification.

MS m/z (ESI): 278.9 [M+1].

### Step 3

### 4-bromo-6-fluoro-1,2,2a,8a-tetrahydrobenzo[b]cyclobuta[e][1,4]dioxine 1e

Compound **1d** (272 g, 779.71 mmol) was dissolved in dimethyl sulphoxide (2700 mL), and caesium carbonate (762.13 g, 2.34 mol) was added. The mixture was warmed to 120°C and stirred for 1 hour. The reaction solution was cooled to room temperature, and water was added. The mixture was extracted with ethyl acetate (500 mL×2). The organic phases were combined, washed sequentially with water and a saturated sodium chloride solution, dried over anhydrous sodium sulphate, filtered to remove the drying agent and then concentrated under reduced pressure. The residue was purified by silica gel column chromatography with eluent system B to obtain the title compound **1e** (201 g, yield: 69.7%).

MS m/z (ESI): 258.9 [M+1].

### Step 4

### 6-fluoro-1,2,2a,8a-tetrahydrobenzo[b]cyclobuta[e][1,4]dioxine-4-carbonitrile 1f

Compound **1e** (5.45 g, 21 mmol) was dissolved in *N,N-*dimethylacetamide (50 mL), and zinc cyanide (4.94 g, 42 mmol), zinc powder (1.44 g, 22.1 mmol) and tetrakis(triphenylphosphine)palladium (1.2 g, 1.05 mmol) were added. The mixture was purged with nitrogen, heated to 100°C and reacted for 16 hours. The reaction solution was cooled to room temperature, diluted in ethyl acetate and then filtered. The filtrate was washed sequentially with water and a saturated sodium chloride solution, dried over anhydrous sodium sulphate, filtered to remove the drying agent and then concentrated under reduced pressure. The residue was purified by silica gel column chromatography with eluent system B to obtain the title compound **1f** (3.52 g, yield: 81.5%).

MS m/z (ESI): 206.2 [M+1].

### Step 5

### 6-fluoro-5-iodo-1,2,2a,8a-tetrahydrobenzo[b]cyclobuta[e][1,4]dioxine-4-carbonitrile 1g

Compound **1f** (2.32 g, 11.3 mmol) was dissolved in tetrahydrofuran (80 mL), and the mixture was cooled to -78°C. A solution of diisopropylaminolithium in tetrahydrofuran (6.3 mL, 2 M) was added dropwise, and the mixture was stirred for 30 minutes. Then, a solution of iodine (3.0 g, 11.8 mmol) in tetrahydrofuran (10 mL) was added, and the reaction was stirred for 30 minutes. The reaction was quenched by adding a saturated sodium sulphite solution and an aqueous sodium bicarbonate solution to the reaction solution. Liquid separation was performed. The aqueous phase was extracted with ethyl acetate (50 mL×3). The organic phases were combined, dried over anhydrous sodium sulphate, filtered to remove the drying agent and then concentrated under reduced pressure. The residue was purified by silica gel column chromatography with eluent system B to obtain the title compound **1g** (1.5 g, yield: 40%).

### Step 6

### 6-fluoro-5-(1-methyl-1H-pyrazol-5-yl)-1,2,2a,8a-tetrahydrobenzo[b]cyclobuta[e][1,4]dioxine-4-carbonitrile 1i

1-Methyl-1*H*-pyrazole-5-boronic acid pinacol ester **1h** (1.78 g, 8.5 mmol, Pharmablock Sciences (Nanjing), Inc.) and compound **1g** (1.42 g, 4.28 mmol) were dissolved in water (6 mL) and 1,4-dioxane (30 mL), and then sodium carbonate (909 mg, 8.6 mmol) and 1,1'-bis(di-tert-butylphosphino)ferrocene palladium dichloride (292 mg, 428.29 µmol) were added. The mixture was purged with nitrogen, heated to 85°C and reacted for 16 hours. The reaction solution was concentrated under reduced pressure. The residue was dissolved in dichloromethane and then filtered, and the filtrate was concentrated under reduced pressure. The residue was purified by silica gel column chromatography with eluent system B to obtain the title compound **1i** (1.22 g, yield: 99%).

MS m/z (ESI): 286.0 [M+1].

### Step 7

### 5-(4-bromo-1-methyl-1H-pyrazol-5-yl)-6-fluoro-1,2,2a,8a-tetrahydrobenzo[b]cyclobuta[e][1,4]dioxine-4-carbonitrile 1j

Compound **1i** (1.22 g, 4.3 mmol) was dissolved in acetonitrile (20 mL), and *N*-bromosuccinimide (837 mg, 4.7 mmol) was added. The reaction was stirred for 2 hours. The reaction solution was concentrated under reduced pressure. The residue was purified by silica gel column chromatography with eluent system B to obtain the title compound **1j** (1.55 g, yield: 99%).

MS m/z (ESI): 364.0 [M+1].

### Step 8

### 5-(4-(4-((1,3-dioxoisoindolin-2-yl)methyl)-1-oxo-1,2-dihydrophthalazin-6-yl)-1-methyl-1H-pyrazol-5-yl)-6-fluoro-1,2,2a,8a-tetrahydrobenzo[b]cyclobuta[e][1,4]dioxine-4-carbonitrile 11

Under nitrogen atmosphere, compound **1j** (1.27 g, 2.94 mmol), 2-((4-oxo-7-(4,4,5,5-tetramethyl-1,3,2-dioxaborolan-2-yl)-3,4-dihydrophthalazin-1-yl)methyl)isoindoline-1,3-dione **1k** (1.27 g, 2.94 mmol, prepared according to the method disclosed in "Journal of Medicinal Chemistry, 2022, vol. 65, # 3, p. 1749 - 1766"), 1,1'-bis(di-tert-butylphosphino)ferrocene palladium dichloride (1.95 g, 2.94 mmol), and sodium bicarbonate (495 mg, 5.89 mmol) were dissolved in 1,4-dioxane (25 mL) and water (5 mL). The reaction was stirred at 80°C for 4 hours. The reaction solution was concentrated under reduced pressure. The residue was dissolved in dichloromethane and then filtered, and the filtrate was concentrated under reduced pressure. The residue was purified by silica gel column chromatography with eluent system A to obtain the title compound **11** (973 mg, yield: 56.1%).

MS m/z (ESI): 589.2 [M+1].

### Step 9 to step 10

### 5-(4-(4-(aminomethyl)-1-oxo-1,2-dihydrophthalazin-6-yl)-1-methyl-1H-pyrazol-5-yl)-6-fluoro-1,2,2a,8a-tetrahydrobenzo[b]cyclobuta[e][1,4]dioxine-4-carbonitrile 1 (M)-(2aS,8aR)-5-(4-(4-(aminomethyl)-1-oxo-1,2-dihydrophthalazin-6-yl)-1-methyl-1H-pyrazol-5-yl)-6-fluoro-1,2,2a,8a-tetrahydrobenzo[b]cyclobuta[e][1,4]dioxine-4-carbonitrile 1-pl (M)-(2aR,8aS)-5-(4-(4-(aminomethyl)-1-oxo-1,2-dihydrophthalazin-6-yl)-1-methyl-1H-pyrazol-5-yl)-6-fluoro-1,2,2a,8a-tetrahydrobenzo[b]cyclobuta[e][1,4]dioxine-4-carbonitrile 1-p2 (P)-(2aS,8aR)-5-(4-(4-(aminomethyl)-1-oxo-1,2-dihydrophthalazin-6-yl)-1-methyl-1H-pyrazol-5-yl)-6-fluoro-1,2,2a,8a-tetrahydrobenzo[b]cyclobuta[e][1,4]dioxine-4-carbonitrile 1-p3 (P)-(2aR,8aS)-5-(4-(4-(aminomethyl)-1-oxo-1,2-dihydrophthalazin-6-yl)-1-methyl-1H-pyrazol-5-yl)-6-fluoro-1,2,2a,8a-tetrahydrobenzo[b]cyclobuta[e][1,4]dioxine-4-carbonitrile 1-p4

Compound **11** (973 mg, 1.65 mmol) was dissolved in ethanol (15 mL), and hydrazine hydrate (780 mg, 13.2 mmol, 85% purity) was added. The reaction was stirred at 80°C for 2 hours. The reaction solution was concentrated under reduced pressure. Dichloromethane/methanol (V : V = 5 : 1) was added to the residue, and the mixture was stirred for 10 minutes and then filtered. The filtrate was concentrated under reduced pressure to obtain the crude compound **1** (760 mg). The compound 1 was purified by preparative high-performance liquid chromatography (Waters-2545, chromatographic column: YMC Triart-Exrs, Prep 30*150 mm, 5 µm; mobile phase: aqueous phase (10 mmol/L ammonium bicarbonate) and acetonitrile, gradient: acetonitrile 30%-42%, flow rate: 30 mL/min) to obtain two fractions: fraction A (180 mg, yield: 24.5%) and fraction B (180 mg, yield: 24.5%).

### Fraction A: short retention time

MS m/z (ESI): 459.1 [M+1].

HPLC analysis: retention time: 1.01 minutes, purity: 99% (chromatographic column: ACQUITY UPLC^{®}BEH, C18, 1.7 µm, 2.1*50 mm; mobile phase: water (10 mM ammonium bicarbonate) and acetonitrile, gradient: acetonitrile 10%-95%).

¹H NMR (500 MHz, DMSO-*d*₆): δ 12.44 (s, 1H), 8.26 (s, 1H), 8.18 (d, 1H), 7.75 (dd, 1H), 7.70 (s, 1H), 7.59 (d, 1H), 4.96-4.92 (m, 2H), 3.76 (d, 2H), 3.75 (s, 3H), 2.24-2.15 (m, 6H).

### Fraction B: long retention time

MS m/z (ESI): 459.1 [M+1].

HPLC analysis: retention time: 1.04 minutes, purity: 96% (chromatographic column: ACQUITY UPLC^{®}BEH, C18, 1.7 µm, 2.1*50 mm; mobile phase: water (10 mM ammonium bicarbonate) and acetonitrile, gradient: acetonitrile 10%-95%).

¹H NMR (500 MHz, DMSO-*d*₆): δ 12.43 (s, 1H), 8.24 (s, 1H), 8.15 (d, 1H), 7.74 (s, 1H), 7.67 (dd, 1H), 7.59 (d, 1H), 4.96-4.90 (m, 2H), 3.79-3.78 (m, 5H), 2.25-2.13 (m, 4H), 1.95 (s, 2H).

Fraction A (100 mg) was resolved by a chiral column (Shimadzu LC-20AP, chromatographic column: Daicel CHIRALPAK IE, 20*250 mm, 10 µm; mobile phase A: n-hexane, mobile phase B: ethanol (0.5% ammonia-methanol solution), gradient: A:B is 55:45, flow rate: 20 mL/min) to obtain the title compounds **1-p4** (30 mg, 30%) and **1-pl** (32 mg, 32%).

The single-configuration compound **1-p4** (with shorter retention time in fraction A): (30 mg, 30%).

Chiral HPLC analysis: retention time: 13.511 minutes, purity: 99% (chromatographic column: CHIRALPAK IE 150*4.6 mm, 5 µm; mobile phase: n-hexane and ethanol (containing 0.1% diethylamine), gradient: A:B is 50:50, flow rate: 1.0 mL/min).

¹H NMR (500 MHz, DMSO-*d*₆): δ 12.45 (s, 1H), 8.26 (s, 1H), 8.18 (d, 1H), 7.75 (dd, 1H), 7.71 (s, 1H), 7.59 (d, 1H), 4.97-4.91 (m, 2H), 3.78 (d, 2H), 3.75 (s, 3H), 2.25-2.15 (m, 6H).

The single-configuration compound **1-pl** (with longer retention time in fraction A): (32 mg, 32%).

Chiral HPLC analysis: retention time: 17.880 minutes, purity: 98% (chromatographic column: CHIRALPAK IE 150*4.6 mm, 5 µm; mobile phase: n-hexane and ethanol (containing 0.1% diethylamine), gradient: A:B is 50:50, flow rate: 1.0 mL/min).

¹H NMR (500 MHz, DMSO-*d*₆): δ 12.45 (s, 1H), 8.26 (s, 1H), 8.18 (d, 1H), 7.75 (dd, 1H), 7.71 (s, 1H), 7.59 (d, 1H), 4.97-4.91 (m, 2H), 3.78 (d, 2H), 3.75 (s, 3H), 2.25-2.15 (m, 6H).

Fraction B (100 mg) was resolved by a chiral column (Shimadzu LC-20AP, chromatographic column: Daicel CHIRALPAK IE, 20*250 mm, 10 µm; mobile phase A: n-hexane, mobile phase B: ethanol (0.5% ammonia-methanol solution), gradient: A : B is 40 : 60, flow rate: 20 mL/min) to obtain the title compounds **1-p3** (28 mg, 28%) and **1-p2** (30 mg, 30%).

The single-configuration compound **1-p3** (with shorter retention time in fraction B): (28 mg, 28%).

Chiral HPLC analysis: retention time: 13.120 minutes, purity: 99% (chromatographic column: CHIRALPAK IE 150*4.6 mm, 5 µm; mobile phase: n-hexane and ethanol (containing 0.1% diethylamine), gradient: A : B is 50 : 50, flow rate: 1.0 mL/min).

¹H NMR (500 MHz, DMSO-*d*₆): δ 12.44 (s, 1H), 8.24 (s, 1H), 8.15 (d, 1H), 7.74 (s, 1H), 7.67 (dd, 1H), 7.58 (d, 1H), 4.96-4.90 (m, 2H), 3.80-3.77 (m, 5H), 2.25-2.13 (m, 4H).

The single-configuration compound **1-p2** (with longer retention time in fraction B): (30 mg, 30%).

Chiral HPLC analysis: retention time: 23.466 minutes, purity: 99% (chromatographic column: CHIRALPAK IE 150*4.6 mm, 5 µm; mobile phase: n-hexane and ethanol (containing 0.1% diethylamine), gradient: A:B is 50:50, flow rate: 1.0 mL/min).

¹H NMR (500 MHz, DMSO-*d*₆): δ 12.44 (s, 1H), 8.24 (s, 1H), 8.15 (d, 1H), 7.74 (s, 1H), 7.67 (dd, 1H), 7.58 (d, 1H), 4.96-4.90 (m, 2H), 3.80-3.77 (m, 5H), 2.25-2.13 (m, 4H).

### Example 2

5-(4-(4-(aminomethyl)-1-oxo-1,2-dihydrophthalazin-6-yl)-1-(methyl-*d*₃)-1*H-*pyrazol-5-yl)-6-fluoro-1,2,2a,8a-tetrahydrobenzo[*b*]cyclobuta[*e*][1,4]dioxine-4-carbonitrile **2** (*M*)-(2*aS*,8*a*R)-5-(4-(4-(aminomethyl)-1-oxo-1,2-dihydrophthalazin-6-yl)-1-(methyl-*d₃*)-1*H*-pyrazol-5-yl)-6-fluoro-1,2,2*a*,8*a-*tetrahydrobenzo[*b*]cyclobuta[*e*][1,4]dioxine-4-carbonitrile **2-p1** (*M*)-(2*aR*,8*aS*)-5-(4-(4-(aminomethyl)-1-oxo-1,2-dihydrophthalazin-6-yl)-1-(methyl-*d₃*)-1*H*-pyrazol-5-yl)-6-fluoro-1,2,2*a*,8*a-*tetrahydrobenzo[*b*]cyclobuta[*e*][1,4]dioxine-4-carbonitrile **2-p2** (*P*)-(2*aS,8a*R)-5-(4-(4-(aminomethyl)-1-oxo-1,2-dihydrophthalazin-6-yl)-1-(methyl-*d₃*)-1*H*-pyrazol-5-yl)-6-fluoro-1,2,2*a*,8*a-*tetrahydrobenzo[*b*]cyclobuta[*e*][1,4]dioxine-4-carbonitrile **2-p3** (*P*)-(2*aR*,8*aS*)-5-(4-(4-(aminomethyl)-1-oxo-1,2-dihydrophthalazin-6-yl)-1-(methyl-*d₃*)-1*H*-pyrazol-5-yl)-6-fluoro-1,2,2*a*,8*a-*tetrahydrobenzo[*b*]cyclobuta[*e*][1,4]dioxine-4-carbonitrile **2-p4**

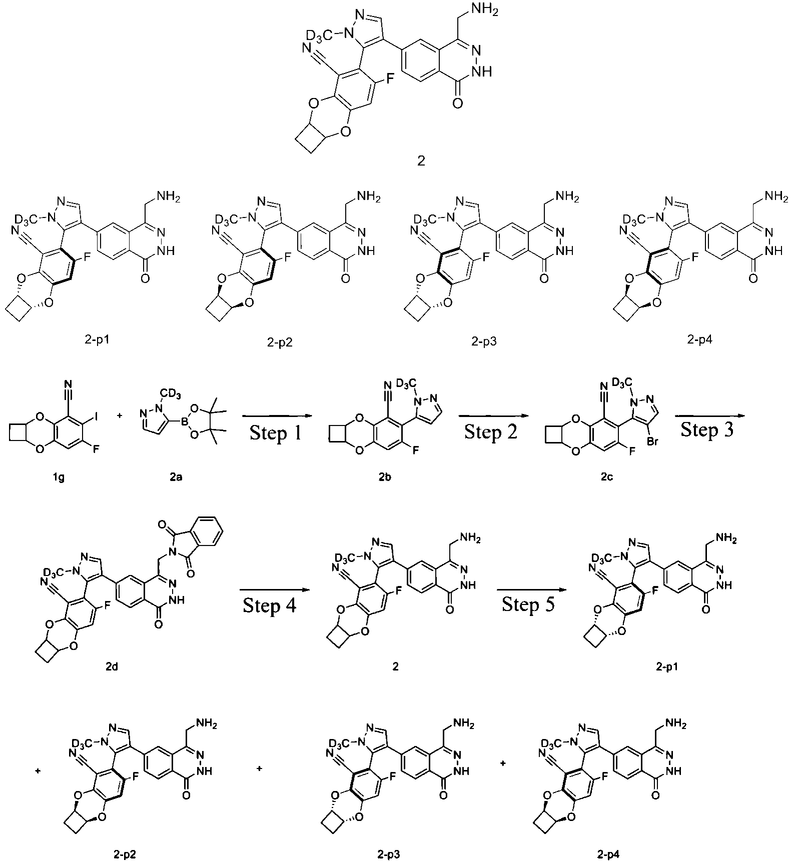

### Step 1

### 6-fluoro-5-(1-(methyl-d₃)-1H-pyrazol-5-yl)-1,2,2a,8a-tetrahydrobenzo[b]cyclobuta[e][1,4]dioxine-4-carbonitrile 2b

1-(methyl-*d*₃)-5-(4,4,5,5-tetramethyl-1,3,2-dioxaborolan-2-yl)-1*H*-pyrazole **2a** (3 g, 14.2 mmol, prepared according to the method disclosed in patent application "WO 2019079469", description, page 178, example 25) and compound **1g** (2.4 g, 7.25 mmol) were dissolved in water (8 mL) and 1,4-dioxane (40 mL), and then sodium carbonate (1.6 g, 15.1 mmol) and 1,1'-bis(di-tert-butylphosphino)ferrocene palladium dichloride (470 mg, 718.9 µmol) were added. The mixture was purged with nitrogen, heated to 85°C and reacted for 16 hours. The reaction solution was concentrated under reduced pressure. The residue was dissolved in dichloromethane and then filtered. The filtrate was concentrated under reduced pressure. The residue was purified by silica gel column chromatography with eluent system B to obtain the title compound **2b** (2 g, yield: 99%).

MS m/z (ESI): 289.0 [M+1].

### Step 2

### 5-(4-bromo-1-(methyl-d₃)-1H-pyrazol-5-yl)-6-fluoro-1,2,2a,8a-tetrahydrobenzo[b]cyclobuta[e][1,4]dioxine-4-carbonitrile 2c

Compound **2b** (1.9 g, 6.6 mmol) was dissolved in acetonitrile (40 mL), and *N-*bromosuccinimide (1.3 g, 7.25 mmol) was added. The reaction was stirred for 2 hours. The reaction solution was concentrated under reduced pressure. The residue was purified by silica gel column chromatography with eluent system B to obtain the title compound **2c** (2.1 g, yield: 86.7%).

MS m/z (ESI): 367.2 [M+1].

### Step 3

### 5-(4-(4-((1,3-dioxoisoindolin-2-yl)methyl)-1-oxo-1,2-dihydrophthalazin-6-yl)-1-(methyl-d₃)-1H-pyrazol-5-yl)-6-fluoro-1,2,2a,8a-tetrahydrobenzo[b]cyclobuta[e][1,4]dioxine-4-carbonitrile 2d

Under nitrogen atmosphere, compound **2c** (1.5 g, 4.08 mmol), compound **1k** (1.8 g, 4.17 mmol), 1,1'-bis(di-tert-butylphosphino)ferrocene palladium dichloride (2.7 g, 4 mmol) and sodium bicarbonate (686 mg, 8.17 mmol) were dissolved in 1,4-dioxane (20 mL) and water (4 mL). The reaction was stirred at 80°C for 4 hours. The reaction solution was concentrated under reduced pressure. The residue was dissolved in dichloromethane and then filtered. The filtrate was concentrated under reduced pressure. The residue was purified by silica gel column chromatography with eluent system A to obtain the title compound **2d** (760 mg, yield: 31.4%).

MS m/z (ESI): 592.2 [M+1].

### Step 4 and step 5

### 5-(4-(4-(aminomethyl)-1-oxo-1,2-dihydrophthalazin-6-yl)-1-(methyl-d₃)-1H-pyrazol-5-yl)-6-fluoro-1,2,2a,8a-tetrahydrobenzo[b]cyclobuta[e][1,4]dioxine-4-carbonitrile 2 (M)-(2aS,8aR)-5-(4-(4-(aminomethyl)-1-oxo-1,2-dihydrophthalazin-6-yl)-1-(methyl-d₃)-1H-pyrazol-5-yl)-6-fluoro-1,2,2a,8a-tetrahydrobenzo[b]cyclobuta[e][1,4]dioxine-4-carbonitrile 2-p1 (M)-(2aR,8aS)-5-(4-(4-(aminomethyl)-1-oxo-1,2-dihydrophthalazin-6-yl)-1-(methyl-d₃)-1H-pyrazol-5-yl)-6-fluoro-1,2,2a,8a-tetrahydrobenzo[b]cyclobuta[e][1,4]dioxine-4-carbonitrile 2-p2 (P)-(2aS,8aR)-5-(4-(4-(aminomethyl)-1-oxo-1,2-dihydrophthalazin-6-yl)-1-(methyl-d₃)-1H-pyrazol-5-yl)-6-fluoro-1,2,2a,8a-tetrahydrobenzo[b]cyclobuta[e][1,4]dioxine-4-carbonitrile 2-p3 (P)-(2aR,8aS)-5-(4-(4-(aminomethyl)-1-oxo-1,2-dihydrophthalazin-6-yl)-1-(methyl-d₃)-1H-pyrazol-5-yl)-6-fluoro-1,2,2a,8a-tetrahydrobenzo[b]cyclobuta[e][1,4]dioxine-4-carbonitrile 2-p4

Compound **2d** (760 mg, 1.28 mmol) was dissolved in ethanol (15 mL), and hydrazine hydrate (605 mg, 10.2 mmol, 85% purity) was added. The reaction was stirred at 80°C for 2 hours. The reaction solution was concentrated under reduced pressure. Dichloromethane/methanol (V : V = 5 : 1) was added to the residue, and the mixture was stirred for 10 minutes and then filtered. The filtrate was concentrated under reduced pressure to obtain the crude compound **2** (590 mg). The compound 2 was purified by preparative high-performance liquid chromatography (Waters-2545, chromatographic column: YMC Triart-Exrs, Prep 30*150 mm, 5 µm; mobile phase: aqueous phase (10 mmol/L ammonium bicarbonate) and acetonitrile, gradient: acetonitrile 30%-42%, flow rate: 30 mL/min) to obtain two fractions: fraction A (with shorter retention time, 130 mg, yield: 22%) and fraction B (with longer retention time, 130 mg, yield: 22%).

Fraction A (130 mg) was resolved by a chiral column (Gilson-281, chromatographic column: CHIRALPAK IE, 20*250 mm, 5 µm; mobile phase A: n-hexane, mobile phase B: ethanol (0.5% 7 M ammonia-methanol solution), gradient: A : B is 50 : 50, flow rate: 15 mL/min) to obtain the title compounds **2-p4** (30 mg, 23%) and **2-pl** (30 mg, 23%).

The single-configuration compound **2-p4** (with shorter retention time in fraction A) (30 mg, 23%).

Chiral HPLC analysis: retention time: 13.617 minutes, purity: 99% (chromatographic column: CHIRALPAK IG 150*4.6 mm, 5 µm; mobile phase: n-hexane and ethanol (containing 0.1% diethylamine), gradient: A:B is 50:50, flow rate: 1.0 mL/min).

MS m/z (ESI): 462.1 [M+1].

¹H NMR (500 MHz, CD₃OD): δ 8.30 (d, 1H), 8.15 (s, 1H), 7.77 (d, 1H), 7.70 (dd, 1H), 7.33 (d, 1H), 4.11-4.00 (m, 2H), 3.34-3.32 (m, 2H), 2.37-2.17 (m, 4H).

The single-configuration compound **2-p1** (with longer retention time in fraction A): (30 mg, 23%).

Chiral HPLC analysis: retention time: 18.128 minutes, purity: 98% (chromatographic column: CHIRALPAK IE 150*4.6 mm, 5 µm; mobile phase: n-hexane and ethanol (containing 0.1% diethylamine), gradient: A:B is 50:50, flow rate: 1.0 mL/min).

MS m/z (ESI): 462.1 [M+1].

¹H NMR (500 MHz, DMSO-*d*₆): δ 12.44 (s, 1H), 8.25 (s, 1H), 8.19-8.17 (m, 1H), 7.76-7.74 (m, 1H), 7.71-7.70 (m, 1H), 7.60-7.58 (m, 1H), 4.97-4.90 (m, 2H), 3.80-3.73 (m, 2H), 2.26-2.13 (m, 4H), 1.91 (brs, 2H).

Fraction B (130 mg) was resolved by a chiral column (Gilson-281, chromatographic column: CHIRALPAK IE, 20*250 mm, 5 µm; mobile phase A: n-hexane, mobile phase B: ethanol (0.5% 7 M ammonia-methanol solution), gradient: A : B is 50 : 50, flow rate: 15 mL/min) to obtain the title compounds **2-p3** (30 mg, 23%) and **2-p2** (47 mg, 36.1%).

The single-configuration compound **2-p3** (with shorter retention time in fraction B): (30 mg, 23%).

Chiral HPLC analysis: retention time: 13.073 minutes, purity: 99% (chromatographic column: CHIRALPAK IE 150*4.6 mm, 5 µm; mobile phase: n-hexane and ethanol (containing 0.1% diethylamine), gradient: A:B is 50:50, flow rate: 1.0 mL/min).

MS m/z (ESI): 462.1 [M+1].

¹H NMR (500 MHz, DMSO-*d*₆): δ 12.42 (s, 1H), 8.24 (s, 1H), 8.15-8.14 (m, 1H), 7.74-7.73 (m, 1H), 7.68-7.66 (m, 1H), 7.59-7.57 (m, 1H), 4.96-4.89 (m, 2H), 3.81-3.74 (m, 2H), 2.26-2.11 (m, 4H), 1.82 (brs, 2H).

The single-configuration compound **2-p2** (with longer retention time in fraction B): (47 mg, 36.1%).

Chiral HPLC analysis: retention time: 23.504 minutes, purity: 99% (chromatographic column: CHIRALPAK IE 150*4.6 mm, 5 µm; mobile phase: n-hexane and ethanol (containing 0.1% diethylamine), gradient: A:B is 50:50, flow rate: 1.0 mL/min).

MS m/z (ESI): 462.1 [M+1].

¹H NMR (500 MHz, CD₃OD): δ 8.27 (d, 1H), 8.15 (s, 1H), 7.73 (d, 1H), 7.68 (dd, 1H), 7.33 (d, 1H), 4.07-3.97 (m, 2H), 3.38-3.34 (m, 2H), 2.35-2.21 (m, 4H).

### Example 3

7-(4-(4-(aminomethyl)-1-oxo-1,2-dihydrophthalazin-6-yl)-1-(methyl-*d₃*)-1*H-*pyrazol-5-yl)-6-fluoro-3*H*-spiro[benzo[*b*][1,4]dioxine-2,1'-cyclopropane]-8-carbonitrile **3**

(*M*)-7-(4-(4-(aminomethyl)-1-oxo-1,2-dihydrophthalazin-6-yl)-1-(methyl-*d₃*)-1*H-*pyrazol-5-yl)-6-fluoro-3*H*-spiro[benzo[*b*][1,4]dioxine-2,1'-cyclopropane]-8-carbonitrile **3-p1**

(*P*)-7-(4-(4-(aminomethyl)-1-oxo-1,2-dihydrophthalazin-6-yl)-1-(methyl-d*s*)-1*H-*pyrazol-5-yl)-6-fluoro-3*H*-spiro[benzo[*b*][1,4]dioxine-2,1'-cyclopropane]-8-carbonitrile **3-p2**

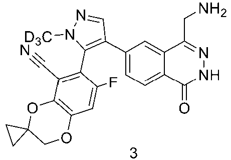

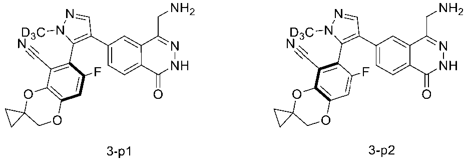

MS m/z (ESI): 462.1 [M+1].

### Example 4

### 6-(4-(4-(aminomethyl)-1-oxo-1,2-dihydrophthalazin-6-yl)-1-(methyl-d₃)-1H pyrazol-5-yl)-5-fluoro-3H-spiro[benzofuran-2,1'-cyclopropane]-7-carbonitrile 4 (M)-6-(4-(4-(aminomethyl)-1-oxo-1,2-dihydrophthalazin-6-yl)-1-(methyl-d₃)-1H-pyrazol-5-yl)-5-fluoro-3H-spiro[benzofuran-2,1'-cyclopropane]-7-carbonitrile 4-p1 (P)-6-(4-(4-(aminomethyl)-1-oxo-1,2-dihydrophthalazin-6-yl)-1-(methyl-d₃)-1H pyrazol-5-yl)-5-fluoro-3H-spiro[benzofuran-2,1'-cyclopropane]-7-carbonitrile 4-p2

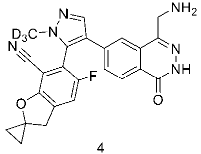

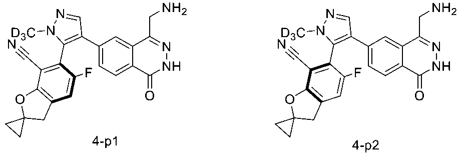

MS m/z (ESI): 446.1 [M+1].

### Example 5

(*R*)-8-(4-(4-(aminomethyl)-1-oxo-1,2-dihydrophthalazin-6-yl)-1-(methyl-*d₃*)-1*H-*pyrazol-5-yl)-7-fluoro-2,3,3a,4-tetrahydro-1H-benzo[*b*]pyrrolo[1,2-*d*][1,4]oxazine-9-carbonitrile **5**

(*M*)-(*R*)-8-(4-(4-(aminomethyl)-1-oxo-1,2-dihydrophthalazin-6-yl)-1-(methyl-*d₃*)-1*H*-pyrazol-5-yl)-7-fluoro-2,3,3a,4-tetrahydro-1H-benzo[*b*]pyrrolo[1,2-*d*][1,4]oxazine-9-carbonitrile **5-p1**

(*P*)-(*R*)-8-(4-(4-(aminomethyl)-1-oxo-1,2-dihydrophthalazin-6-yl)-1-(methyl-*d₃*)-1*H*-pyrazol-5-yl)-7-fluoro-2,3,3a,4-tetrahydro-1H-benzo[b]pyrrolo[1,2-*d*][1,4]oxazine-9-carbonitrile **5-p2**

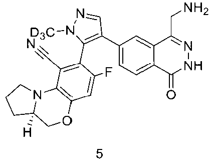

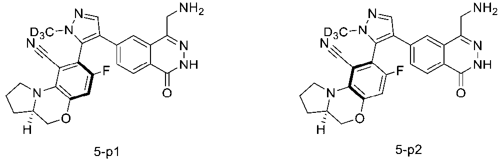

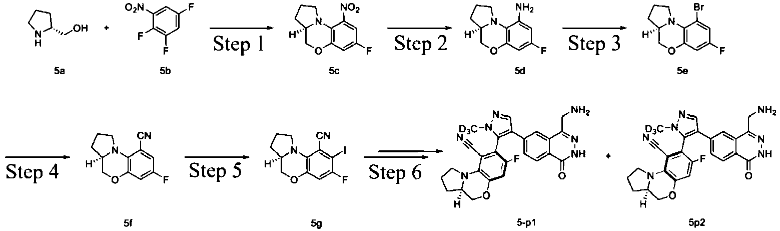

### Step 1

(*R*)-7-fluoro-9-nitro-2,3,3a,4-tetrahydro-1*H*-benzo[*b*]pyrrolo[1,2-*d*][1,4]oxazine **5c** *D*-prolinol **5a** (3.4 g, 33.6 mmol, Shaoyuan Technology (Shanghai) Co., Ltd.) and 2,3,5-trifluoronitrobenzene **5b** (5 g, 28.2 mmol, Shanghai Bepharm Science&Technology Co., Ltd.) were dissolved in dimethyl sulphoxide (20 mL), and potassium hydroxide (5.54 g, 98.7 mmol) was added. The reaction was stirred at 65°C for 4 hours. The reaction solution was cooled to room temperature, and then water was added. The mixture was extracted with dichloromethane (50 mL×3). The organic phases were combined, washed sequentially with water and a saturated sodium chloride solution, dried over anhydrous sodium sulphate and filtered to remove the drying agent, and then the filtrate was concentrated under reduced pressure. The residue was purified by silica gel column chromatography with eluent system B to obtain the title compound **5c** (5 g, yield: 74.6%).

MS m/z (ESI): 239.3 [M+1].

### Step 2

### (R)-7-fluoro-2,3,3a,4-tetrahydro-1H-benzo[b]pyrrolo[1,2-d][1,4]oxazine-9-amine 5d

Compound **5c** (1 g, 4.2 mmol) was dissolved in methanol (20 mL) and tetrahydrofuran (4 mL), and a 10% palladium on carbon hydrogenation catalyst (wet) (260 mg) was added. The mixture was purged with hydrogen, and the reaction was stirred for 16 hours. The reaction solution was filtered. The filtrate was concentrated under reduced pressure to obtain the crude title compound **5d** (900 mg), which was directly used in the next reaction without purification.

MS m/z (ESI): 223.3 [M+1].

### Step 3

### (R)-9-bromo-7-fluoro-2,3,3a,4-tetrahydro-1H-benzo[b]pyrrolo[1,2-d][1,4]oxazine 5e

At 0°C, cuprous bromide (744 mg, 5.18 mmol) was added to acetonitrile (10 mL), followed by dropwise addition of tert-butyl nitrite (668.5 mg, 6.5 mmol). The mixture was stirred for 10 minutes while maintaining the temperature, and then a solution of compound **5d** (900 mg, 4.3 mmol) in acetonitrile (3 mL) was added. The reaction was stirred at room temperature for 16 hours. The reaction solution was filtered. The filtrate was concentrated under reduced pressure. The residue was purified by silica gel column chromatography with eluent system B to obtain the title compound **5e** (160 mg, yield: 13.6%).

MS m/z (ESI): 272.2 [M+1].

### Step 4

### (R)-7-fluoro-2,3,3a,4-tetrahydro-1H-benzo[b]pyrrolo[1,2-d][1,4]oxazine-9-carbonitrile 5f

Compound **5e** (3 g, 11 mmol) was dissolved in *N,N*-dimethylacetamide (50 mL), and cuprous cyanide (4.9 g, 54.7 mmol) was added. The mixture was heated to 120°C and reacted for 16 hours. The reaction solution was cooled to room temperature, diluted in ethyl acetate and then filtered. The filtrate was washed sequentially with water and a saturated sodium chloride solution, dried over anhydrous sodium sulphate, filtered to remove the drying agent and then concentrated under reduced pressure. The residue was purified by silica gel column chromatography with eluent system B to obtain the title compound **5f** (1 g, yield: 41.5%).

MS m/z (ESI): 219.3 [M+1].

### Step 5

### (R)-7-fluoro-8-iodo-2,3,3a,4-tetrahydro-1H-benzo[b]pyrrolo[1,2-d][1,4]oxazine-9-carbonitrile 5g

Compound **5f** (1 g, 4.6 mmol) was dissolved in tetrahydrofuran (30 mL), and the mixture was cooled to -78°C. A solution of diisopropylaminolithium in tetrahydrofuran (2.5 mL, 2 M) was added dropwise, and the mixture was stirred for 30 minutes. Then, a solution of iodine (1.22 g, 4.8 mmol) in tetrahydrofuran (5 mL) was added, and the reaction was stirred for 30 minutes. The reaction was quenched by adding a saturated sodium sulphite solution and an aqueous sodium bicarbonate solution to the reaction solution. Liquid separation was performed. The aqueous phase was extracted with ethyl acetate (50 mL×3). The organic phases were combined, dried over anhydrous sodium sulphate, filtered to remove the drying agent and then concentrated under reduced pressure. The residue was purified by silica gel column chromatography with eluent system B to obtain the title compound **5g** (500 mg, yield: 31.8%).

MS m/z (ESI): 345.2 [M+1].

### Step 6

### (M)-(R)-8-(4-(4-(aminomethyl)-1-oxo-1,2-dihydrophthalazin-6-yl)-1-(methyl-d₃)-1H-pyrazol-5-yl)-7-fluoro-2,3,3a,4-tetrahydro-1H-benzo[b]pyrrolo[1,2-d][1,4]oxazine-9-carbonitrile 5-p1

(*P*)-(*R*)-8-(4-(4-(aminomethyl)-1-oxo-1,2-dihydrophthalazin-6-yl)-1-(methyl-*d₃*)-1*H*-pyrazol-5-yl)-7-fluoro-2,3,3a,4-tetrahydro-1*H*-benzo[*b*]pyrrolo[1,2-*d*][1,4]oxazine-9-carbonitrile **5-p2**

Using the synthetic route in Example 2, the title compounds **5-p1** (22 mg, yield: 14%) and **5-p2** (22 mg, yield: 14%) were obtained by replacing the starting material compound **1g** in step 1 with compound **5g.**

The single-configuration compound **5-p1** (with shorter retention time) (22 mg, yield: 14%).

MS m/z (ESI): 475.1 [M+1].

HPLC analysis: retention time: 1.19 minutes, purity: 98% (chromatographic column: ACQUITY UPLC^{®}BEH, C18, 1.7 µm, 2.1*50 mm; mobile phase: water (10 mM ammonium bicarbonate) and acetonitrile, gradient: acetonitrile 10%-95%).

¹H NMR (500 MHz, CD₃OD): δ 8.29 (d, 1H), 8.14 (s, 1H), 7.78 (dd, 1H), 7.74 -7.70 (m, 1H), 7.11 (d, 1H), 4.49 (dd, 1H), 4.18-4.07 (m, 1H), 3.99 (dd, 2H), 3.69-3.54 (m, 3H), 2.23 (dt, 1H), 2.01 (qdd, 2H), 1.76-1.65 (m, 1H).

The single-configuration compound **5-p2** (with longer retention time): (22 mg, yield: 14%).

MS m/z (ESI): 475.1 [M+1].

HPLC analysis: retention time: 1.21 minutes, purity: 98% (chromatographic column: ACQUITY UPLC^{®}BEH, C18, 1.7 µm, 2.1*50 mm; mobile phase: water (10 mM ammonium bicarbonate) and acetonitrile, gradient: acetonitrile 10%-95%).

¹H NMR (500 MHz, CD₃OD): δ 8.28 (d, 1H), 8.15 (s, 1H), 7.81-7.64 (m, 2H), 7.14 (d, 1H), 4.49 (d, 1H), 4.23-4.03 (m, 1H), 4.00 (d, 2H), 3.66-3.53 (m, 2H), 3.47-3.33 (m, 1H), 2.21 (dd, 1H), 2.00-1.85 (m, 2H), 1.69 (ddd, 1H).

### Example 6

6-(4-(4-(aminomethyl)-1-oxo-1,2-dihydrophthalazin-6-yl)-1-(methyl-*d₃*)-1*H* pyrazol-5-yl)-7-fluoro-2,3,3*a*,9*a*-tetrahydro-1*H-*benzo[*b*]cyclopenta[*e*][1,4]dioxine-5-carbonitrile **6** (*M*)-(3*aS*,9*aR*)-6-(4-(4-(aminomethyl)-1-oxo-1,2-dihydrophthalazin-6-yl)-1-(methyl-*d₃*)-1*H*-pyrazol-5-yl)-7-fluoro-2,3,3a,9a-tetrahydro-1*H-*benzo[b]cyclopenta[*e*][1,4]dioxine-5-carbonitrile **6-p1** (*M*)-(3*aR*,9*aS*)-6-(4-(4-(aminomethyl)-1-oxo-1,2-dihydrophthalazin-6-yl)-1-(methyl-*d₃*)-1*H*-pyrazol-5-yl)-7-fluoro-2,3,3*a*,9*a*-tetrahydro-1*H-*benzo[*b*]cyclopenta[*e*][1,4]dioxine-5-carbonitrile **6-p2** (*P*)-(3*aS*,9*aR*)-6-(4-(4-(aminomethyl)-1-oxo-1,2-dihydrophthalazin-6-yl)-1-(methyl-*d₃*)-1*H*-pyrazol-5-yl)-7-fluoro-2,3,3a,9a-tetrahydro-1*H-*benzo[*b*]cyclopenta[*e*][1,4]dioxine-5-carbonitrile **6-p3** (*P*)-(3*aR*,9*aS*)-6-(4-(4-(aminomethyl)-1-oxo-1,2-dihydrophthalazin-6-yl)-1-(methyl-*d₃*)-1*H*-pyrazol-5-yl)-7-fluoro-2,3,3a,9a-tetrahydro-1*H-*benzo[*b*]cyclopenta[*e*][1,4]dioxine-5-carbonitrile **6-p4**

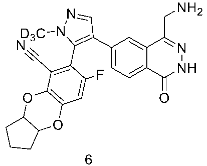

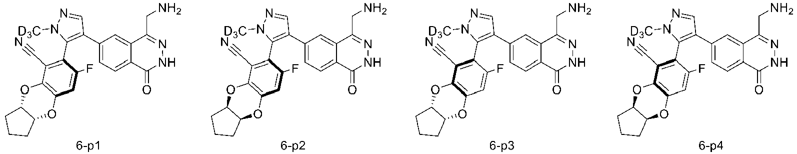

MS m/z (ESI): 476.1 [M+1].

### Example 7

2-(4-(4-(aminomethyl)-1-oxo-1,2-dihydrophthalazin-6-yl)-1-(methyl-*d3*)-1*H-*pyrazol-5-yl)-4*a*,5,6,6*a*-tetrahydrocyclobuta[b]thieno[2,3-*e*][1,4]dioxine-3-carbonitrile **7**

(4*aS*,6*aR*)-2-(4-(4-(aminomethyl)-1-oxo-1,2-dihydrophthalazin-6-yl)-1-(methyl-*d3*)-1*H-*pyrazol-5-yl)-4*a* 5,6,6*a*-tetrahydrocyclobuta[*b*]thieno[2,3-*e*][1,4]dioxine-3-carbonitrile **7-p1**

(4*aR*,6*aS*)-2-(4-(4-(aminomethyl)-1-oxo-1,2-dihydrophthalazin-6-yl)-1-(methyl-*d3*)-1*H*-pyrazol-5-yl)-4*a*,5,6,6*a*-tetrahydrocyclobuta[*b*]thieno[2,3-*e*][1,4]dioxine-3-carbonitrile **7-p2**

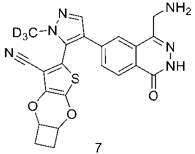

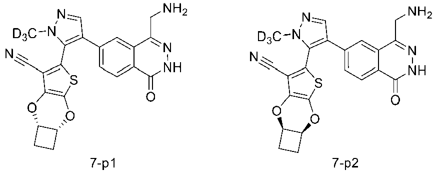

MS m/z (ESI): 450.1 [M+1].

### Example 8

(*S*)-8-(4-(4-(aminomethyl)-1-oxo-1,2-dihydrophthalazin-6-yl)-1-(methyl-*d₃*)-1*H-*pyrazol-5-yl)-7-fluoro-2,3,3a,4-tetrahydro-1*H*-benzo[*b*]pyrrolo[1,2-*d*][1,4]oxazine-9-carbonitrile **8**

(*M*)-(*S*)-8-(4-(4-(aminomethyl)-1-oxo-1,2-dihydrophthalazin-6-yl)-1-(methyl-*d₃*)-1*H*-pyrazol-5-yl)-7-fluoro-2,3,3a,4-tetrahydro-1*H*-benzo[*b*]pyrrolo[1,2-*d*][1,4]oxazine-9-carbonitrile **8-p1**

(*P*)-(*S*)-8-(4-(4-(aminomethyl)-1-oxo-1,2-dihydrophthalazin-6-yl)-1-(methyl-*d₃*)-1*H*-pyrazol-5-yl)-7-fluoro-2,3,3a,4-tetrahydro-1*H*-benzo[*b*]pyrrolo[1,2-*d*][1,4]oxazine-9-carbonitrile **8-p2**

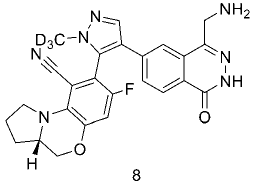

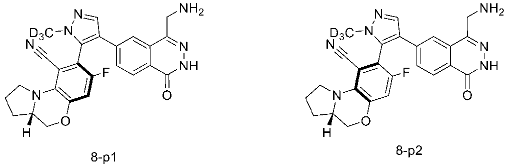

Using the synthetic route in Example 5, the title compounds **8-p2** (50 mg, yield: 7.4%) and **8-p1** (50 mg, yield: 7.4%) were obtained by replacing the starting material *D*-prolinol **5a** in step 1 with L-proline.

The single-configuration compound **8-p2** (with shorter retention time): (50 mg, yield: 7.4%).

MS m/z (ESI): 475.1 [M+1].

HPLC analysis: retention time: 1.09 minutes, purity: 98% (chromatographic column: ACQUITY UPLC^{®}BEH, C18, 1.7 µm, 2.1*50 mm; mobile phase: water (10 mM ammonium bicarbonate) and acetonitrile, gradient: acetonitrile 10%-95%).

¹H NMR (500 MHz, CD₃OD): δ 8.29 (d, 1H), 8.15 (s, 1H), 7.77 (dd, 1H), 7.73 (s, 1H), 7.12 (d, 1H), 4.49 (dd, 1H), 4.12 (dt, 1H), 4.03 (t, 2H), 3.64-3.54 (m, 3H), 2.23 (dq, 1H), 2.06-1.95 (m, 2H), 1.70 (dq, 1H).

The single-configuration compound **8-p1** (with longer retention time): (50 mg, yield: 7.4%).

MS m/z (ESI): 475.1 [M+1].

HPLC analysis: retention time: 1.11 minutes, purity: 98% (chromatographic column: ACQUITY UPLC^{®}BEH, C18, 1.7 µm, 2.1*50 mm; mobile phase: water (10 mM ammonium bicarbonate) and acetonitrile, gradient: acetonitrile 10%-95%).

MS m/z (ESI): 475.1 [M+1].

¹H NMR (500 MHz, CD₃OD): δ 8.28 (d, 1H), 8.16 (s, 1H), 7.78-7.71 (m, 2H), 7.15 (d, 1H), 4.49 (d, 1H), 4.10 (ddd, 1H), 4.07-3.96 (m, 2H), 3.66-3.56 (m, 2H), 3.42-3.35 (m, 1H), 2.21 (dq, 1H), 2.04-1.90 (m, 2H), 1.70 (dt, 1H).

### Example 9

(*R*)-9-(4-(4-(aminomethyl)-1-oxo-1,2-dihydrophthalazin-6-yl)-1-(methyl-*d₃*)-1*H-*pyrazol-5-yl)-8-fluoro-1,2,4*a*,5-tetrahydro-4*H*-benzo[*b*][1,4]oxazino[4,3-*d*][1,4]oxazine-10-carbonitrile 9

(*M*)-(*R*)-9-(4-(4-(aminomethyl)-1-oxo-1,2-dihydrophthalazin-6-yl)-1-(methyl-*d₃*)-1*H*-pyrazol-5-yl)-8-fluoro-1,2,4*a*,5-tetrahydro-4*H*-benzo[*b*][1,4]oxazino[4,3-*d*][1,4]oxazine-10-carbonitrile **9-p1**

(*P*)-(*R*)-9-(4-(4-(aminomethyl)-1-oxo-1,2-dihydrophthalazin-6-yl)-1-(methyl-*d₃*)-1*H*-pyrazol-5-yl)-8-fluoro-1,2,4*a*,5-tetrahydro-4*H*-benzo[*b*][1,4]oxazino[4,3-*d*][1,4]oxazine-10-carbonitrile **9-p2**

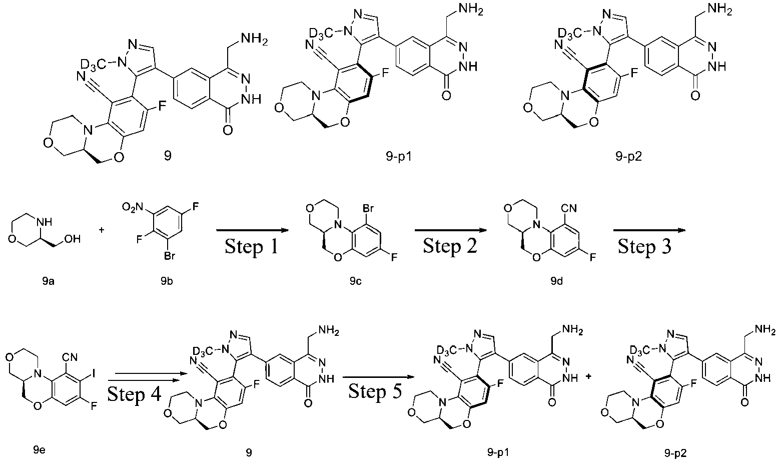

### Step 1

### (R)-10-bromo-8-fluoro-1,2,4a,5-tetrahydro-4H-benzo[b][1,4]oxazino[4,3-d][1,4]oxazine 9c

(*S*)-morpholin-3-ylmethanol **9a** (5 g, 42.6 mmol, Shanghai Titan Scientific Co., Ltd.) and 1-bromo-2,5-difluoro-3-nitrobenzene **9b** (8.4 g, 35.5 mmol, Jiangsu Aikon) were dissolved in dimethyl sulphoxide (60 mL), and potassium hydroxide (7 g, 124.4 mmol) was added. The reaction was stirred at 65°C for 4 hours. The reaction solution was cooled to room temperature, and then water was added. The mixture was extracted with dichloromethane (50 mL×3). The organic phases were combined, washed sequentially with water and a saturated sodium chloride solution, dried over anhydrous sodium sulphate and filtered to remove the drying agent, and then the filtrate was concentrated under reduced pressure. The residue was purified by silica gel column chromatography with eluent system B to obtain the title compound **9c** (5 g, yield: 48%).

MS m/z (ESI): 287.9 [M+1].

### Step 2

### (R)-8-fluoro-1,2,4a,5-tetrahydro-4H-benzo[b][1,4]oxazino[4,3-d][1,4]oxazine-10-carbonitrile 9d

Compound **9c** (5 *g,* 17.2 mmol) was dissolved in *N,N-*dimethylacetamide (100 mL), and zinc cyanide (3.5 g, 30.2 mmol), zinc powder (1.2 g, 18.1 mmol) and tetrakis(triphenylphosphine)palladium (2 g, 1.7 mmol) were added. The mixture was purged with nitrogen, heated to 100°C and reacted for 16 hours. The reaction solution was cooled to room temperature, diluted in ethyl acetate and then filtered. The filtrate was washed sequentially with water and a saturated sodium chloride solution, dried over anhydrous sodium sulphate, filtered to remove the drying agent and then concentrated under reduced pressure. The residue was purified by silica gel column chromatography with eluent system B to obtain the title compound **9d** (3.9 g, yield: 97%).

MS m/z (ESI): 235.3 [M+1].

### Step 3

### (R)-8-fluoro-9-iodo-1,2,4a,5-tetrahydro-4H-benzo[b][1,4]oxazino[4,3-d][1,4]oxazine-10-carbonitrile 9e

Compound **9d** (3.94 g, 16.8 mmol) was dissolved in tetrahydrofuran (250 mL), and the mixture was cooled to -78°C. A solution of diisopropylaminolithium in tetrahydrofuran (8.4 mL, 2 M) was added dropwise, and the mixture was stirred for 30 minutes. Then, a solution of iodine (4.2 g, 16.8 mmol) in tetrahydrofuran (50 mL) was added, and the reaction was stirred for 30 minutes. The reaction was quenched by adding a saturated sodium sulphite solution and an aqueous sodium bicarbonate solution to the reaction solution. Liquid separation was performed. The aqueous phase was extracted with ethyl acetate (50 mL×3). The organic phases were combined, dried over anhydrous sodium sulphate, filtered to remove the drying agent and then concentrated under reduced pressure. The residue was purified by silica gel column chromatography with eluent system B to obtain the title compound **9e** (1.1 g, yield: 18%).

MS m/z (ESI): 361.3 [M+1].

### Step 4 and step 5

(*R*)-9-(4-(4-(aminomethyl)-1-oxo-1,2-dihydrophthalazin-6-yl)-1-(methyl-_{d3})-1*H-*pyrazol-5-yl)-8-fluoro-1,2,4*a*,5-tetrahydro-4*H*-benzo[*b*][1,4]oxazino[4,3-*d*][1,4]oxazine-10-carbonitrile **9**

(*M*)-(*R*)-9-(4-(4-(aminomethyl)-1-oxo-1,2-dihydrophthalazin-6-yl)-1-(methyl-*d₃*)-1*H*-pyrazol-5-yl)-8-fluoro-1,2,4*a*,5-tetrahydro-4*H*-benzo[*b*][1,4]oxazino[4,3-*d*][1,4]oxazine-10-carbonitrile **9-p1**

(*P*)-(*R*)-9-(4-(4-(aminomethyl)-1-oxo-1,2-dihydrophthalazin-6-yl)-1-(methyl-*d₃*)-1*H*-pyrazol-5-yl)-8-fluoro-1,2,4*a*,5-tetrahydro-4*H*-benzo[*b*][1,4]oxazino[4,3-*d*][1,4]oxazine-10-carbonitrile **9-p2**

Using the synthetic route in Example 2, the crude title compound **9** (117 mg) was obtained by replacing the starting material compound **1g** in step 1 with compound **9e.** The crude title compound was resolved by a chiral column (Gilson-281, chromatographic column: CHIRALPAK IE, 20*250 mm, 5 µm; mobile phase A: n-hexane, mobile phase B: ethanol (0.5% 7 M ammonia-methanol solution), gradient: A:B is 50:50, flow rate: 15 mL/min) to obtain the title compounds **9-p2** (15.6 mg, yield: 13.3%) and **9-p1** (15 mg, yield: 12.8%).

The single-configuration compound **9-p2** (with shorter retention time) (15.6 mg, yield: 13.3%).

MS m/z (ESI): 491.2 [M+1].

Chiral HPLC analysis: retention time: 12.342 minutes, purity: 97.6% (chromatographic column: CHIRALPAKAD-H 250*4.6 mm, 5 µm; mobile phase: A: n-hexane, B: ethanol, gradient: A : B is 50 : 50, flow rate: 1.0 mL/min).

¹H NMR (500 MHz, DMSO-*d*₆): *δ* 12.42 (s, 1H), 8.24-8.12 (m, 2H), 7.80-7.66 (m, 2H), 7.45-7.40 (m, 1H), 4.48-4.26 (m, 2H), 3.87-3.64 (m, 6H), 3.48-3.44 (m, 3H), 2.12-1.84 (m, 2H).

The single-configuration compound **9-p1** (with longer retention time): (15 mg, yield: 12.8%).

MS m/z (ESI): 491.2 [M+1].

Chiral HPLC analysis: retention time: 18.774 minutes, purity: 83.2% (chromatographic column: CHIRALPAK AD-H 250*4.6 mm, 5 µm; mobile phase: A: n-hexane, B: ethanol, gradient: A:B is 90:10, flow rate: 1.0 mL/min).

¹H NMR (500 MHz, DMSO-*d*₆): *δ* 12.42 (s, 1H), 8.23-8.15 (m, 2H), 7.86-7.67 (m, 2H), 7.50-7.40 (m, 1H), 4.43-4.33 (m, 2H), 3.88-3.61 (m, 6H), 3.51-3.49 (m, 3H), 2.00-1.69 (m, 2H).

### Example 10

### (S)-9-(4-(4-(aminomethyl)-1-oxo-1,2-dihydrophthalazin-6-yl)-1-(methyl-d₃)-1H-pyrazol-5-yl)-8-fluoro-1,2,4a,5-tetrahydro-4H-benzo[b][1,4]oxazino[4,3-d][1,4]oxazine-10-carbonitrile 10

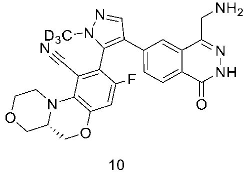

Using the synthetic route in Example 9, the title compound **10** (71.6 mg, yield: 24.2%) was obtained by replacing the starting material compound **9a** in step 1 with (*R*)-morpholin-3-ylmethanol (Shanghai Titan Scientific Co., Ltd.).

MS m/z (ESI): 491.2 [M+1].

¹H NMR (500 MHz, DMSO-*d*₆): *δ* 12.43 (s, 1H), 8.24-8.15 (m, 2H), 7.74-7.66 (m, 2H), 7.43-7.41 (m, 1H), 4.50-4.20 (m, 2H), 4.01-3.67 (m, 6H), 3.47-3.45 (m, 3H), 2.01-1.76 (m, 2H).

### Example 11

(*M*)-(2*aS*,8*aR*)-5-(4-(4-(aminomethyl)-1-oxo-1,2-dihydrophthalazin-6-yl)-1-(methyl-*d₃*)-1*H*-pyrazol-5-yl)-7-chloro-6-fluoro-1,2,2a,8a-tetrahydrobenzo[*b*]cyclobuta[*e*][1,4]dioxine-4-carbonitrile **11-p3** (*P*)-(2*aS*,8*aR*)-5-(4-(4-(aminomethyl)-1-oxo-1,2-dihydrophthalazin-6-yl)-1-(methyl-*d₃*)-1*H*-pyrazol-5-yl)-7-chloro-6-fluoro-1,2,2a,8a-tetrahydrobenzo[*b*]cyclobuta[*e*][1,4]dioxine-4-carbonitrile **11-p4**

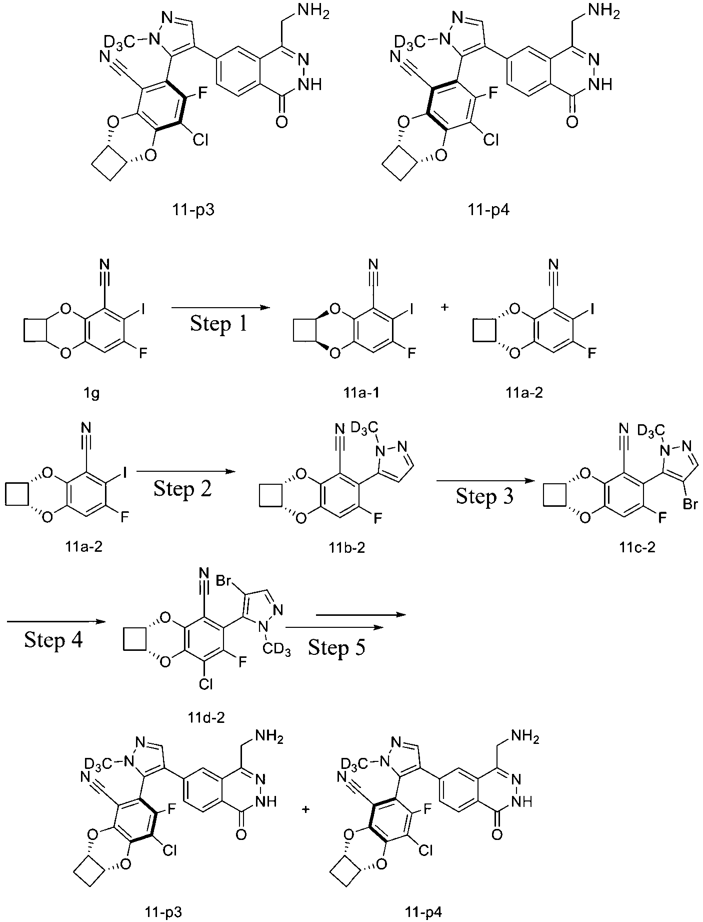

### Step 1

### (2aR,8aS)-6-fluoro-5-iodo-1,2,2a,8a-tetrahydrobenzo[b]cyclobuta[e][1,4]dioxine-4-carbonitrile 11a-1

### (2aS, 8aR)-6-fluoro-5-iodo-1,2,2a,8a-tetrahydrobenzo[b]cyclobuta[e][1,4]dioxine-4-carbonitrile 11a-2

Compound **1g** (11.6 g) was resolved by a chiral column (Waters SFC 150, chromatographic column: DAICELCHIRALCEL^{®}OJ, 40*250 mm, 10 µm; mobile phase A: supercritical carbon dioxide, mobile phase B: methanol (+0.1% 7 M ammonia-methanol solution), gradient: A : B = 70 : 30, flow rate: 140 mL/min) to obtain the title compounds (5.4 g, yield: 46.5%) and (5.5 g, yield: 47.4%).

The single-configuration compound **11a-1** (with shorter retention time): (5.4 g, yield: 46.5%)

Chiral HPLC analysis: retention time: 1.599 minutes, purity: 99% (chromatographic column: DAICELCHIRALCEL^{®}OJ, 100*3 mm, 3 µm; mobile phase A: supercritical carbon dioxide, mobile phase B: methanol (+0.1% diethylamine), gradient: A : B = 90 : 10, flow rate: 1.5 mL/min).

The single-configuration compound **11a-2** (with longer retention time): (5.5 g, yield: 47.4%)

Chiral HPLC analysis: retention time: 2.274 minutes, purity: 99% (chromatographic column: DAICELCHIRALCEL^{®}OJ, 100*3 mm, 3 µm; mobile phase A: supercritical carbon dioxide, mobile phase B: methanol (+0.1% diethylamine), gradient: A : B = 90 : 10, flow rate: 1.5 mL/min).

### Step 2

### (2aS,8aR)-6-fluoro-5-(1-(methyl-d₃)-1H-pyrazol-5-yl)-1,2,2a,8a-tetrahydrobenzo[b]cyclobuta[e][1,4]dioxine-4-carbonitrile 11b-2

Compound **2a** (7 g, 33.1 mmol) and compound **11a-2** (5.5 g, 33.2 mmol) were dissolved in water (24 mL) and 1,4-dioxane (120 mL), and then sodium carbonate (3.5 g, 15.1 mmol) and 1,1'-bis(di-tert-butylphosphino)ferrocene palladium dichloride (1.1 g, 1.7 mmol) were added. The mixture was heated to 85°C and reacted for 16 hours. The reaction solution was concentrated under reduced pressure. The residue was dissolved in dichloromethane and then filtered, and the filtrate was concentrated under reduced pressure. The residue was purified by silica gel column chromatography with eluent system B to obtain the title compound **11b-2** (4.15 g, yield: 86.7%).

MS m/z (ESI): 289.0 [M+1].

### Step 3

### (2aS,8aR)-5-(4-bromo-1-(methyl-d₃)-1H-pyrazol-5-yl)-6-fluoro-1,2,2a,8a-tetrahydrobenzo[b]cyclobuta[e][1,4]dioxine-4-carbonitrile 11c-2

Compound **11b-2** (4.15 g, 14.4 mmol) was dissolved in acetonitrile (70 mL), and N-bromosuccinimide (2.82 g, 15.7 mmol) was added. The mixture was heated to 50°C and reacted for 3 hours. The reaction solution was cooled to room temperature, and then the reaction was quenched with a saturated sodium bicarbonate solution and a saturated sodium thiosulphate solution. The organic phase was separated, and the aqueous phase was extracted with ethyl acetate (50 mL ×3). The organic phases were combined, dried over anhydrous sodium sulphate, filtered to remove the drying agent and then concentrated under reduced pressure. The residue was purified by silica gel column chromatography with eluent system B to obtain the title compound **11c-2** (5.1 g, yield: 96.7%).

MS m/z (ESI): 367.0 [M+1].

### Step 4

### (2aS,8aR)-5-(4-bromo-1-(methyl-d₃)-1H-pyrazol-5-yl)-7-chloro-6-fluoro-1,2,2a,8a-tetrahydrobenzo[b]cyclobuta[e][1,4]dioxine-4-carbonitrile 11d-2

Compound **11c-2** (530 mg, 1.44 mmol) was dissolved in tetrahydrofuran (10 mL), and the mixture was cooled to -78°C. A solution of diisopropylaminolithium in tetrahydrofuran (0.87 mL, 2 M) was added dropwise, and the mixture was stirred for 30 minutes. Then, a solution of hexachloroethane (512.6 mg, 2.16 mmol) in tetrahydrofuran (2 mL) was added, and the reaction was stirred for 30 minutes. The reaction was quenched by adding a saturated sodium sulphite solution and an aqueous sodium bicarbonate solution to the reaction solution. Liquid separation was performed. The aqueous phase was extracted with ethyl acetate (15 mL×3). The organic phases were combined, dried over anhydrous sodium sulphate, filtered to remove the drying agent and then concentrated under reduced pressure. The residue was purified by silica gel column chromatography with eluent system B to obtain the title compound **11d-2** (500 mg, yield: 86%).

MS m/z (ESI): 401.0 [M+1].

### Step 5

(*M*)-(2*aS*,8*aR*)-5-(4-(4-(aminomethyl)-1-oxo-1,2-dihydrophthalazin-6-yl)-1-(methyl-*d*₃)-1*H*-pyrazol-5-yl)-7-chloro-6-fluoro-1,2,2*a*,8*a-*tetrahydrobenzo[*b*]cyclobuta[*e*][1,4]dioxine-4-carbonitrile **11-p3**

(*P*)-(2*aS*,8*aR*)-5-(4-(4-(aminomethyl)-1-oxo-1,2-dihydrophthalazin-6-yl)-1-(methyl-*d₃*)-1*H*-pyrazol-5-yl)-7-chloro-6-fluoro-1,2,2*a*,8*a-*tetrahydrobenzo[*b*]cyclobuta[*e*][1,4]dioxine-4-carbonitrile **11-p4**

Using step 3 to step 5 of the synthetic route in Example 2, the title compounds **11-p3** (6 mg, yield: 4.7%) and **11-p4** (10 mg, yield: 7.9%) were obtained by replacing the starting material compound **2c** in step 3 with compound **11d-2.**

The single-configuration compound **11-p3** (with shorter retention time) (6 mg, yield: 4.7%).

MS m/z (ESI): 496.1 [M+1].

HPLC analysis: retention time: 1.08 minutes, purity: 98% (chromatographic column: ACQUITY UPLC^{®}BEH, C18, 1.7 µm, 2.1*50 mm; mobile phase: water (10 mM ammonium bicarbonate) and acetonitrile, gradient: acetonitrile 10%-95%).

¹H NMR (500 MHz, CD₃OD): δ 8.30 (d, 1H), 8.16 (s, 1H), 7.80 (s, 1H), 7.66 (d, 1H), 5.00 (dq, 1H), 4.96 - 4.91 (m, 1H), 4.15 - 4.01 (m, 2H), 2.42 - 2.20 (m, 4H).

The single-configuration compound **11-p4** (with longer retention time): (10 mg, yield: 7.9%).

MS m/z (ESI): 496.1 [M+1].

HPLC analysis: retention time: 1.11 minutes, purity: 94% (chromatographic column: ACQUITY UPLC^{®}BEH, C18, 1.7 µm, 2.1*50 mm; mobile phase: water (10 mM ammonium bicarbonate) and acetonitrile, gradient: acetonitrile 10%-95%).

¹H NMR (500 MHz, CD₃OD): δ 8.28 (d, 1H), 8.16 (s, 1H), 7.79 (s, 1H), 7.63 (d, 1H), 5.00 (qt, 1H), 4.96 - 4.92 (m, 1H), 4.13 - 4.00 (m, 2H), 2.43 - 2.15 (m, 4H).

### Example 12

### 6-(4-(4-(aminomethyl)-1-oxo-1,2-dihydrophthalazin-6-yl)-1-(methyl-d₃)-1H-pyrazol-5-yl)-5-fluoro-2H-spiro[benzofuran-3,1'-cyclopropane]-7-carbonitrile 12

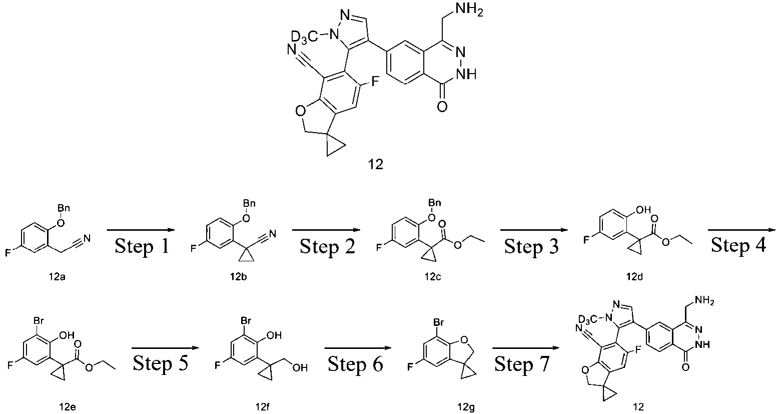

### Step 1

### 1-(2-(benzyloxy)-5-fluorophenyl)cyclopropane-1-carbonitrile 12b

2-(2-(benzyloxy)-5-fluorophenyl) acetonitrile **12a** (23.6 g, 97.8 mmol, prepared according to the method disclosed in patent application WO 2011046771, description, page 148, example 13) and tetrabutylammonium bromide (1.5 g, 5.24 mmol) were dissolved in *N,N*-dimethylformamide (DMF) (400 mL). In an ice bath, sodium hydride (8.22 g, 205.51 mmol, purity: 60%) was added, and the mixture was reacted at room temperature for 15 minutes. A solution of 1,2-dibromoethane (21 g, 111.78 mmol) in *N,N*-dimethylformamide (DMF) (100 mL) was slowly added dropwise, and the reaction was stirred for 3 hours, quenched with a saturated ammonium chloride solution, and extracted with dichloromethane (150 mL × 3). The organic phases were combined and concentrated under reduced pressure. The residue was purified by silica gel column chromatography with eluent system B to obtain the title compound **12b** (25 g, yield: 95.6%)

MS m/z (ESI): 268.2 [M+1].

### Step 2

### Ethyl 1-(2-(benzyloxy)-5-fluorophenyl)cyclopropane-1-carboxylate 12c

Compound **12b** (25 g, 93.5 mmol) was dissolved in ethanol (300 mL), and concentrated sulphuric acid (300 mL) was added. The mixture was reacted at 80°C for 16 hours. The reaction solution was concentrated under reduced pressure. The residue was poured into ice water, and the mixture was extracted with ethyl acetate (100 mL × 3). The organic phases were combined and concentrated under reduced pressure. The residue was purified by silica gel column chromatography with eluent system B to obtain the title compound **12c** (15 g, yield: 51%)

MS m/z (ESI): 315.2 [M+1].

### Step 3

### Ethyl 1-(5-fluoro-2-hydroxylphenyl)cyclopropane-1-carboxylate 12d

Compound **12c** (1 g, 3.18 mmol) was dissolved in methanol (20 mL) and, and a palladium on carbon hydrogenation catalyst (wet) (200 mg) was added. The mixture was purged with hydrogen, and the reaction was stirred for 3 hours. The reaction solution was filtered. The filtrate was concentrated under reduced pressure to obtain the crude title compound **12d** (650 mg), which was directly used in the next reaction without purification.

### Step 4

### Ethyl 1-(3-bromo-5-fluoro-2-hydroxylphenyl)cyclopropane-1-carboxylate 12e

Compound **12d** (730 mg, 3.2 mmol) was dissolved in acetonitrile (20 mL), and *N*-bromosuccinimide (584 mg, 3.2 mmol) was added. The reaction was stirred for 2 hours and quenched by adding a saturated sodium sulphite solution and an aqueous sodium bicarbonate solution to the reaction solution. The liquid separation was performed, and the aqueous phase was extracted with ethyl acetate (20 mL ×3). The organic phases were combined, dried over anhydrous sodium sulphate, filtered to remove the drying agent and then concentrated under reduced pressure. The residue was purified by silica gel column chromatography with eluent system B to obtain the title compound **12e** (820 mg, yield: 83%).

MS m/z (ESI): 301.2 [M-1].

### Step 5

### 2-bromo-4-fluoro-6-(1-(hydroxymethyl)cyclopropyl)phenol 12f

Compound **12e** (820 mg, 2.7 mmol) was dissolved in tetrahydrofuran (50 mL). At 0°C, a solution of lithium aluminium hydride in tetrahydrofuran (3.2 mL, 1 M) was added dropwise. The reaction was allowed to warm to room temperature naturally and stirred for 2 hours. Water (0.2 mL) and a sodium hydroxide solution (0.2 mL, 20%) were sequentially added to the reaction solution, and the mixture was stirred for 10 minutes and then filtered. The filtrate was dried over anhydrous sodium sulphate, filtered to remove the drying agent and then concentrated under reduced pressure. The residue was purified by silica gel column chromatography with eluent system B to obtain the title compound **12f** (580 mg, yield: 82%).

MS m/z (ESI): 261.0 [M+1].

### Step 6

### 7-bromo-5-fluoro-2H-spiro[benzofuran-3,1'-cyclopropane] 12g

Compound **12f** (580 mg, 2.22 mmol) and azodicarboxylic acid dipiperidide (ADDP) (841 mg, 3.33 mmol) were dissolved in toluene (20 mL). The mixture was purged with nitrogen and heated to 50°C. Tributylphosphine (675 mg, 3.33 mmol) was added, and the reaction was further stirred for 17 hours. The reaction solution was concentrated under reduced pressure. The residue was purified by silica gel column chromatography with eluent system B to obtain the title compound **12g** (540 mg, yield: 99%).

### Step 7

### 6-(4-(4-(aminomethyl)-1-oxo-1,2-dihydrophthalazin-6-yl)-1-(methyl-d₃)-1H-pyrazol-5-yl)-5-fluoro-2H-spiro[benzofuran-3,1'-cyclopropane]-7-carbonitrile 12

Using step 2 to step 4 of the synthetic route in Example 9, the title compound **12** (102 mg, yield: 41.1%) was obtained by replacing the starting material compound **9c** in step 2 with compound **12g.**

MS m/z (ESI): 446.2 [M+1].

¹H NMR (500 MHz, CD₃OD): δ 7.29-7.27 (m, 1H), 8.14 (s, 1H), 7.74-7.73 (m, 1H), 7.70-7.68 (m, 1H), 7.19-7.18 (m, 1H), 4.81-4.76 (m, 2H), 4.03-3.96 (m, 2H), 1.38-1.30 (m, 4H).

### Example 13

(*S*)-8-(4-(4-(aminomethyl)-1-oxo-1,2-dihydrophthalazin-6-yl)-1-(methyl-*d₃*)-1*H-*pyrazol-5-yl)-6-chloro-7-fluoro-2,3,3a,4-tetrahydro-1*H*-benzo[b]pyrrolo[1,2-*d*][1,4]oxazine-9-carbonitrile **13**

(*M*)-(*S*)-8-(4-(4-(aminomethyl)-1-oxo-1,2-dihydrophthalazin-6-yl)-1-(methyl-d₃)-1*H*-pyrazol-5-yl)-6-chloro-7-fluoro-2,3,3a,4-tetrahydro-1*H*-benzo[*b*]pyrrolo[1,2-*d*][1,4]oxazine-9-carbonitrile **13-p1**

(*P*)-(*S*)-8-(4-(4-(aminomethyl)-1-oxo-1,2-dihydrophthalazin-6-yl)-1-(methyl-*d₃*)-1*H*-pyrazol-5-yl)-6-chloro-7-fluoro-2,3,3a,4-tetrahydro-1*H*-benzo[*b*]pyrrolo[1,2-*d*][1,4]oxazine-9-carbonitrile **13-p2**

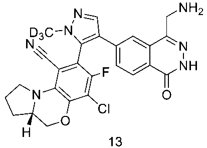

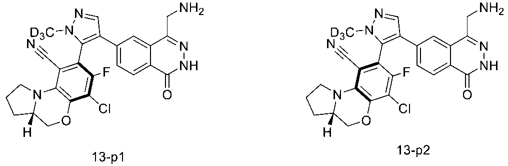

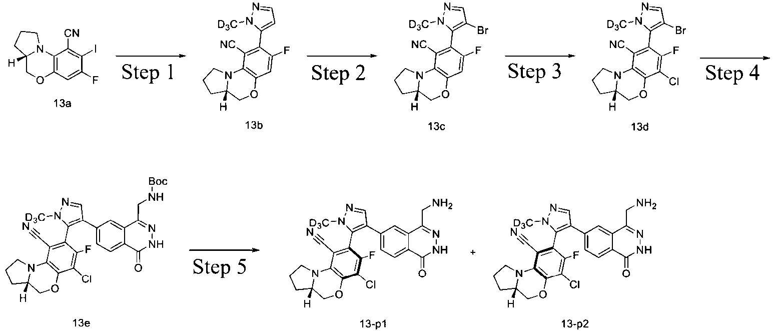

### Step 1

### (S)-7-fluoro-8-(1-(methyl-d₃)-1H-pyrazol-5-yl)-2,3,3a,4-tetrahydro-1H-benzo[b]pyrrolo[1,2-d][1,4]oxazine-9-carbonitrile 13b

Compound **13a** (12.3 g, 35.7 mmol, obtained by using the synthetic route for 5g in Example 5 and replacing the starting material *D*-prolinol 5a in step 1 with *L-*prolinol) and compound **2a** (15.1 g, 71.5 mmol) were dissolved in water (40 mL) and 1,4-dioxane (200 mL), and then sodium carbonate (7.6 g, 71.4 mmol) and 1,1'-bis(di-tert-butylphosphino)ferrocene palladium dichloride (2.43 mg, 3.56 mmol) were added. The mixture was purged with nitrogen, heated to 85°C and reacted for 16 hours. The reaction solution was concentrated under reduced pressure. The residue was dissolved in dichloromethane and then filtered, and the filtrate was concentrated under reduced pressure. The residue was purified by silica gel column chromatography with eluent system B to obtain the title compound **13b** (9 g, yield: 83%).

MS m/z (ESI): 302.1 [M+1].

### Step 2

### (S)-8-(4-bromo-1-(methyl-d₃)-1H-pyrazol-5-yl)-7-fluoro-2,3,3a,4-tetrahydro-1H-benzo[b]pyrrolo[1,2-d][1,4]oxazine-9-carbonitrile 13c

Compound **13b** (9 g, 29.8 mmol) was dissolved in acetonitrile (150 mL), and *N*-bromosuccinimide (5.84 g, 32.8 mmol) was added. The reaction was stirred for 2 hours. The reaction solution was concentrated under reduced pressure. The residue was purified by silica gel column chromatography with eluent system B to obtain the title compound **13c** (8.4 g, yield: 74.0%).

MS m/z (ESI): 380.0 [M+1].

### Step 3

### (S)-8-(4-bromo-1-(methyl-d₃)-1H-pyrazol-5-yl)-6-chloro-7-fluoro-2,3,3a,4-tetrahydro-1H-benzo[b]pyrrolo[1,2-d][1,4]oxazine-9-carbonitrile 13d

Compound **13c** (480 mg, 1.26 mmol) was dissolved in tetrahydrofuran (10 mL), and the mixture was cooled to -78°C. A solution of diisopropylaminolithium in tetrahydrofuran (0.88 mL, 2 M) was added dropwise, and the mixture was stirred for 30 minutes. Then, a solution of hexachloroethane (448 mg, 1.89 mmol) in tetrahydrofuran (2 mL) was added, and the reaction was stirred for 30 minutes. The reaction was quenched by adding a saturated sodium sulphite solution and an aqueous sodium bicarbonate solution to the reaction solution. Liquid separation was performed. The aqueous phase was extracted with ethyl acetate (15 mL×3). The organic phases were combined, dried over anhydrous sodium sulphate, filtered to remove the drying agent and then concentrated under reduced pressure. The residue was purified by silica gel column chromatography with eluent system B to obtain the title compound **13d** (288 mg, yield: 55%).

MS m/z (ESI): 414.0 [M+1].

### Step 4

### Tert-butyl (S)-((7-(5-(6-chloro-9-cyano-7-fluoro-2,3,3a,4-tetrahydro-1H-benzo[b]pyrrolo[1,2-d][1,4]oxazin-8-yl)-1-(methyl-d₃)-1H-pyrazol-4-yl)-4-oxo-3,4-dihydrophthalazin-1-yl)methyl)carbamate 13e

Under nitrogen atmosphere, compound **13d** (54 mg, 0.131 mmol), tert-butyl((4-oxo-7-(4,4,5,5-tetramethyl-1,3,2-dioxoboron-2-yl)-3,4-dihydrophthalazin-1-yl)methyl)carbamate (105 mg, 0.262 mmol, prepared according to the method disclosed in Org. Process Res. Dev. 2023, 27, 5, 954-971), 1,1'-bis(di-tert-butylphosphino)ferrocene palladium dichloride (18.6 mg, 0.026 mmol) and sodium carbonate (35 mg, 0.327 mmol) were dissolved in 1,4-dioxane (2 mL) and water (0.4 mL). The reaction was stirred overnight at 85°C. The reaction solution was concentrated under reduced pressure. The residue was dissolved in dichloromethane and then filtered. The filtrate was concentrated under reduced pressure to obtain the crude compound **13e,** which was directly used in the next step without purification (80 mg).

MS m/z (ESI): 609.2 [M+1].

### Step 5

(*M*)-(*S*)-8-(4-(4-(aminomethyl)-1-oxo-1,2-dihydrophthalazin-6-yl)-1-(methyl-*d₃*)-1*H*-pyrazol-5-yl)-6-chloro-7-fluoro-2,3,3*a*,4-tetrahydro-1*H*-benzo[*b*]pyrrolo[1,2-*d*][1,4]oxazine-9-carbonitrile **13-p1**

(*P*)-(*S*)-8-(4-(4-(aminomethyl)-1-oxo-1,2-dihydrophthalazin-6-yl)-1-(methyl-*d₃*)-1*H*-pyrazol-5-yl)-6-chloro-7-fluoro-2,3,3*a*,4-tetrahydro-1*H*-benzo[*b*]pyrrolo[1,2-*d*][1,4]oxazine-9-carbonitrile **13-p2**

Compound **13e** (80 mg, 0.131 mmol) was dissolved in dichloromethane (2 mL) and trifluoroacetic acid (2 mL). At room temperature, the reaction was stirred for 1 hour. The reaction solution was concentrated under reduced pressure, and the residue was purified by high-performance liquid chromatography (Waters-2545 YMC Triart-Exrs, Prep 30*150 mm, 5 µm (10 mmol/L) 30%-42%, 30 mL/min) to obtain the target compound.

The single-configuration compound **13-p2** with shorter retention time: (4 mg, 6%)

HPLC analysis: retention time: 0.98 minutes, purity: 90% (chromatographic column: ACQUITY UPLC^{®}BEH, C18, 1.7 µm, 2.1*50 mm; mobile phase: water (10 mM ammonium bicarbonate) and acetonitrile, gradient: acetonitrile 10%-95%).

¹H NMR (500 MHz, CD₃OD) δ 8.29 (d, 1H), 8.17 (s, 1H), 7.87 (d, 1H), 7.62 (dd, 1H), 4.69 - 4.61 (m, 1H), 4.58 - 4.47 (m, 2H), 4.13 (dt, 1H), 3.70 - 3.60 (m, 3H), 2.30 - 2.19 (m, 1H), 2.02 (tdd, 2H), 1.80 - 1.70 (m, 1H).

MS m/z (ESI): 509.2 [M+1].

The single-configuration compound **13-p1** with longer retention time: (4 mg, 6%)

¹H NMR (500 MHz, CD₃OD) δ 8.27 (d, 1H), 8.18 (s, 1H), 7.95 (d, 1H), 7.54 (dd, 1H), 4.67 - 4.61 (m, 1H), 4.61 - 4.52 (m, 2H), 4.10 (dt, 1H), 3.70 - 3.60 (m, 2H), 3.43 (dt, 1H), 2.28 - 2.19 (m, 2H), 2.00 (pd, 2H), 1.76 - 1.68 (m, 1H).

HPLC analysis: retention time: 1.01 minutes, purity: 90% (chromatographic column: ACQUITY UPLC^{®}BEH, C18, 1.7 µm, 2.1*50 mm; mobile phase: water (10 mM ammonium bicarbonate) and acetonitrile, gradient: acetonitrile 10%-95%).

MS m/z (ESI): 509.2 [M+1].

### Biological evaluation

The present disclosure will be further described and explained below in conjunction with test examples, but these examples are not intended to limit the scope of the present disclosure.

### Test example 1: Inhibitory activity of compounds of the present disclosure against human PRMT5 enzymatic activity

### I. Experimental materials and instruments

1. Recombinant human PRMT5: MEP50 protein (active motif, 31521)
2. EPIgeneous Methyltransferase kit (cisbio, 62SAHPEH)
3. 96-well plate (Sangon Biotech, F610960-0010)
4. 384-well white plate (Perkin Elmer, 6007290)
5. 1 M Tris-HCl [pH 8.5] (Sangon Biotech, B548141)
6. Histone H4 peptide substrates (1-21) (BPS bioscience, 52014)
7. 5'-deoxy-5'-(methylthio)adenosine (Aladdin, D168979-25 mg)
8. Tween-20 (Sangon Biotech, A100777-0500)
9. DMSO (Sigma, D2650)
10. Incubator (Shanghai Boxun Medical Biological Instrument Corp.)
11. PHERAstar FS microplate reader (BMG Labtech)

### II. Experimental procedure

PRMT5 can induce the transfer of a methyl group from S-adenosylmethionine (SAM) to a histone H4 peptide substrate (1-21), generating S-adenosylhomocysteine (SAH). In this experiment, the amount of SAH produced in the reaction is quantified using a Homogeneous Time-Resolved Fluorescence (HTRF) method to evaluate the inhibitory activity of compounds against PRMT5 enzymatic activity.

### 1. Standard curve plotting

The sample diluent containing (or not containing) 1 µM 5 '-deoxy-5' - (methylthiyl) adenosine (MTA) was prepared using the EPIgeneous Diluent Buffer from the EPIgeneous Methyltransferase kit. With reference to the instructions, SAM and SAH standards were serially diluted and dispensed at 10 µL per well (in duplicate for each concentration). 2 µL of EPIgeneous Detection Buffer 1 was added to each well, and the plate was centrifuged, shaken to mix thoroughly, and placed at room temperature for 10 min. The SAH d2 Reagent was diluted 32-fold with EPIgeneous Detection Buffer 2, and 4 µL of the diluted SAH-d2 solution was added to each well. The plate was centrifuged and shaken for 2 min to mix thoroughly. The SAH Tb Cryptate Antibody was diluted 50-fold in EPIgeneous Detection Buffer 2. 4 µL of the diluted Tb Cryptate Antibody solution was added to each well, and the plate was centrifuged, shaken for 2 min to mix thoroughly, and left to stand at room temperature for 60 min, followed by reading on a PHERAstar FS microplate reader to obtain the 665/620 ratio. Plotting SAH concentration against the ratio to generate an x-y curve yields a corresponding calculation formula.

### 2. Compound detection

Reaction buffer (1×): 50 mM Tris-HCl [pH 8.5], 1 mM DTT, 0.005% Tween-20.

Compound preparation: a 20 mM solution of test compound in 100% DMSO was diluted in 100% DMSO to 1 mM as the highest concentration, and the solution at this concentration was then subjected to a 10-point, 3-fold serial dilution in 100% DMSO in a 96-well plate. Wells containing 100% DMSO served as the blank control. The above-described serially diluted compounds and the 100% DMSO were diluted 20-fold with the reaction buffer to prepare 5× compound solutions, which were mixed thoroughly for use.

The PRMT5:MEP50 was diluted in the reaction buffer to a final concentration of 12.5 nM. Then, 4 µL of the 12.5 nM PRMT5:MEP50 solution was added to each well of a 384-well plate. Wells containing 4 µL of the reaction buffer served as the negative control wells. 2 µL of the 5× compound solution was added to each well. For the negative control wells (no enzyme, no compound) and the positive control wells (no compound), 2 µL of 5% DMSO was added. The plate was sealed with a membrane, centrifuged, shaken for 2 min to mix thoroughly, and placed at room temperature for 20 min.

Preparation of substrate mixture solution: The substrate mixture solution containing 1.5 µM histone H4 peptide substrate (1-21) and 2.5 µM SAM, or 1.5 µM histone H4 peptide substrate (1-21), 2.5 µM SAM and 2.5 µM MTA was prepared in the reaction buffer.

The reaction was initiated by adding 4 µL of the substrate mixture solution (-/+ MTA) to each well. The plate was sealed with a membrane, centrifuged, shaken for 2 min to mix thoroughly, and incubated at 37°C for 120 min in a constant temperature incubator.

Detection: The detection was performed by using the EPIgeneous Methyltransferase kit. 2 µL of EPIgeneous Detection Buffer 1 was added to each well, and the plate was centrifuged, shaken to mix thoroughly, and placed at room temperature for 10 min. The SAH d2 Reagent was diluted 32-fold with EPIgeneous Detection Buffer 2, and 4 µL of the diluted SAH-d2 solution was added to each well. The plate was centrifuged and shaken for 2 min to mix thoroughly. The SAH Tb Cryptate Antibody was diluted 50-fold in EPIgeneous Detection Buffer 2. 4 µL of the diluted Tb Cryptate Antibody solution was added to each well, and the plate was centrifuged, shaken for 2 min to mix thoroughly, and left to stand at room temperature for 60 min, followed by reading on a PHERAstar FS microplate reader to obtain the 665/620 ratio.

The ratio values were substituted into the formula for conversion. Using Graphpad Prism software, an inhibition curve was plotted based on the concentration of each compound and its corresponding inhibition rate. The compound concentration at which the inhibition rate reached 50% was then calculated, i.e., the IC₅₀ value.

**Table 1. IC₅₀ values of inhibitory activity of compounds of the present disclosure against human PRMT5**

| Compound No. | IC₅₀ (nM) +1 µM MTA |
|---|---|
| 1-p4 | 5.48 |
| 1-p1 | 4.91 |
| 2-p4 | 5.87 |
| 2-p1 | 5.23 |
| 2-p3 | 5.31 |
| 2-p2 | 7.01 |
| 5-p1 | 7.67 |
| 5-p2 | 4.91 |
| 8-p2 | 3.86 |
| 8-p1 | 5.19 |
| 11-p3 | 5.40 |
| 12 | 6.39 |
| 13-p1 | 2.80 |

| | |
|---|---|
| Conclusion: The compounds of the present disclosure exhibit good inhibitory activity against PRMT5 (+MTA). | |

### Test example 2: HCT116 and HCT116 MTAP-/- cell proliferation assay

I. Experimental materials and instruments
   1. HCT116 cell line (Nanjing Kebai Biotechnology Co., Ltd., CBP60028)
   2. HCT116 MTAP-/- cell line (Nanjing Kebai Biotechnology Co., Ltd., CBP75002)
   3. DMSO (Sigma, D2650)
   4. McCoy's 5A medium (Gibco, 16600-082)
   5. Foetal bovine serum (FBS) (Gibco, 10091148)
   6. Phosphate buffered saline (DPBS) pH 7.4 (Gibco, 14190-144)
   7. Pen strep (PS) (Gibco, 15140-122)
   8. 0.25% Typsin-EDTA (1×) (Gibco, 25200-072)
   9. CellTiter-Glo^{®} Luminescent Cell Viability Assay (Promega, G7572)
   10. 96-well round-bottom plate (JET, TCP002096)
   11. 96-well black clear bottom plate (Hangzhou Xinyou Biotechnology Co., Ltd., 060096)
   12. 1.2 mL 96-well deep well plate, clear, sterile, square-well, V-bottom (Titan, 02089063)
   13. 15-mL centrifuge tube (Titan)
   14. Biosafety cabinet (Thermo, 1300AII)
   15. Cell counter (Countstar, IC1000)
   16. Incubator (Thermo, I160)
   17. Centrifuge (Beckman coulter, Allegra X-12 centrifuge)
   18. PHERAstar FS microplate reader (BMG Labtech)
II. Experimental procedure
   1. Cell plating (Day 0)
      a) The cell morphology was observed under a microscope to ensure confluence reached approximately 90%.
      b) The cell supernatant was aspirated, the cells were washed once with PBS, and then the PBS was removed; and the cells were digested by adding an appropriate amount of trypsin, and left to stand at 37°C for 3 minutes.
      c) The digestion was terminated by adding an equal volume of McCoy's 5A medium containing 10% FBS, and the cell suspension was collected and centrifuged at 300 g for 3 minutes; and the cells were resuspended in an appropriate amount of fresh medium.
      d) The resuspended cell suspension was taken for counting.
      e) The cell suspension was diluted to 1e4/mL using McCoy's 5A medium containing 10% FBS, and seeded at 50 µL per well, corresponding to 500 cells/well for HCT116 and 500 cells/well for HCT116 MTAP-/-.
      f) The cell plate was placed in a 37°C, 5% carbon dioxide incubator and incubated overnight.
   2. Compound treatment (Day 1)
      a) Each compound was serially diluted in DMSO to generate a 9-point concentration gradient (starting concentration: 10000 µM, 3-fold dilution; the highest concentration may be adjusted individually based on IC50 of different compounds); for example, in a 96-well round-bottom compound-dilution plate, 5 µL of the compound was sequentially diluted into 10 µL of DMSO.
      b) Each concentration point of every compound was then diluted 250-fold into the corresponding volume of McCoy's 5A medium.
      c) To each cell plate, 50 µL of the diluted compound solution as described above was added sequentially to the cell supernatant (50 µL/well).
      d) The compound-treated cell plate was placed in a 37°C, 5% carbon dioxide incubator and incubated.
   3. Re-digestion, re-plating and compound treatment (Day 5)
      a) After 5 days of compound treatment, the compound-containing medium was aspirated, and the cells were rinsed once with PBS (100 µL/well); and the PBS was removed immediately.
      b) The cells were digested by adding 25 µL of trypsin and incubated at 37°C for 3 minutes, and then the digestion was terminated by adding 175 µL/well of McCoy's 5A medium containing 10% FBS.
      c) The cells were mixed thoroughly using a pipette, and re-plated at 1 : 20; and specifically, 10 µL of the cell suspension was aspirated to a new 96-well plate (pre-filled with 40 µL of McCoy's 5A medium containing 10% FBS.
      d) Compounds were prepared and added according to steps a) to c) in Section 2, with 50 µL added per well.
      e) The compound-treated cell plate was placed in a 37°C, 5% carbon dioxide incubator and incubated.
   4. CTG assay (Day 10)
      a) Prior to use, the CellTiter-Glo buffer and lyophilised CellTiter-Glo substrate were equilibrated to room temperature and mixed thoroughly to prepare 100 mL of the CellTiter-Glo reagent (or the pre-mixed CellTiter-Glo reagent was taken out from -20°C and equilibrated to room temperature.
      b) The assay plate was removed from the incubator and equilibrated to room temperature, and then, 50 µL of the CellTiter-Glo reagent was added to each well.
      c) The plate was shaken for 2 minutes to mix thoroughly and ensure complete cell lysis.
      d) The plate was placed at room temperature for 28 minutes to allow signal stabilisation, followed by assay on Pherastar FS.

Comparative example 1 (with reference to WO 2022192745 A1, Examples 16-90) has the following structure:

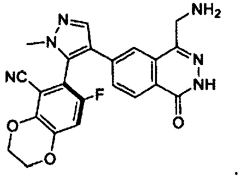

**Table 2: IC₅₀ values of compounds of the present disclosure for growth Inhibition of HCT116 MTAP-/- cells**

| Compound No. | HCT116 MTAP-/-IC₅₀ (nM) | HCT116 IC₅₀ (nM) |
|---|---|---|
| 1-p1 | 11.88 | 1449 |
| 1-p2 | 13.32 | 2347 |
| 2-p1 | 10.20 | 1296 |
| 2-p2 | 13.46 | 2374 |
| 8-p1 | 16.15 | 1454 |
| 11-p3 | 4.60 | - |
| 13-p1 | 15.02 | - |
| Comparative example 1 | 70.79 | - |

| | | |
|---|---|---|
| Conclusion: The compounds of the present disclosure exhibit good selective inhibitory effects on the growth of HCT116 MTAP-/- cells. Compared to comparative example 1, the compounds of the present disclosure exhibit better inhibitory effects on the growth of HCT116 MTAP-/- cells. | | |

### Test example 3: Pharmacokinetic evaluation

### I. Nu/Nu mouse experiment

### 1. Summary

Using Nu/Nu mice as the test animals, the plasma concentrations of the example compounds at different time points were determined by LC-MS/MS following oral gavage (i.g.) administration. The pharmacokinetic behaviours of the compounds of the present disclosure in Nu/Nu mice were investigated, and their pharmacokinetic profiles were evaluated.

### 2. Experimental Protocol

### 2.1 Experimental compounds

Compound 1-p1, compound 2-p1, compound 8-p1 and comparative example 1.

### 2.2 Experimental animals

A total of 36 female Nu/Nu mice (supplied by Vital River Laboratory Animal Technology Co., Ltd.) were randomly divided into 4 groups.

### 2.3 Drug preparation

An appropriate amount of each test compound was weighed and dissolved in 5% DMSO+20% PEG400+70% (10% TPGS)+5% (1% HMPC K100LV) to prepare a 10 mg/mL colourless clear solution.

### 2.4. Administration

The administration dosage was 100 mg/kg, and the administration volume was 10 mL/kg.

### 3. Procedures

Mice were not fasted. After oral gavage administration, blood (0.1 mL) was collected from the orbital cavity before administration, and at 0.25 h, 0.5 h, 1.0 h, 2.0 h, 4.0 h, 6.0 h, 8.0 h, 11.0 h and 24.0 h after administration. The blood was placed into EDTA-K2 anticoagulant tubes and centrifuged at 10000 rpm for 1 minute (4°C) to separate plasma. The plasma was separated within 1 h and stored at -20°C until analysis. The entire process from blood collection to centrifugation was performed in ice bath conditions.

Determination of test compound contents in plasma of Nu/Nu mice after administration of different compounds: To 20 µL of plasma samples from each time point in Nu/Nu mice, 200 µL of acetonitrile and 25 µL of verapamil (100 ng/mL) were added. The mixture was vortex-mixed for 5 minutes and centrifuged at 4000 rpm for 15 minutes. 90 µL of water and 90 µL of the supernatant were vortex-mixed for 5 minutes. 2 µL (for compound 1-p1), 2 µL (for compound 2-p1), 1 µL (for compound 8-p1), or 0.5 µL (for comparative example 1) of the supernatant was taken for LC-MS/MS analysis receptively.

### 4. Pharmacokinetic parameter results

**Table 3. Pharmacokinetic parameters of compounds of the present disclosure in Nu/Nu mice**

| Compound No. | Administration method/ administration dosage | Plasma concentration Cmax (ng/mL) | Area under the curve AUC₀₋ₜ (h*ng/mL) | Half life T1/2 (h) | Clearance CL/F (mL/min/kg) | Apparent volume of distribution V/F (mL/kg) |
|---|---|---|---|---|---|---|
| 1-p1 | i.g./ 100 mg/kg | 3247 | 8647 | 2.2 | 189 | 35382 |
| 2-p1 | i.g./ 100 mg/kg | 2943 | 7136 | 1.2 | 233 | 23314 |
| 8-p1 | i.g./ 100 mg/kg | 2533 | 7635 | 1.3 | 218 | 23936 |
| Comparative example 1 | i.g./ 100 mg/kg | 657 | 1719 | 1.6 | 960 | 132312 |

| | | | | | | |
|---|---|---|---|---|---|---|
| Conclusion: Compared to comparative example 1, the compounds of the present disclosure exhibit higher plasma concentrations, greater exposure, and lower clearance in Nu/Nu mice, demonstrating pharmacokinetic advantages. | | | | | | |

### Test example 4: Efficacy test

### 1. Experimental objective

To evaluate the inhibitory effect of the compounds of the present disclosure on tumour growth in a subcutaneous xenograft model using the human lung giant cell carcinoma cell line Lu99 in female NUNU nude mice

### 2. Experimental drugs

### Compound 2-p1

Using a solution of 20% PEG400 + 70% (10% TPGS) + 5%DMSO+5% (1% HPMCK100LV) solution

### 3. Experimental methods and experimental materials

### 3.1 Laboratory animals and housing conditions

Laboratory animals: Female NUNU nude mice, purchased from Zhejiang Vital River Laboratory Animal Technology Co., Ltd. (License No.: SCXK(Zhe)2024-0001; animal qualification certificate No.: 20240524Abzz0619000789), weighing approximately 20-24 g at arrival.

Housing conditions: The mice were housed 5 per cage under a 12/12 h light/dark cycle, constant temperature of 23°C ± 1°C, humidity of 50%-60%, with free access to food and water.

### 3.2 Animal grouping

After acclimatisation, NUNU nude mice were grouped and administered as follows:

**Table 4. Animal grouping and dosing regimen for in vivo efficacy experiment**

| Grouping | Number of animals (n) | Dosing regimen |
|---|---|---|
| Vehicle control | 12 | 20% PEG400 + 70% (10% TPGS) + 5%DMSO+5% (1% HPMCK100LV) solution (i.g./qd) |
| Compound 2-p1 | 12 | 50 mpk (i.g./qd) |
| Compound 2-p1 | 12 | 100 mpk (i.g./qd) |
| Compound 2-p1 | 12 | 200 mpk (i.g./qd) |

| | | |
|---|---|---|
| Notes: qd: administered once daily; i.g.: oral gavage. | | |

### 3.3 Experimental methods:

0.1 ml (5 × 10⁵ cells ± 50% matrigel) of Lu99 cells (cell-to-matrigel volume ratio = 1 : 1) in the logarithmic growth phase was subcutaneously injected into the right upper limb of each mouse. When the mean tumour volume reached 75 mm³, mice were randomly assigned to 4 groups (12 mice per group) based on tumour volume and body weight, as shown in Table 4. The day of grouping was designated as D0, and oral gavage administration was initiated once or twice daily for a total of 21 days. The 21st day after administration was designated as D21 (Table 5). Tumour volume and body weight of tumour-bearing mice were measured twice weekly using a calliper and a balance, respectively, and the data were recorded.

### 3.4 Data statistics

All data were plotted and statistically analysed using Excel and GraphPad Prism 10 software.

Tumour volume (V) was calculated using the formula: V = 1/2 × a × b², where a represents the length and b represents the width.

Relative tumour proliferation rate: T/C (%) = (T-T₀)/(C-C₀) × 100(%), where T and C are the tumour volumes of the treated and control groups at the end of the experiment, and T₀ and C₀ are the corresponding tumour volumes at the start of the experiment.

Tumour growth inhibition: TGI (%) = 100-T/C (%).

### 4. Results

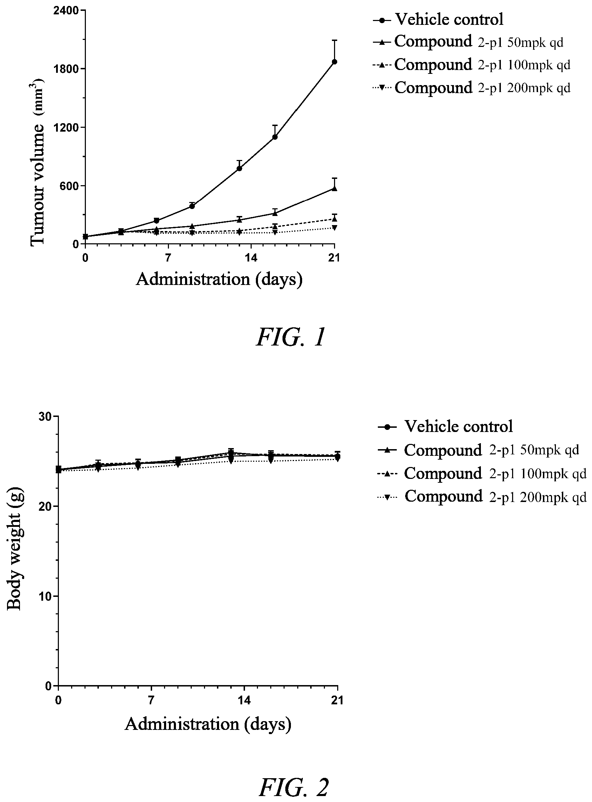

The efficacy data of compound 2-p1 against Lu99 xenografts in NUNU nude mice are shown in Table 5 and FIG. 1.

The effect of compound 2-p1 on the body weight of NUNU nude mice is shown in FIG. 2.

**Table 5. Efficacy of compounds of the present disclosure against Lu99 xenografts in NUNU nude mice**

| Grouping | Administr ation | Route Dosage (mpk) | Mean tumour volume (mm³) | | | | TGI (%) D21 | p | Surviving animals/gr oup |
|---|---|---|---|---|---|---|---|---|---|
| | | | D0 | SEM | D21 | SEM | | (vs vehicle control) | |
| Vehicle control | qd/21d | i.g., 0 | 75 | 4 | 1870 1 | 220 | / | / | 12/12 |
| Compound 2-p1 | qd/21d | i.g., 50 | 75 | 4 | 574 | 104 | 72 | <0.0001 | 12/12 |
| Compound 2-p1 | qd/21d | i.g., 100 | 75 | 4 | 256 | 48 | 90 | <0.0001 | 12/12 |
| Compound 2-p1 | qd/21d | i.g., 200 | 75 | 4 | 165 | 12 | 95 | <0.0001 | 12/12 |

| | | | | | | | | | |
|---|---|---|---|---|---|---|---|---|---|
| Notes: qd: administered once daily; d: day(s); i.g.: oral gavage; SEM: standard error. | | | | | | | | | |

### 5. Conclusion

Compound 2-p1, administered once daily for 21 days, exhibits tumour growth inhibition of 72% at the low dose (50 mpk/qd), 90% at the medium dose (100 mpk/qd), and 95% at the high dose (200 mpk/qd), with no effects on body weight of the mice.

## Claims

1. A compound represented by general formula (I) or a pharmaceutically acceptable salt thereof: 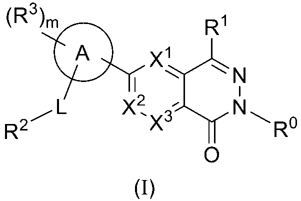 **characterised in that**:
ring A is aryl or heteroaryl;
R⁰ is selected from a hydrogen atom, alkyl, haloalkyl, deuterioalkyl, hydroxyalkyl, alkoxy, haloalkoxy, deuterioalkoxy, alkoxyalkyl, aminoalkyl, cycloalkyl, cycloalkylalkyl, heterocyclyl and heterocyclylalkyl;
R¹ is selected from -(CR^{1a}R^{1b})r-NH₂, 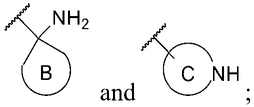
R^{1a} and R^{1b} are the same or different, and are each independently selected from a hydrogen atom, a deuterium atom, halogen, hydroxyl, cyano, amino, alkyl, haloalkyl, deuterioalkyl, hydroxyalkyl, alkoxy, haloalkoxy, deuterioalkoxy and cycloalkyl;
or R^{1a} and R^{1b} on the same carbon atom together form =O;
or R^{1a} and R^{1b} together with the carbon atom to which they are attached form cycloalkyl or heterocyclyl, wherein the cycloalkyl or heterocyclyl is optionally substituted with one or more R;
ring B is cycloalkyl or heterocyclyl, wherein the cycloalkyl or heterocyclyl is optionally substituted with one or more R;
ring C is nitrogen-containing heterocyclyl, wherein the nitrogen-containing heterocyclyl is optionally substituted with one or more R;
L is selected from a bond, alkylene, O, S(O)ᵥ, NR^{a}, C(O), NR^{a}C(O) and C(O)NR^{a};
R^{a} is selected from a hydrogen atom, alkyl, haloalkyl, hydroxyalkyl, cycloalkyl and cycloalkylalkyl;
R² is selected from tricyclic cycloalkyl, tricyclic heterocyclyl, tricyclic aryl and tricyclic heteroaryl, wherein the tricyclic cycloalkyl, tricyclic heterocyclyl, tricyclic aryl and tricyclic heteroaryl are each independently and optionally substituted with one or more R⁴;
each of R³ and R⁴ is the same or different, and is independently selected from a deuterium atom, halogen, hydroxyl, cyano, nitro, amino, alkenyl, alkynyl, alkyl, haloalkyl, deuterioalkyl, hydroxyalkyl, alkoxy, haloalkoxy, deuterioalkoxy, alkoxyalkyl, aminoalkyl, cycloalkyl, cycloalkylalkyl, cycloalkyloxy, heterocyclyl, heterocyclylalkyl, heterocyclyloxy, aryl, arylalkyl, heteroaryl, heteroarylalkyl, - (CH₂)ₜNR¹⁴R¹⁵, -OR¹⁶, -S(O)ᵥR¹⁶, -C(O)NR¹⁴R¹⁵, -C(O)R¹⁶, -C(O)OR¹⁶, - NR¹⁷C(O)R¹⁶, -S(O)ᵥNR¹⁴R¹⁵ and -NR¹⁷S(O)ᵥR¹⁶, wherein the alkyl, alkenyl, alkynyl, alkoxy, cycloalkyl, cycloalkylalkyl, cycloalkyloxy, heterocyclyl, heterocyclylalkyl, heterocyclyloxy, aryl, arylalkyl, heteroaryl and heteroarylalkyl are each independently and optionally substituted with one or more R;
or two R⁴ on the same atom together form =O;
or two R⁴ together with the ring atom on R² to which they are attached form cycloalkyl, heterocyclyl, aryl or heteroaryl, wherein the cycloalkyl, heterocyclyl, aryl or heteroaryl is optionally substituted with one or more R;
X¹ is CR^{5a} or N;
X² is CR^{5b} or N;
X³ is CR⁵ or N;
R^{5a}, R^{5b} and R⁵ are the same or different, and are each independently selected from a hydrogen atom, a deuterium atom, halogen, hydroxyl, cyano, amino, alkenyl, alkynyl, alkyl, haloalkyl, deuterioalkyl, hydroxyalkyl, alkoxy, haloalkoxy, deuterioalkoxy, alkoxyalkyl, aminoalkyl, cycloalkyl, cycloalkylalkyl, heterocyclyl, heterocyclylalkyl, aryl, arylalkyl, heteroaryl, heteroarylalkyl, -(CH₂)ₜNR¹⁴R¹⁵, - OR¹⁶, -C(O)NR¹⁴R¹⁵, -C(O)R¹⁶, -C(O)OR¹⁶ and -NR¹⁷C(O)R¹⁶, wherein the alkyl, alkenyl, alkynyl, alkoxy, cycloalkyl, cycloalkylalkyl, heterocyclyl, heterocyclylalkyl, aryl, arylalkyl, heteroaryl and heteroarylalkyl are each independently and optionally substituted with one or more R;
R¹⁴, R¹⁵ and R¹⁶ at each occurrence are the same or different, and are each independently selected from a hydrogen atom, alkyl, alkenyl, alkynyl, cycloalkyl, cycloalkylalkyl, heterocyclyl, heterocyclylalkyl, aryl and heteroaryl, wherein the alkyl, alkenyl, alkynyl, cycloalkyl, cycloalkylalkyl, heterocyclyl, heterocyclylalkyl, aryl and heteroaryl are each independently and optionally substituted with one or more R;
or R¹⁴ and R¹⁵ together with the nitrogen atom to which they are attached form heterocyclyl, wherein the heterocyclyl is optionally substituted with one or more R;
each R¹⁷ at each occurrence is the same or different, and is independently selected from a hydrogen atom, alkyl, cycloalkyl and cycloalkylalkyl;
each R at each occurrence is the same or different, and is independently selected from oxo, a deuterium atom, halogen, hydroxyl, alkenyl, alkynyl, cyano, nitro, amino, -NH alkyl, -N(alkyl)₂, alkyl, haloalkyl, deuterioalkyl, hydroxyalkyl, alkoxy, haloalkoxy, deuterioalkoxy, alkoxyalkyl, aminoalkyl, cycloalkyl, cycloalkylalkyl, cycloalkyloxy, heterocyclyl, heterocyclylalkyl, heterocyclyloxy, aryl, arylalkyl, aryloxy, heteroaryl, heteroarylalkyl and heteroaryloxy;
m is 0, 1, 2, 3 or 4;
r is 0, 1, 2 or 3;
v is 0, 1 or 2; and
t is 0, 1, 2 or 3.

2. The compound represented by general formula (I) or the pharmaceutically acceptable salt thereof according to claim 1, **characterised in that** ring A is 5- to 10-membered heteroaryl; preferably, ring A is pyrazolyl.

3. The compound represented by general formula (I) or the pharmaceutically acceptable salt thereof according to claim 1, **characterised in that** the compound or the pharmaceutically acceptable salt thereof is a compound represented by general formula (II) or a pharmaceutically acceptable salt thereof: wherein: 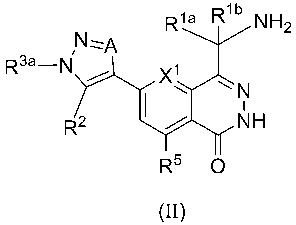
R^{3a} is selected from a hydrogen atom, alkenyl, alkynyl, alkyl, haloalkyl, deuterioalkyl, hydroxyalkyl, alkoxyalkyl, aminoalkyl, cycloalkyl, cycloalkylalkyl, heterocyclyl, heterocyclylalkyl, aryl, arylalkyl, heteroaryl and heteroarylalkyl, wherein the alkyl, alkenyl, alkynyl, cycloalkyl, cycloalkylalkyl, heterocyclyl, heterocyclylalkyl, aryl, arylalkyl, heteroaryl and heteroarylalkyl are each independently and optionally substituted with one or more R;
A is N or CR^{3b};
R^{3b} is a hydrogen atom or R³;
X¹, R, R^{1a}, R^{1b}, R², R³ and R⁵ are as defined in claim 1.

4. The compound represented by general formula (I) or the pharmaceutically acceptable salt thereof according to any one of claims 1 to 3, **characterised in that** R^{1a} and R^{1b} are the same or different, and are each independently a hydrogen atom or a deuterium atom; preferably, R^{1a} and R^{1b} are hydrogen atoms.

5. The compound represented by general formula (I) or the pharmaceutically acceptable salt thereof according to any one of claims 1 to 4, **characterised in that** X¹ is CR^{5a}; R^{5a} is selected from a hydrogen atom, halogen and C₁₋₆ alkyl; preferably, X¹ is CH.

6. The compound represented by general formula (I) or the pharmaceutically acceptable salt thereof according to any one of claims 3 to 5, **characterised in that** A is CR^{3b}; R^{3b} is selected from a hydrogen atom, halogen and C₁₋₆ alkyl; preferably, A is CH.

7. The compound represented by general formula (I) or the pharmaceutically acceptable salt thereof according to any one of claims 1 to 6, **characterised in that** the compound or the pharmaceutically acceptable salt thereof is a compound represented by general formula (IV) or a pharmaceutically acceptable salt thereof: 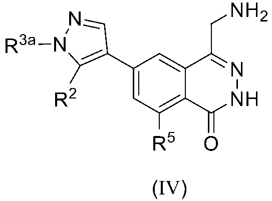 wherein:
R², R^{3a} and R⁵ are as defined in claim 3.

8. The compound represented by general formula (I) or the pharmaceutically acceptable salt thereof according to any one of claims 1 to 7, **characterised in that** R² is selected from 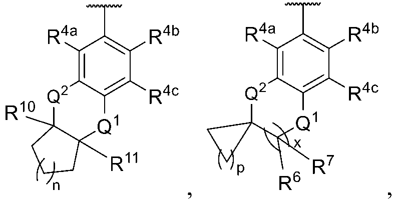 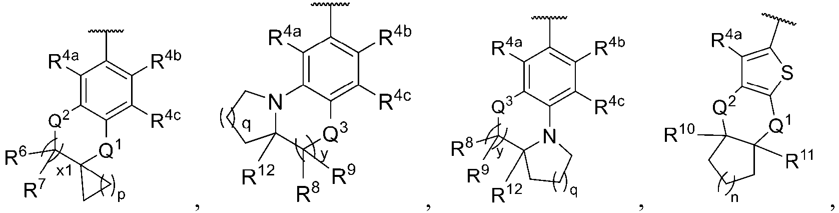 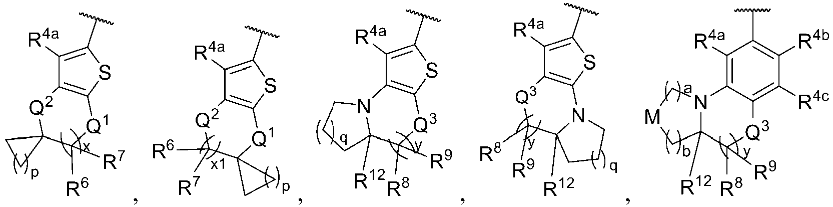 and 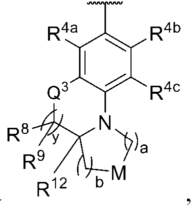
wherein: M is selected from CR⁴R⁴, O, S and NR^{m}; R^{m} is selected from a hydrogen atom, alkyl, haloalkyl, deuterioalkyl, hydroxyalkyl, alkoxyalkyl, aminoalkyl, cycloalkyl, cycloalkylalkyl, heterocyclyl and heterocyclylalkyl; Q¹, Q² and Q³ are the same or different, and are each independently selected from a bond, O, S(O)ᵥ, CR^{4d}R^{4e}, C(O) and NR^{4f}; R^{4a}, R^{4b}, R^{4c}, R^{4d} and R^{4e} are the same or different, and are each independently a hydrogen atom or R⁴; or R^{4d} and R^{4e} together with the carbon atom to which they are attached form cycloalkyl or heterocyclyl, wherein the cycloalkyl or heterocyclyl is optionally substituted with one or more R;
R^{4f} is selected from a hydrogen atom, alkyl, haloalkyl, deuterioalkyl, hydroxyalkyl, alkoxyalkyl, aminoalkyl, cycloalkyl, cycloalkylalkyl, heterocyclyl and heterocyclylalkyl;
R⁶, R⁷, R⁸, R⁹, R¹⁰, R¹¹ and R¹² are the same or different, and are each independently a hydrogen atom or R⁴; or R⁶ and R⁷ together with the carbon atom to which they are attached form cycloalkyl or heterocyclyl, wherein the cycloalkyl or heterocyclyl is optionally substituted with one or more R; or R⁸ and R⁹ together with the carbon atom to which they are attached form cycloalkyl or heterocyclyl, wherein the cycloalkyl or heterocyclyl is optionally substituted with one or more R; or R⁶ and R⁷ on the same carbon atom together form =O; or R⁸ and R⁹ on the same carbon atom together form =O; n is 0, 1, 2, 3 or 4; p is 1, 2, 3, 4, 5 or 6; q is 0, 1, 2, 3 or 4; x is 0, 1, 2, 3, 4 or 5; x1 is 1, 2, 3, 4 or 5; y is 0, 1, 2, 3 or 4; a is 0, 1, 2, 3 or 4; b is 0, 1, 2, 3 or 4; R⁴, R and v are as defined in claim 1;
preferably, R² is selected from 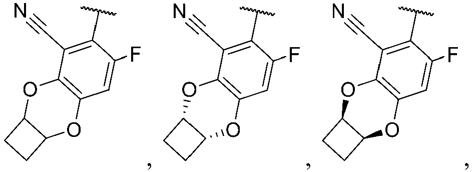 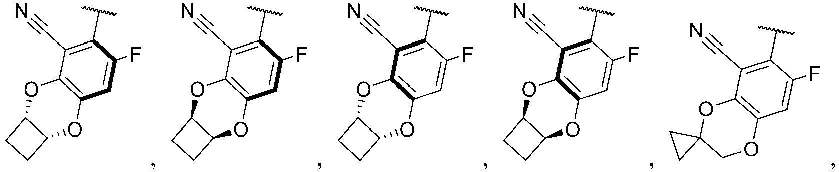 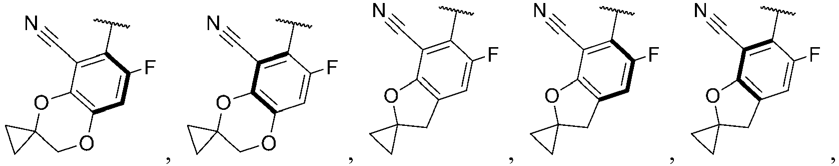 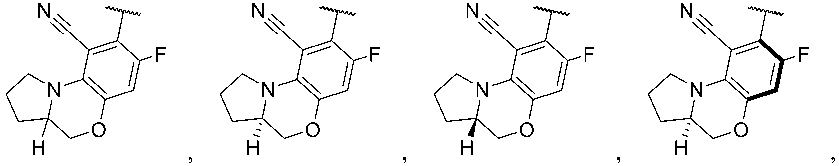 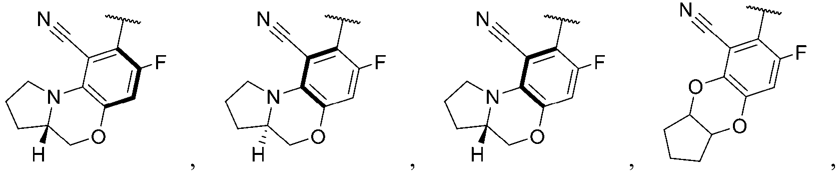 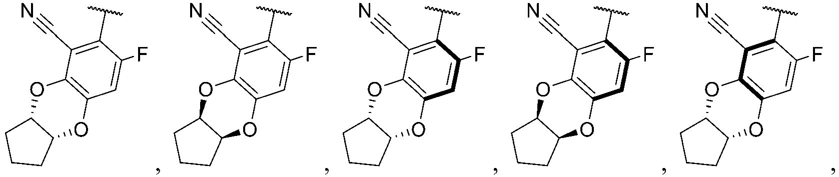 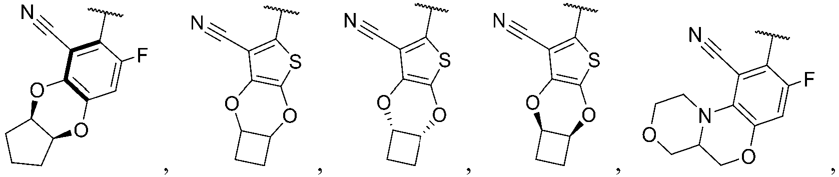 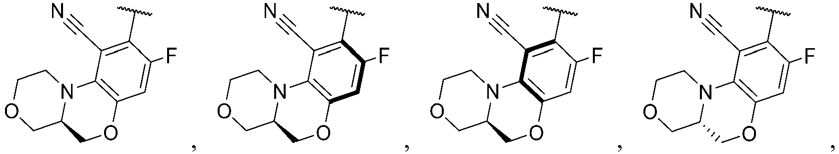 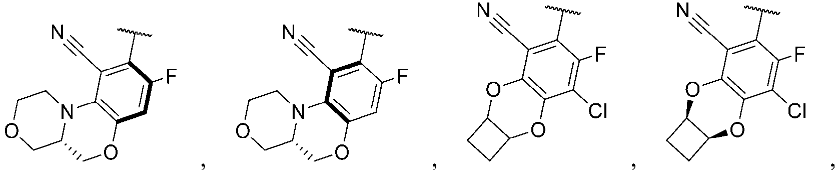 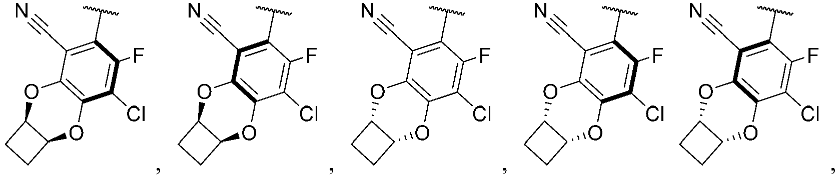 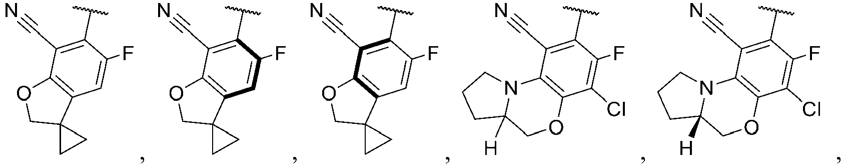 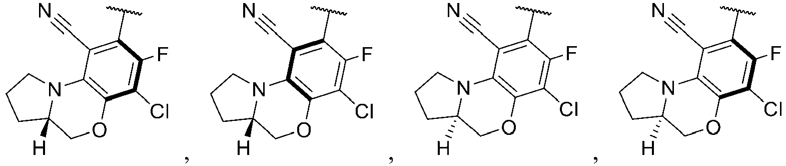 and

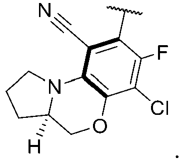

9. The compound represented by general formula (I) or the pharmaceutically acceptable salt thereof according to any one of claims 3 to 8, **characterised in that** R^{3a} is C₁₋₆ alkyl or C₁₋₆ deuterioalkyl; preferably, R^{3a} is C₁₋₆ deuterioalkyl.

10. The compound represented by general formula (I) or the pharmaceutically acceptable salt thereof according to any one of claims 1 to 9, **characterised in that** R⁵ is selected from a hydrogen atom, halogen, cyano, C₁₋₆ alkyl, 3- to 6-membered cycloalkyl and 3- to 6-membered heterocyclyl; preferably, R⁵ is a hydrogen atom.

11. The compound represented by general formula (I) or the pharmaceutically acceptable salt thereof according to any one of claims 1 to 10, **characterised in that** the compound is selected from any one of: 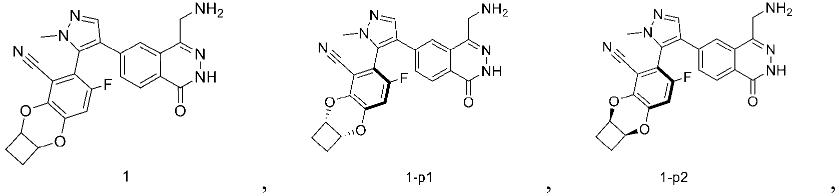 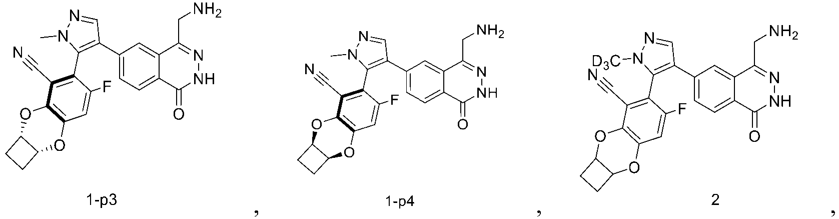 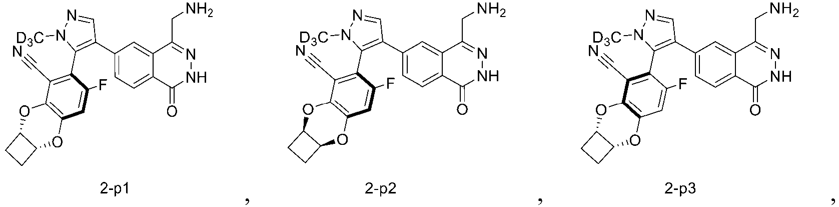 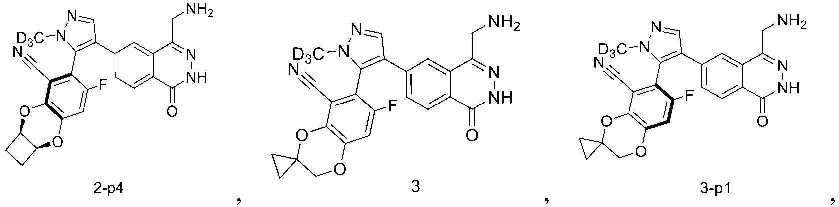 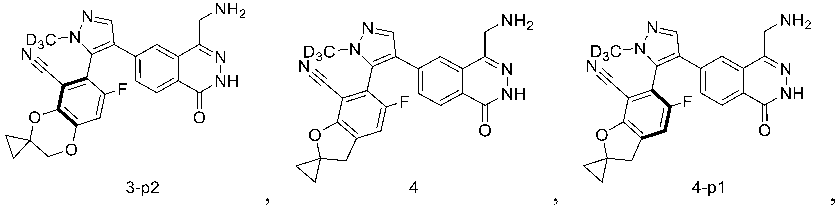 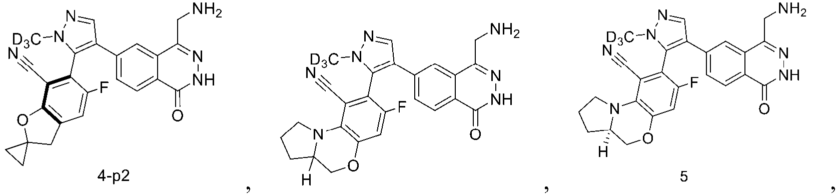 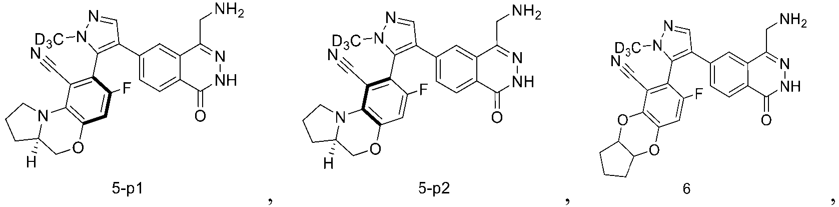 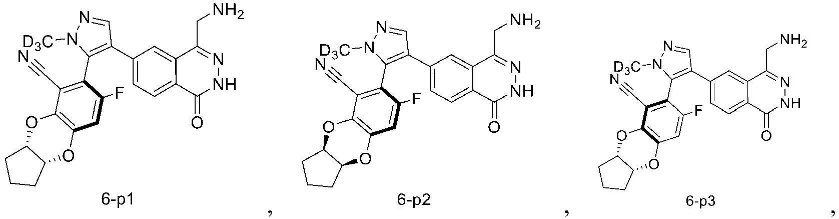 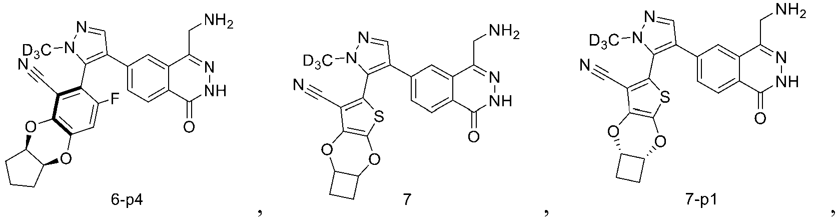 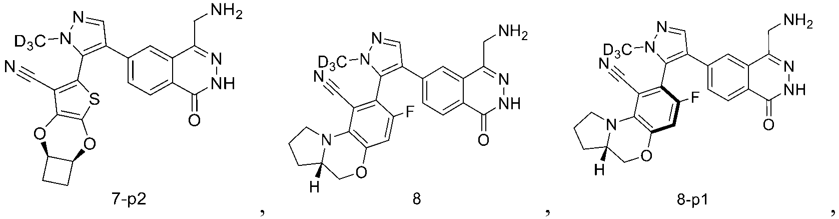 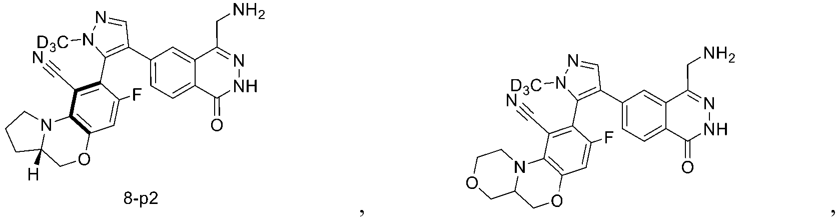 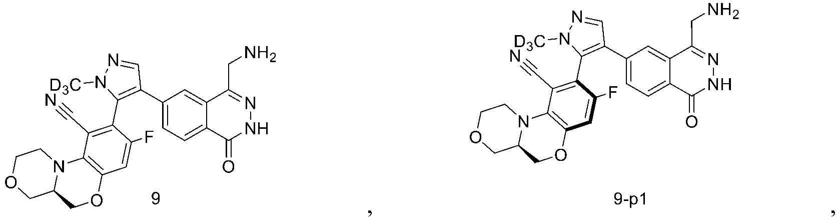 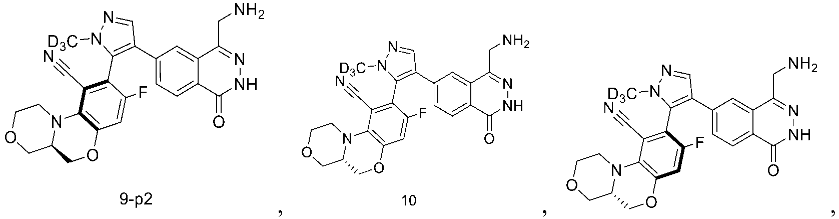 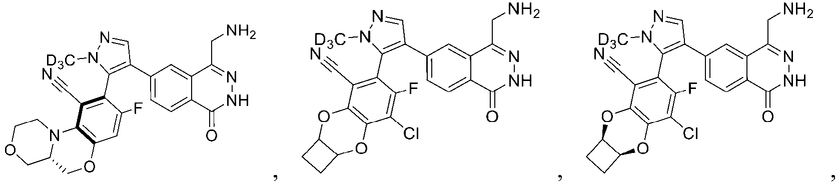 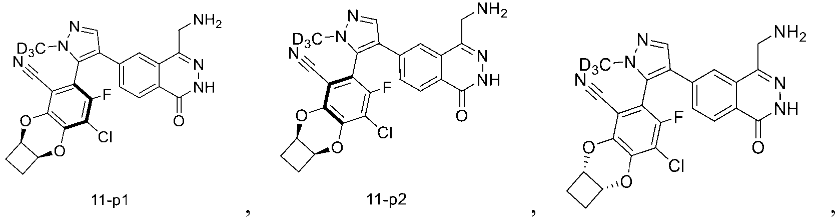 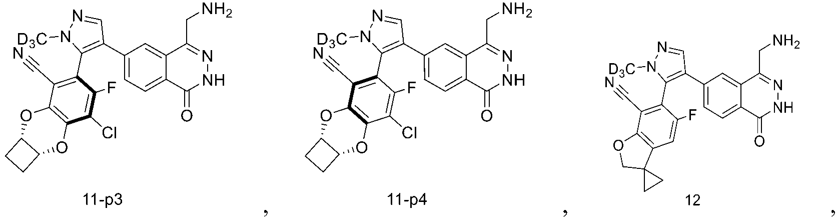 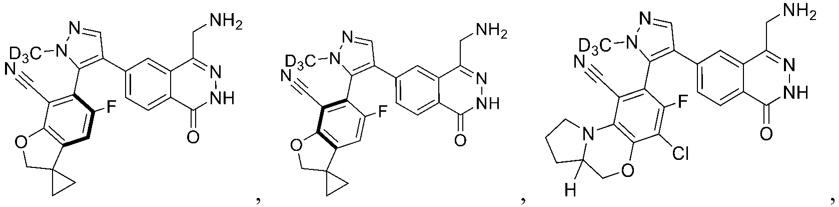 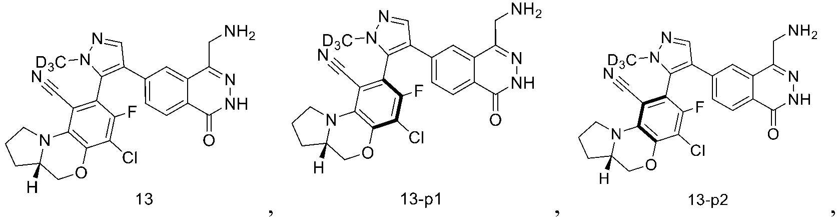 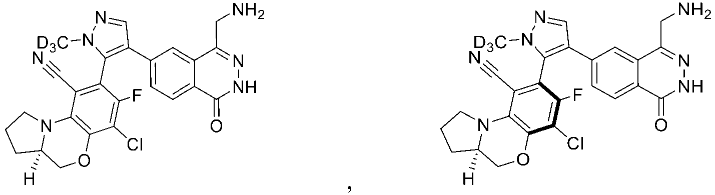 and

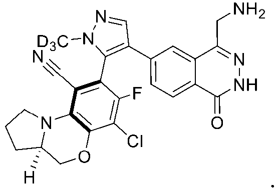

12. A compound represented by general formula (IIA) or a salt thereof: 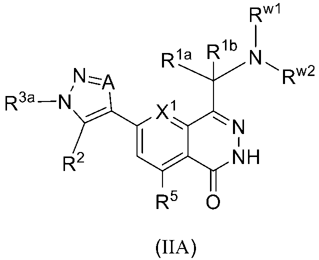 **characterised in that**:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido;
A, X¹, R^{1a}, R^{1b}, R², R^{3a} and R⁵ are as defined in claim 3.

13. The compound represented by general formula (IIA) or the salt thereof according to claim 12, **characterised in that** the compound is selected from any one of:

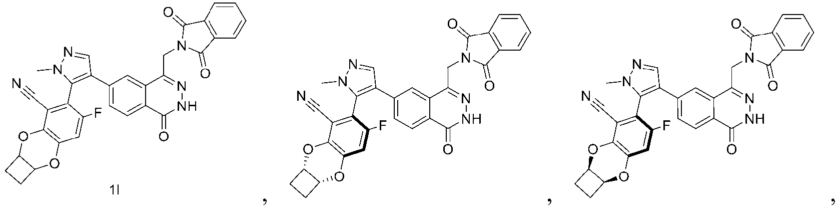

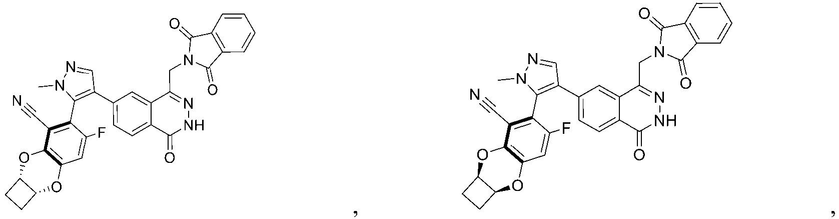

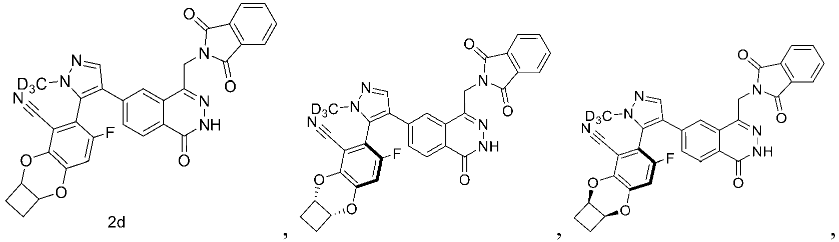

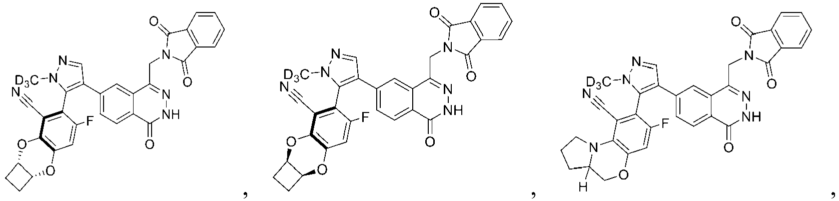

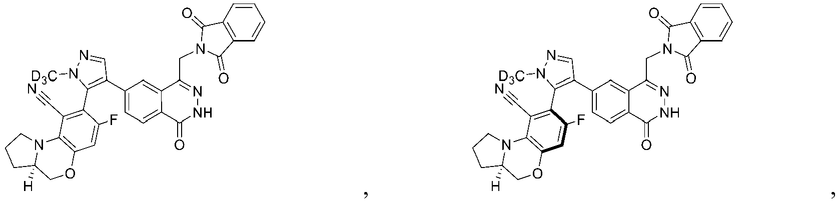

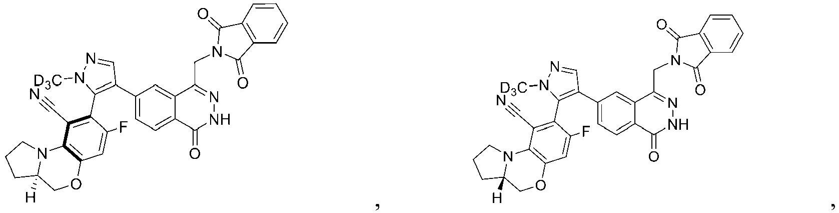

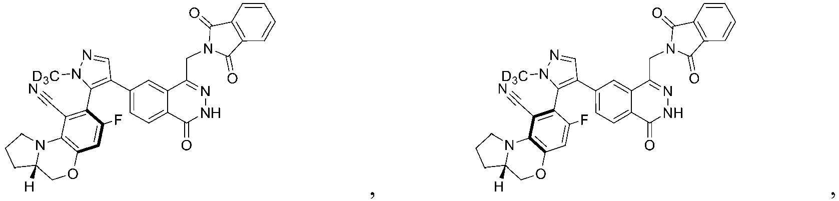

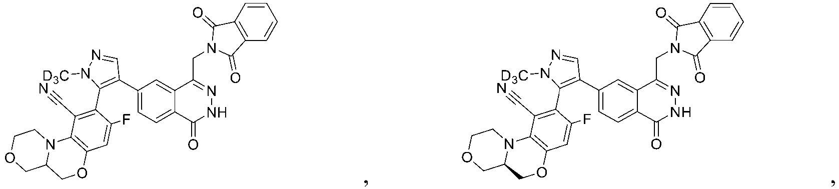

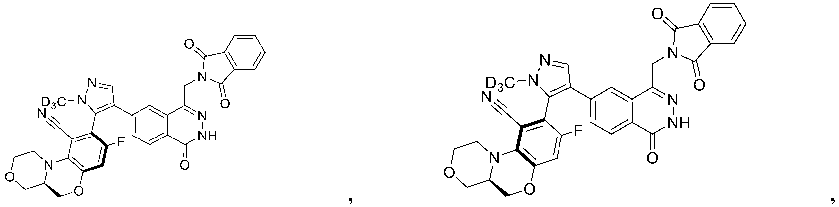

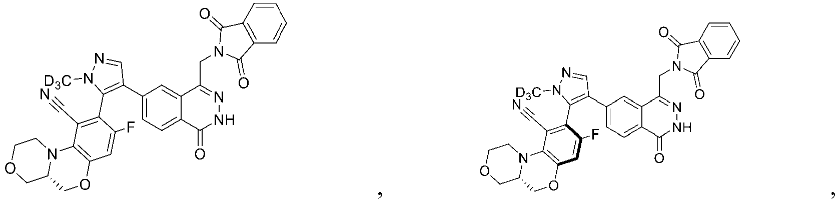

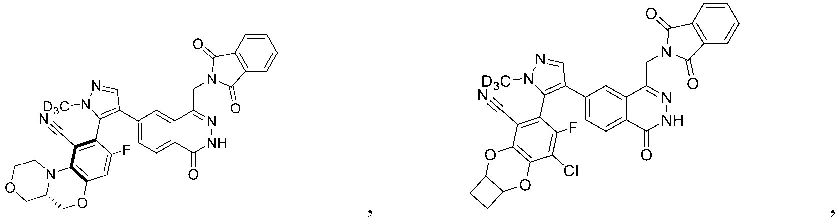

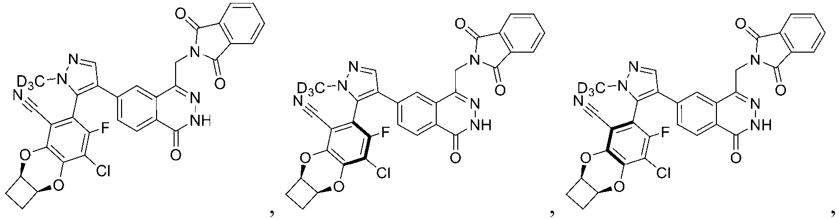

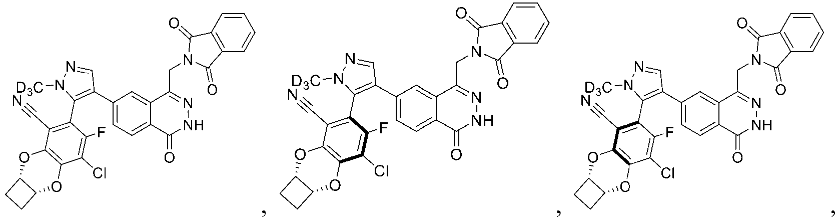

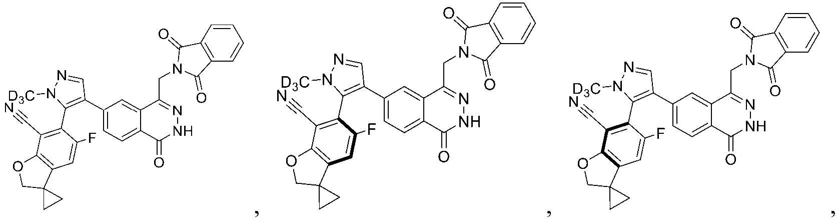

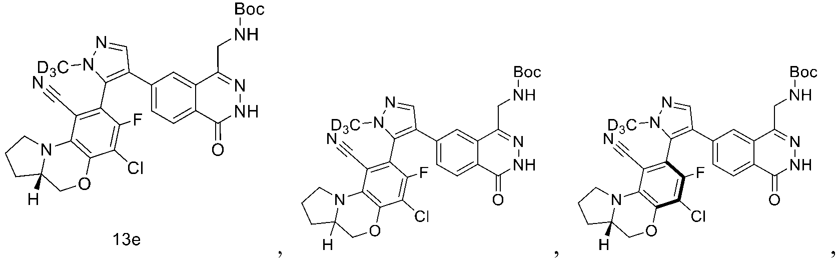

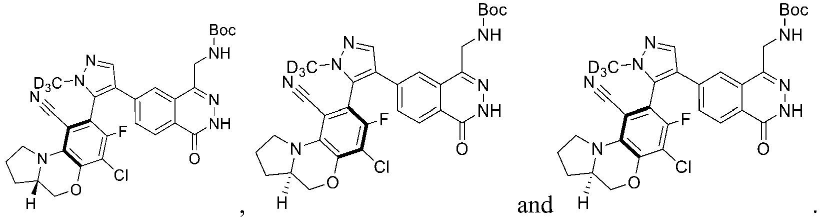

14. A method for preparing the compound represented by general formula (I) or the pharmaceutically acceptable salt thereof according to claim 3, **characterised in that** the method comprises: 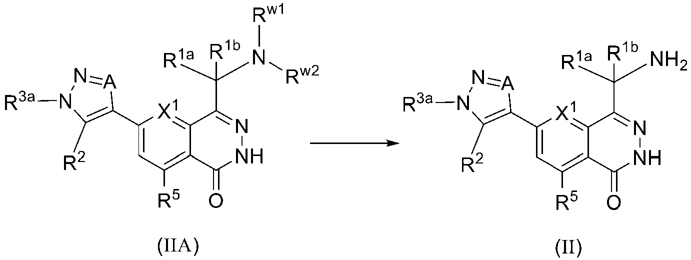
removing an amino protecting group from a compound represented by general formula (IIA) or a salt thereof to obtain a compound represented by general formula (II) or a pharmaceutically acceptable salt thereof,
wherein:
R^{w1} is a hydrogen atom or an amino protecting group;
R^{w2} is a hydrogen atom or an amino protecting group; provided that at least one of R^{w1} and R^{w2} is an amino protecting group;
or R^{w1} and R^{w2} together with the nitrogen atom to which they are attached form phthalimido;
A, X¹, R^{1a}, R^{1b}, R², R^{3a} and R⁵ are as defined in claim 3.

15. A pharmaceutical composition, **characterised by** comprising the compound represented by general formula (I) or the pharmaceutically acceptable salt thereof according to any one of claims 1 to 11, and one or more pharmaceutically acceptable carriers, diluents or excipients.

16. Use of the compound represented by general formula (I) or the pharmaceutically acceptable salt thereof according to any one of claims 1 to 11 or the pharmaceutical composition according to claim 15 in the preparation of a PRMT5 inhibitor.

17. Use of the compound represented by general formula (I) or the pharmaceutically acceptable salt thereof according to any one of claims 1 to 11 or the pharmaceutical composition according to claim 15 in the preparation of a medicament for treating and/or preventing cancer, **characterised in that** the cancer is preferably selected from lung cancer, kidney cancer, liver cancer, head and neck cancer, oesophageal cancer, lymphoma, spongioblastoma, glioblastoma multiforme, colorectal cancer, malignant peripheral nerve sheath tumour (MPNST), melanoma, stomach cancer, pancreatic cancer, cholangiocarcinoma, bladder cancer, breast cancer, ovarian cancer, vaginal cancer, cervical cancer, endometrial cancer, prostate cancer, testicular cancer, seminoma, myeloma, leukaemia, acoustic neuroma, basal cell carcinoma, brain cancer, bronchial cancer, sarcoma, choriocarcinoma, craniopharyngioma, cystadenocarcinoma, haemangioendothelioma, ependymoma, epithelial carcinoma, glioma, astrocytoma, hemangioblastoma, medulloblastoma, meningioma, mesothelioma, neuroblastoma, bone cancer, nasopharyngeal carcinoma, oral cancer, laryngeal cancer, thyroid cancer, retinoblastoma, skin cancer, squamous cell carcinoma, synovial sarcoma, hidradenocarcinoma and myelodysplastic syndrome; preferably, the cancer is selected from ovarian cancer, lymphoma, pancreatic cancer, bladder cancer, stomach cancer, colorectal cancer, cholangiocarcinoma, mesothelioma, malignant peripheral nerve sheath tumour (MPNST), glioblastoma multiforme and lung cancer.
